# EUROPEAN PATENT APPLICATION

(11) **EP 4 837 567 A1**
(43) Date of publication of application: **11.11.2026**
(21) Application number: 25736348.1
(22) Date of filing: 03.01.2025
(51) Int. Cl.: A61K 9/28, A61K 38/07, A61P 17/04

(54) **DIFELIKEFALIN ORAL PHARMACEUTICAL COMPOSITION**

(30) Priority: 05.01.2024 CN 202410017810; 27.08.2024 CN 202411179938
(71) Applicant: Chengdu Gowell Biopharmaceutical Co., Ltd., Chengdu, Sichuan 610093 (CN)
(72) Inventor: LV, Yan, Chengdu, Sichuan 610093 (CN); GUO, Wenping, Chengdu, Sichuan 610093 (CN); LI, Qingran, Chengdu, Sichuan 610093 (CN); HUANG, Yi, Chengdu, Sichuan 610093 (CN); YU, Peiting, Chengdu, Sichuan 610093 (CN)
(74) Representative: Gassner, Birgitta
(86) International application number: PCT/CN2025/070491
(87) International publication number: WO 2025/146144

(57) **Abstract**

A difelikefalin oral pharmaceutical composition. The pharmaceutical composition comprises difelikefalin, a delivery agent, an auxiliary agent, and optionally a pharmaceutically acceptable non-active ingredient, and may have good bioavailability and reduced variability in drug effectiveness.

## Description

### TECHNICAL FIELD

The present application relates to the field of medical technology, in particular to a difelikefalin oral pharmaceutical composition.

### BACKGROUND

Difelikefalin is an opioid receptor agonist. The currently marketed products are injectable drugs, which are mainly administered by physicians, bringing great inconvenience to patients. Oral preparations, however, enable self-administration by patients, do not directly damage the skin and mucous membranes, thereby reducing patient discomfort and improving their compliance. However, as a polypeptide, difelikefalin is easily degraded and destroyed by proteolytic enzymes in the digestive tract and is not easy to pass through the gastrointestinal mucosa. If difelikefalin is designed to be taken as an oral preparation, a delivery agent is required to facilitate the absorption of difelikefalin through the gastrointestinal mucosa.

However, the absorption of the oral preparation of difelikefalin with a delivery agent described above is highly correlated with difelikefalin, the delivery agent, and the preparation form, thereby causing a relatively high variability in the effective concentration or bioavailability of the drug of difelikefalin *in vivo*. Therefore, it is necessary to provide a difelikefalin oral preparation, which can reduce the variability of the effective concentration or bioavailability of difelikefalin *in vivo* and improve the bioavailability of difelikefalin.

### SUMMARY OF THE INVENTION

The embodiment of the present application provides a difelikefalin oral pharmaceutical composition, comprising difelikefalin, a delivery agent, an auxiliary agent, and optionally a pharmaceutically acceptable non-active ingredient.

In some embodiments, the difelikefalin oral pharmaceutical composition comprises difelikefalin, wherein the mass content of difelikefalin is from about 0.05 wt% to about 5 wt% based on the total mass of the pharmaceutical composition; a delivery agent, wherein the mass content of the delivery agent is from about 15 wt% to about 75 wt% based on the total mass of the pharmaceutical composition; an auxiliary agent, wherein the mass content of the auxiliary agent is from about 5 wt% to about 70 wt% based on the total mass of the pharmaceutical composition; and optionally a pharmaceutically acceptable non-active ingredient, wherein the mass content of optionally the pharmaceutically acceptable non-active ingredient is from about 20 wt% to about 60 wt% based on the total mass of the pharmaceutical composition.

In some embodiments, the delivery agent can be selected from one or more of N-[8-(2-hydroxybenzoyl)amino]octanoic acid (NAC), N-[6-(2-hydroxybenzoyl)amino]hexanoic acid (NAH), N-[10-(2-hydroxybenzoyl)amino]decanoic acid (NAD), N-[8-(5-chlorosalicyloyl) amino]octanoic acid (5-CNAC), N-[8-(2-hydroxy-4-methoxybenzoyl)amino]octanoic acid (4-MOAC), N-[4-(2-hydroxy-4-chlorobenzoyl)amino]butyric acid (4-CNAB), and pharmaceutically acceptable salts thereof.

In some embodiments, the delivery agent can be further selected from one or more of sodium N-[8-(2-hydroxybenzoyl)amino]octanoate (SNAC), potassium N-[8-(2-hydroxybenzoyl)amino]octanoate, and/or calcium N-[8-(2-hydroxybenzoyl)amino]octanoate.

In some embodiments, the delivery agent can preferably be SNAC.

In some embodiments, a mass ratio of the delivery agent to difelikefalin can be from about 5:1 to about 500:1.

In some embodiments, the auxiliary agent can comprise one or more of the following compounds: sodium phosphate, disodium hydrogen phosphate, sodium tartrate, sodium acetate, sodium citrate, sodium glutamate, hydrotalcite, and other pharmaceutically acceptable salts of the same acid radicals.

In some embodiments, a mass ratio of the delivery agent to the auxiliary agent can be from about 0.5:1 to about 10:1.

In some embodiments, the pharmaceutical composition can comprise one kind of granule. Alternatively, in some embodiments, the pharmaceutical composition can be made of two or more granules with different compositions.

In some embodiments, the pharmaceutical composition can comprise a first granule and a second granule, wherein the first granule can comprise difelikefalin and optionally a pharmaceutically acceptable non-active ingredient, and the second granule can comprise a delivery agent, an auxiliary agent, and optionally a pharmaceutically acceptable non-active ingredient.

In some embodiments, the present application provides a difelikefalin oral pharmaceutical composition, comprising an outer layer and a core enclosed by the outer layer, wherein,
the core comprises difelikefalin and a delivery agent; and
the outer layer comprises a delivery agent.

In some embodiments, the core can further comprise an auxiliary agent. In some embodiments, the core can further comprise a diluent. In some embodiments, the core can further comprise one or more of a lubricant, a disintegrant, and a binder. In some embodiments, the core comprises difelikefalin, a delivery agent, an auxiliary agent, and one or more of a diluent, a lubricant, a disintegrant, and a binder.

In some embodiments, the outer layer can further comprise an auxiliary agent. In some embodiments, the outer layer can further comprise a diluent. In some embodiments, the outer layer can further comprise one or more of a lubricant, a disintegrant, and a binder. In some embodiments, the outer layer comprises a delivery agent and one or more of an auxiliary agent, a diluent, a lubricant, a disintegrant, and a binder.

In some embodiments, the pharmaceutical composition can comprise an outer layer and a core enclosed by the outer layer, wherein the outer layer does not comprise difelikefalin, the outer layer can comprise a delivery agent, an auxiliary agent, and optionally a pharmaceutically acceptable non-active ingredient, and the core can comprise difelikefalin and optionally a pharmaceutically acceptable non-active ingredient.

In some embodiments, the core can further comprise a delivery agent and/or an auxiliary agent. In some embodiments, the core can further comprise a delivery agent and an auxiliary agent. In some embodiments, the core can further comprise a lubricant.

In some embodiments, the core can further comprise a diluent.

In some embodiments, based on the mass of the core, the mass content of the diluent in the core is 5 wt%-86 wt%, 5 wt%-80 wt%, 5 wt%-75 wt%, 5 wt%-70 wt%, 5 wt%-75 wt%, 5 wt%-60 wt%, 15 wt%-60 wt%, 20 wt%-60 wt%, 5 wt%-40 wt%, 25 wt%-55 wt%, 10 wt%-35 wt%, 30 wt%-50 wt%, 15 wt%-30 wt%, 18 wt%-28 wt%, 35 wt%-45 wt%, 20 wt%-25 wt%, 25 wt%-30 wt%, 30 wt%-35 wt%, 35 wt%-40 wt%, 40 wt%-45 wt%, 45 wt%-50 wt%, 50 wt%-55 wt%, 55 wt%-60 wt%.

In some embodiments, based on the mass of the core, the mass content of the diluent in the core is 5 wt%-40 wt%, 10 wt%-35 wt%, 15 wt%-30 wt%, 18 wt%-28 wt%, 20 wt%-25 wt%.

In some embodiments, the core can further comprise a disintegrant and/or a binder.

In some embodiments, based on the mass of the core, the mass content of the disintegrant in the core is 0.5 wt%-10 wt%, 1 wt%-9.5 wt%, 1.5 wt%-9 wt%, 2 wt%-8.5 wt%, 2.5 wt%-8 wt%, 3 wt%-7.5 wt%, 3.5 wt%-7 wt%, 4 wt%-6.5 wt%, 4.5 wt%-6 wt%, 5 wt%-6.5 wt%, 5.5 wt%-7.5 wt%, 6 wt%-9 wt%, 1.5 wt%-6 wt%, 2 wt%-5.5 wt%, 2.5 wt%-5 wt%, 1.5 wt%-4.5 wt%.

In some embodiments, based on the mass of the core, the mass content of the disintegrant in the core is 2 wt%-10 wt%, 2 wt%-9.5 wt%, 2 wt%-9 wt%, 2 wt%-8.5 wt%, 2 wt%-8 wt%, 2 wt%-7.5 wt%, 2 wt%-7 wt%, 2 wt%-6.5 wt%, 2 wt%-6 wt%, 2 wt%-5.5 wt%, 2 wt%-5 wt%, 2 wt%-4.5 wt%, 2 wt%-4 wt%, 2 wt%-3.5 wt%, 2 wt%-3 wt%, or 2.5 wt%-3 wt%.

In some embodiments, based on the mass of the core, the mass content of the binder in the core is 0.5 wt%-7.5 wt%, 1 wt%-7 wt%, 1.5 wt%-6.5 wt%, 2 wt%-6 wt%, 2.5 wt%-5.5 wt%, 3 wt%-5 wt%, 2.5 wt%-4.5 wt%, 2 wt%-4 wt%, 1.5 wt%-3.5 wt%, 1 wt%-3 wt%, 0.5 wt%-2.5 wt%, 3.5 wt%-5.5 wt%, 4 wt%-6 wt%, 4.5 wt%-6.5 wt%, 5 wt%-7 wt%, 6.5 wt%-7.5 wt%.

In some embodiments, based on the mass of the core, the mass content of the binder in the core is 1 wt%-7.5 wt%, 1 wt%-7 wt%, 1.5 wt%-6.5 wt%, 1.5 wt%-6 wt%, 1.8 wt%-5.5 wt%, 1.8 wt%-5 wt%, 2 wt%-4.5 wt%, 2 wt%-4 wt%, 2 wt%-3.5 wt%, 2 wt%-3 wt%, 2 wt%-2.5 wt%, 1 wt%-2.5 wt%, 2.5 wt%-5.5 wt%, 3 wt%-6 wt%, 3.5 wt%-6.5 wt%, 4 wt%-6 wt%.

In some embodiments, the outer layer further comprises a diluent. In some embodiments, the outer layer further comprises a lubricant.

In some embodiments, the outer layer further comprises a disintegrant and/or a binder.

In some embodiments, the diluent is selected from any one or any combination of microcrystalline cellulose, lactose, mannitol and the like, and a compound excipient comprising any one or more of microcrystalline cellulose, lactose, mannitol and the like.

In some embodiments, the diluent can preferably be microcrystalline cellulose, and a microcrystalline cellulose-mannitol compound excipient.

In some embodiments, the disintegrant is selected from one or more of cross-linked povidone, croscarmellose sodium, low-substituted hydroxypropylcellulose, and carboxymethyl starch sodium, and the binder is selected from one or more of hydroxypropylcellulose, povidone, copovidone, hypromellose, sodium carboxymethylcellulose, and ethyl cellulose.

In some embodiments, the disintegrant can preferably be cross-linked povidone, and the binder can preferably be hydroxypropylcellulose.

The embodiments of the present application provide use of a pharmaceutical composition as described in any embodiment of the present disclosure in preparing a medicament for treating and/or preventing chronic kidney disease-associated pruritus, chronic liver disease-associated pruritus, and atopic dermatitis. Alternatively, the embodiments of the present application provide a method of treating and/or preventing chronic kidney disease-associated pruritus, chronic liver disease-associated pruritus, and atopic dermatitis in a patient in need thereof, comprising: administering to the patient a therapeutically effective amount of a pharmaceutical composition as described in any embodiment of the present disclosure.

The embodiments of the present application provide a method of treating and/or preventing chronic kidney disease-associated pruritus, chronic liver disease-associated pruritus, and atopic dermatitis, comprising orally administering a pharmaceutical composition as described in any embodiment of the present disclosure to a subject in need thereof.

The embodiments of the present application provide a pharmaceutical composition as described in any embodiment of the present disclosure for treating and/or preventing chronic kidney disease-associated pruritus, chronic liver disease-associated pruritus, and atopic dermatitis.

### EMBODIMENTS OF THE INVENTION

In order to more clearly illustrate the technical solutions of the present invention, the technical solutions of the present invention will be described using embodiments below. It should be understood that the description of the embodiments is only for facilitating the understanding of the technical solutions, rather than limiting the present invention to the specific embodiments shown. The headings in the present application are provided for convenience only and should not be construed as limiting the present invention in any way. Embodiments shown under any heading can be combined with embodiments shown under any other heading. For the skilled in the art, various modifications, improvements, and corrections can be made to these embodiments without paying any creative efforts. Therefore, such modifications, improvements, and corrections still fall within the spirit and scope of the exemplary embodiments of the present disclosure.

The present application provides a difelikefalin oral pharmaceutical composition. In some embodiments, the pharmaceutical preparation (i.e., oral pharmaceutical composition) can comprise difelikefalin, a delivery agent, and an auxiliary agent. In some embodiments, the pharmaceutical preparation can comprise difelikefalin, a delivery agent, an auxiliary agent, and optionally a pharmaceutically acceptable non-active ingredient. In some embodiments, the oral pharmaceutical composition can comprise difelikefalin, a delivery agent, an auxiliary agent, a diluent, and a lubricant. In some embodiments, the oral pharmaceutical composition can comprise difelikefalin, a delivery agent, an auxiliary agent, at least one of a disintegrant and a binder, a diluent, and a lubricant. In some embodiments, the oral pharmaceutical composition can comprise difelikefalin, a delivery agent, an auxiliary agent, a disintegrant, a diluent, and a lubricant. In some embodiments, the oral pharmaceutical composition can comprise difelikefalin, a delivery agent, an auxiliary agent, a disintegrant, a binder, a diluent, and a lubricant.

The term "difelikefalin" used in the present application refers to D-Phe-D-Phe-D-Leu-D-Lys-[ω(4-aminopiperidine-4-carboxylic acid)]-OH, also known as CR845. The difelikefalin useful in the present application can be used or prepared in alternative forms. For example, difelikefalin is used or prepared as a pharmaceutically acceptable salt. Usually, such a salt improves the separation and handling properties. For example, according to the reagents and reaction conditions, and the like, the difelikefalin described in the present application can be used or prepared in the form of a pharmaceutically acceptable acid salt, such as sulfate, citrate, acetate, oxalate, hydrochloride, hydrobromide, nitrate, phosphate, lactate, citrate,tartrate, or fumarate. Isomorphic crystalline forms, hydrates, solvates, and acid salt hydrates are also contemplated as being within the scope of the present application. CR845 is an agonist with a drug target as κ-opioid receptor. Conditions that can be alleviated by activating κ-opioid receptors can be treated or prevented by administering the pharmaceutical preparation of the present application, including conditions such as pain, inflammation, pruritus, hyponatremia, hypokalemia, congestive heart failure, liver cirrhosis, nephrotic syndrome, hypertension, edema, intestinal obstruction, cough, and glaucoma.

In some embodiments, the pharmaceutical composition of the present application can be used to treat or prevent chronic kidney disease-associated pruritus, chronic liver disease-associated pruritus, and atopic dermatitis.

In some embodiments, the pharmaceutical composition of the present application can be used for the preparation of a medicament for treating and/or preventing chronic kidney disease-associated pruritus, chronic liver disease-associated pruritus, and atopic dermatitis.

In some embodiments, the method of the present application for treating and/or preventing chronic kidney disease-associated pruritus, chronic liver disease-associated pruritus, and atopic dermatitis can comprise orally administering the pharmaceutical composition of the present application to a subject in need thereof. The term "treat" or "treating" in relation to a given disease or condition includes, but is not limited to, inhibiting the disease or condition, such as preventing the development of the disease or condition; alleviating the disease or condition, for example, causing regression of the disease or condition; or alleviating a condition caused by the disease or condition, for example, alleviating, preventing or treating a symptom of the disease or condition. The term "prevent" or "preventing" in relation to a given disease or condition means preventing the onset of the disease development if it has not occurred, preventing the disease or condition from occurring in a subject who may be susceptible to the disease or condition but has not yet been diagnosed as having the disease or condition, and/or preventing further disease/condition development if it already exists.

In some embodiments, the pharmaceutical composition of the present application can be co-administered with one or more other therapeutic compounds or adjuvants. The one or more other therapeutic compounds or adjuvants include, but are not limited to, other opioids, cannabinoids, antidepressants, anticonvulsants, tranquilizers, antihistamines, acetaminophen, corticosteroids, ion channel blockers, nonsteroidal anti-inflammatory drugs (NSAIDs), and diuretics, many of which have synergistic effects with the pharmaceutical composition of the present application.

In some embodiments, the pharmaceutical composition can be in the dosage form of tablets, pills, capsules, granules, oral solutions or suspensions, and the like. In some embodiments, the pharmaceutical composition can preferably be a tablet.

In some embodiments, the unit mass of the pharmaceutical composition can be from about 50 mg to about 2000 mg. In some embodiments, the unit mass of the pharmaceutical composition can preferably be from about 100 mg to about 600 mg.

In some embodiments, difelikefalin is present in the unit dosage form of the pharmaceutical composition of the present application in an amount of about 0.05 mg to about 30 mg. In some embodiments, difelikefalin is preferably present in the unit dosage form of the pharmaceutical composition of the present application in an amount of about 0.1 mg to about 10 mg.

In some embodiments, based on the total mass of the pharmaceutical composition, the mass content of difelikefalin can be about 0.01 wt% to about 10 wt%. In some embodiments, based on the total mass of the pharmaceutical composition, the mass content of difelikefalin can be about 0.01 wt% to about 10 wt%, about 0.01 wt% to about 9 wt%, about 0.01 wt% to about 8 wt%, about 0.01 wt% to about 7 wt%, about 0.01 wt% to about 6 wt%, about 0.01 wt% to about 5 wt%, about 0.01 wt% to about 4 wt%, about 0.01 wt% to about 3 wt%, about 0.01 wt% to about 2 wt%, about 0.01 wt% to about 1 wt%, about 0.05 wt% to about 10 wt%, about 0.08 wt% to about 10 wt%, about 0.1 wt% to about 10 wt%, about 0.2 wt% to about 10 wt%, about 0.5 wt% to about 10 wt%, about 1 wt% to about 10 wt%, about 2 wt% to about 10 wt%, about 3 wt% to about 10 wt%, about 4 wt% to about 10 wt%, about 5 wt% to about 10 wt%, about 6 wt% to about 10 wt%, about 7 wt% to about 10 wt%, about 8 wt% to about 10 wt%, about 9 wt% to about 10 wt%, about 1 wt% to about 9 wt%, about 1 wt% to about 8 wt%, about 2 wt% to about 7 wt%, about 2 wt% to about 6 wt%, about 2 wt% to about 5 wt%, about 2 wt% to about 4 wt%, about 2 wt% to about 3 wt%, about 3 wt% to about 6 wt%, about 3.0 wt% to about 5 wt%, about 3 wt% to about 4 wt%, or about 4 wt% to about 5 wt%. In some embodiments, based on the total mass of the pharmaceutical composition, the mass content of difelikefalin can be about 0.1 wt% to about 10 wt%. In some embodiments, based on the total mass of the pharmaceutical composition, the mass content of difelikefalin can be about 0.1 wt% to about 10 wt%, about 0.1 wt% to about 9 wt%, about 0.1 wt% to about 8 wt%, about 0.1 wt% to about 7 wt%, about 0.1 wt% to about 6 wt%, about 0.1 wt% to about 5 wt%, about 0.1 wt% to about 4 wt%, about 0.1 wt% to about 3 wt%, about 0.1 wt% to about 2 wt%, about 0.1 wt% to about 1 wt%, about 0.2 wt% to about 10 wt%, about 0.3 wt% to about 10 wt%, about 0.4 wt% to about 10 wt%, about 0.5 wt% to about 10 wt%, about 0.6 wt% to about 10 wt%, about 0.7 wt% to about 10 wt%, about 0.8 wt% to about 10 wt%, about 0.9 wt% to about 10 wt%, about 1 wt% to about 10 wt%, about 2 wt% to about 10 wt%, about 3 wt% to about 10 wt%, about 4 wt% to about 10 wt%, about 5 wt% to about 10 wt%, about 6 wt% to about 10 wt%, about 7 wt% to about 10 wt%, about 8 wt% to about 10 wt%, about 9 wt% to about 10 wt%, about 1 wt% to about 9 wt%, about 1 wt% to about 8 wt%, about 2 wt% to about 7 wt%, about 2 wt% to about 6 wt%, about 2 wt% to about 5 wt%, about 2 wt% to about 4 wt%, about 2 wt% to about 3 wt%, about 3 wt% to about 6 wt%, about 3.0 wt% to about 5 wt%, about 3 wt% to about 4 wt%, or about 4 wt% to about 5 wt%. In some embodiments, based on the total mass of the pharmaceutical composition, the mass content of difelikefalin can be about 0.05 wt% to about 5 wt%. In some embodiments, based on the total mass of the pharmaceutical composition, the mass content of difelikefalin can be about 0.05 wt% to about 5 wt%, about 0.05 wt% to about 4 wt%, about 0.05 wt% to about 3 wt%, about 0.05 wt% to about 2 wt%, about 0.05 wt% to about 1 wt%, about 0.06 wt% to about 5 wt%, about 0.07 wt% to about 5 wt%, about 0.08 wt% to about 5 wt%, about 0.09 wt% to about 5 wt%, about 0.1 wt% to about 5 wt%, about 0.2 wt% to about 4 wt%, about 0.3 wt% to about 3 wt%, about 0.4 wt% to about 2 wt%, about 0.5 wt% to about 1 wt%, about 0.5 wt% to about 5 wt%, about 0.5 wt% to about 4.5 wt%, about 1.0 wt% to about 4 wt%, about 1.5 wt% to about 3.5 wt%, or about 2.0 wt% to about 3 wt%. In some embodiments, based on the total mass of the pharmaceutical composition, the mass content of difelikefalin can be about 0.1 wt% to about 2 wt%. In some embodiments, based on the total mass of the pharmaceutical composition, the mass content of difelikefalin can be about 0.1 wt% to about 2.5 wt%. In some embodiments, based on the total mass of the pharmaceutical composition, the mass content of difelikefalin can be about 0.1 wt% to about 2.5 wt%, about 0.1 wt% to about 2.2 wt%, about 0.1 wt% to about 2 wt%, about 0.1 wt% to about 1.9 wt%, about 0.1 wt% to about 1.8 wt%, about 0.1 wt% to about 1.7 wt%, about 0.1 wt% to about 1.6 wt%, about 0.1 wt% to about 1.5 wt%, about 0.2 wt% to about 1.5 wt%, about 0.3 wt% to about 1.5 wt%, about 0.4 wt% to about 1.5 wt%, about 0.5 wt% to about 1.5 wt%, about 0.5 wt% to about 1.4 wt%, about 0.5 wt% to about 1.3 wt%, about 0.5 wt% to about 1.2 wt%, about 0.5 wt% to about 1.1 wt%, about 0.5 wt% to about 1.0 wt%, about 0.5 wt% to about 0.9 wt%, about 0.5 wt% to about 0.8 wt%, about 0.5 wt% to about 0.7 wt%, about 0.5 wt% to about 0.6 wt%, about 0.6 wt% to about 1.0 wt%, about 0.6 wt% to about 0.9 wt%, about 0.6 wt% to about 0.8 wt%, about 0.6 wt% to about 0.7 wt%, about 0.7 wt% to about 1.0 wt%, about 0.7 wt% to about 0.9 wt%, about 0.7 wt% to about 0.8 wt%, about 0.8 wt% to about 1.0 wt%, about 0.8 wt% to about 0.9 wt%, or about 0.9 wt% to about 1.0 wt%, about 1.0 wt% to about 2.5 wt%, about 1.5 wt% to about 2.5 wt%, about 1.5 wt% to about 2.2 wt%, about 1.8 wt% to about 2.5 wt%, about 1.8 wt% to about 2.2 wt%, about 2.0 wt% to about 2.5 wt%. In some embodiments, based on the total mass of the pharmaceutical composition, the mass content of difelikefalin can be about 0.1 wt% to about 1 wt%, about 0.2 wt% to about 0.9 wt%, about 0.25 wt% to about 2.5 wt%. In some embodiments, based on the total mass of the pharmaceutical composition, the mass content of difelikefalin in the core can be about 0.1 wt% to about 2.5 wt%, about 0.1 wt% to about 1 wt%, about 0.2 wt% to about 0.9 wt%, about 0.2 wt% to 2.5 wt%.

The "mass of difelikefalin" described in the present application refers to the mass of the free base of difelikefalin when the substance providing difelikefalin to the composition is a pharmaceutically acceptable salt of difelikefalin.

In some embodiments, the delivery agent can be selected from one or more of N-[8-(2-hydroxybenzoyl)amino]octanoic acid (NAC), N-[6-(2-hydroxybenzoyl)amino]hexanoic acid (NAH), N-[10-(2-hydroxybenzoyl)amino]decanoic acid (NAD), N-[8-(5-chlorosalicyloyl) amino]octanoic acid (5-CNAC), N-[8-(2-hydroxy-4-methoxybenzoyl)amino]octanoic acid (4-MOAC), N-[4-(2-hydroxy-4-chlorobenzoyl)amino]butyric acid (4-CNAB), and pharmaceutically acceptable salts thereof. In some embodiments, the delivery agent can be preferably selected from one or more of N-[8-(2-hydroxybenzoyl)amino]octanoic acid (NAC), N-[6-(2-hydroxybenzoyl)amino]hexanoic acid (NAH), N-[10-(2-hydroxybenzoyl)amino] decanoic acid (NAD), and pharmaceutically acceptable salts thereof. In some embodiments, the delivery agent can be preferably selected from one or more of N-[8-(2-hydroxybenzoyl)amino]octanoic acid (NAC) and pharmaceutically acceptable salts thereof. The pharmaceutically acceptable salts described above are selected from sodium salts, potassium salts or calcium salts. In some embodiments, the delivery agent is preferably sodium N-[8-(2-hydroxybenzoyl)amino]octanoate, hereinafter abbreviated as SNAC. In some embodiments, the delivery agent is preferably potassium N-[8-(2-hydroxybenzoyl) amino]octanoate. In some embodiments, the delivery agent is preferably calcium N-[8-(2-hydroxybenzoyl)amino]octanoate.

In some embodiments, the delivery agent can be present in the unit dosage form of the pharmaceutical composition of the present application in an amount of about 50 mg to about 500 mg. In some embodiments, the amount of the delivery agent can be about 60 mg to about 500 mg, about 70 mg to about 500 mg, about 80 mg to about 500 mg, about 90 mg to about 500 mg, about 100 mg to about 500 mg, about 100 mg to about 450 mg, about 100 mg to about 440 mg, about 100 mg to about 430 mg, about 100 mg to about 420 mg, about 100 mg to about 410 mg, about 100 mg to about 400 mg, about 110 mg to about 400 mg, about 120 mg to about 400 mg, about 130 mg to about 400 mg, about 140 mg to about 400 mg, about 150 mg to about 400 mg, about 160 mg to about 400 mg, about 170 mg to about 400 mg, about 180 mg to about 400 mg, about 190 mg to about 400 mg, or about 200 mg to about 400 mg. In some embodiments, the amount of the delivery agent can preferably be about 200 mg to about 400 mg, about 210 mg to about 390 mg, about 220 mg to about 380 mg, about 230 mg to about 370 mg, about 240 mg to about 360 mg, about 250 mg to about 350 mg, about 260 mg to about 340 mg, about 270 mg to about 330 mg, about 270 mg to about 320 mg, about 270 mg to about 310 mg, about 270 mg to about 300 mg, about 280 mg to about 300 mg, about 280 mg to about 310 mg, about 280 mg to about 320 mg, about 280 mg to about 330 mg, about 290 mg to about 330 mg, about 290 mg to about 320 mg, about 290 mg to about 310 mg, or about 290 mg to about 300 mg. In some embodiments, the amount of the delivery agent can preferably be about 200 mg to about 350 mg, about 200 mg to about 300 mg, about 200 mg to about 250 mg, about 200 mg to about 220 mg, about 200 mg to about 210 mg, about 210 mg to about 350 mg, about 210 mg to about 300 mg, about 210 mg to about 250 mg, about 210 mg to about 220 mg, about 250 mg to about 350 mg, about 250 mg to about 300 mg, about 300 mg to about 350 mg.

In some embodiments, based on the total mass of the pharmaceutical composition, the mass content of the delivery agent can be about 15 wt% to about 75 wt%. In some embodiments, based on the total mass of the pharmaceutical composition, the mass content of the delivery agent can be about 12 wt%-75 wt%, 12 wt%-70 wt%, 12 wt%-65 wt%, 12 wt%-60 wt%, 12 wt%-55 wt%, 12 wt%-50 wt%, 12 wt%-45 wt%, 12 wt%-40 wt%, 12 wt%-35 wt%, 12 wt%-30 wt%, 12 wt%-25 wt%, 12 wt%-20 wt%, 15 wt% to about 75 wt%, about 15 wt% to about 70 wt%, about 15 wt% to about 65 wt%, about 15 wt% to about 60 wt%, about 15 wt% to about 55 wt%, about 15 wt% to about 50 wt%, about 15 wt% to about 45 wt%, about 15 wt% to about 40 wt%, about 15 wt% to about 35 wt%, about 15 wt% to about 30 wt%, about 15 wt% to about 25 wt%, about 15 wt% to about 20 wt%, about 16 wt% to about 75 wt%, about 17 wt% to about 75 wt%, about 18 wt% to about 75 wt%, about 19 wt% to about 75 wt%, about 20 wt% to about 75 wt%, about 20 wt% to about 74 wt%, about 20 wt% to about 73 wt%, about 20 wt% to about 72 wt%, about 20 wt% to about 71 wt%, about 20 wt% to about 70 wt%, about 20 wt% to about 65 wt%, about 20 wt% to about 60 wt%, about 20 wt% to about 55 wt%, about 20 wt% to about 50 wt%, about 20 wt% to about 45 wt%, about 20 wt% to about 40 wt%, about 20 wt% to about 35 wt%, about 20 wt% to about 30 wt%, about 21 wt% to about 70 wt%, about 22 wt% to about 70 wt%, about 23 wt% to about 70 wt%, about 24 wt% to about 70 wt%, about 25 wt% to about 70 wt%, about 25 wt% to about 69 wt%, about 25 wt% to about 68 wt%, about 25 wt% to about 67 wt%, about 25 wt% to about 66 wt%, about 25 wt% to about 65 wt%, about 25 wt% to about 60 wt%, about 25 wt% to about 55 wt%, about 25 wt% to about 50 wt%, about 25 wt% to about 45 wt%, about 25 wt% to about 40 wt%, about 25 wt% to about 35 wt%, about 26 wt% to about 65 wt%, about 27 wt% to about 65 wt%, about 28 wt% to about 65 wt%, about 29 wt% to about 65 wt%, about 30 wt% to about 65 wt%, about 30 wt% to about 64 wt%, about 30 wt% to about 63 wt%, about 30 wt% to about 62 wt%, about 30 wt% to about 61 wt%, about 30 wt% to about 60 wt%, about 30 wt% to about 55 wt%, about 30 wt% to about 50 wt%, about 30 wt% to about 45 wt%, about 30 wt% to about 40 wt%, or about 30 wt% to about 35 wt%.

In some embodiments, based on the total mass of the pharmaceutical composition, the mass content of the delivery agent can preferably be about 30 wt% to about 60 wt%. In some embodiments, based on the total mass of the pharmaceutical composition, the mass content of the delivery agent can be about 31 wt% to about 60 wt%, about 32 wt% to about 60 wt%, about 33 wt% to about 60 wt%, about 34 wt% to about 60 wt%, about 35 wt% to about 60 wt%, about 35 wt% to about 59 wt%, about 35 wt% to about 58 wt%, about 35 wt% to about 57 wt%, about 35 wt% to about 56 wt%, about 35 wt% to about 55 wt%, about 35 wt% to about 50 wt%, about 35 wt% to about 45 wt%, about 35 wt% to about 40 wt%, about 36 wt% to about 55 wt%, about 37 wt% to about 55 wt%, about 38 wt% to about 55 wt%, about 39 wt% to about 55 wt%, about 40 wt% to about 55 wt%, about 40 wt% to about 54 wt%, about 40 wt% to about 53 wt%, about 40 wt% to about 52 wt%, about 40 wt% to about 51 wt%, about 40 wt% to about 50 wt%, about 40 wt% to about 49 wt%, about 40 wt% to about 48 wt%, about 40 wt% to about 47 wt%, about 40 wt% to about 46 wt%, about 40 wt% to about 45 wt%, about 40 wt% to about 44 wt%, about 40 wt% to about 43 wt%, about 40 wt% to about 42 wt%, about 45 wt% to about 60 wt%, about 45 wt% to about 59 wt%, about 45 wt% to about 58 wt%, about 45 wt% to about 57 wt%, about 45 wt% to about 56 wt%, about 45 wt% to about 55 wt%, about 45 wt% to about 54 wt%, about 45 wt% to about 53 wt%, about 45 wt% to about 52 wt%, about 45 wt% to about 51 wt%, about 45 wt% to about 50 wt%, about 45 wt% to about 49 wt%, about 45 wt% to about 48 wt%, about 45 wt% to about 47 wt%, about 45 wt% to about 57 wt%, about 50 wt% to about 60 wt%, about 50 wt% to about 59 wt%, about 50 wt% to about 58 wt%, about 50 wt% to about 57 wt%, about 50 wt% to about 56 wt%, about 50 wt% to about 55 wt%, about 50 wt% to about 54 wt%, about 50 wt% to about 53 wt%, about 50 wt% to about 52 wt%, about 55 wt% to about 60 wt%, about 55 wt% to about 59 wt%, about 55 wt% to about 58 wt%, or about 55 wt% to about 57 wt%.

In some embodiments, in the pharmaceutical composition of the present application, the mass ratio of the delivery agent to difelikefalin can be about 5:1 to about 500:1. In some embodiments, in the pharmaceutical composition of the present application, the mass ratio of the delivery agent to difelikefalin can be about 5:1 to about 500:1, about 10:1 to about 500:1, about 15:1 to about 500:1, about 20:1 to about 500:1, about 25:1 to about 500:1, about 30:1 to about 500:1, about 35:1 to about 500:1, about 40:1 to about 500:1, about 45:1 to about 500:1, about 50:1 to about 500:1, about 50:1 to about 490:1, about 50:1 to about 480:1, about 50:1 to about 470:1, about 50:1 to about 460:1, about 50:1 to about 450:1, about 50:1 to about 440:1, about 50:1 to about 430:1, about 50:1 to about 420:1, about 50:1 to about 410:1, about 50:1 to about 400:1, about 60:1 to about 400:1, about 70:1 to about 400:1, about 80:1 to about 400:1, about 90:1 to about 400:1, about 100:1 to about 400:1, about 100:1 to about 390:1, about 100:1 to about 380:1, about 100:1 to about 370:1, about 100:1 to about 360:1, about 100:1 to about 350:1, about 110:1 to about 350:1, about 120:1 to about 350:1, about 130:1 to about 350:1, about 140:1 to about 350:1, about 150:1 to about 350:1, about 150:1 to about 340:1, about 150:1 to about 330:1, about 150:1 to about 320:1, about 150:1 to about 310:1, about 150:1 to about 300:1, about 160:1 to about 300:1, about 170:1 to about 300:1, about 180:1 to about 300:1, about 190:1 to about 300:1, or about 200:1 to about 300:1.

In some embodiments, in the pharmaceutical composition of the present application, the mass ratio of the delivery agent to difelikefalin can preferably be about 10:1 to about 300:1, about 20:1 to about 270:1. In some embodiments, in the pharmaceutical composition of the present application, the mass ratio of the delivery agent to difelikefalin can preferably be about 200:1 to about 300:1. In some embodiments, in the pharmaceutical composition of the present application, the mass ratio of the delivery agent to difelikefalin can preferably be about 210:1 to about 300:1, about 220:1 to about 300:1, about 230:1 to about 300:1, about 240:1 to about 300:1, about 250:1 to about 300:1, about 260:1 to about 300:1, about 270:1 to about 300:1, about 280:1 to about 300:1, about 290:1 to about 300:1, about 210:1 to about 290:1, about 220:1 to about 290:1, about 230:1 to about 290:1, about 240:1 to about 290:1, about 250:1 to about 290:1, about 260:1 to about 290:1, about 270:1 to about 290:1, about 280:1 to about 290:1, about 210:1 to about 280:1, about 220:1 to about 280:1, about 230:1 to about 280:1, about 240:1 to about 280:1, about 250:1 to about 280:1, about 260:1 to about 280:1, about 270:1 to about 280:1, about 210:1 to about 270:1, about 220:1 to about 270:1, about 230:1 to about 270:1, about 240:1 to about 270:1, about 250:1 to about 270:1, about 260:1 to about 270:1, about 210:1 to about 260:1, about 220:1 to about 260:1, about 230:1 to about 260:1, about 240:1 to about 260:1, about 250:1 to about 260:1, about 210:1 to about 250:1, about 220:1 to about 250:1, about 230:1 to about 250:1, about 240:1 to about 250:1, about 210:1 to about 240:1, about 220:1 to about 240:1, about 230:1 to about 240:1, about 210:1 to about 230:1, about 220:1 to about 230:1, or about 210:1 to about 220:1.

In some embodiments, in the pharmaceutical composition of the present application, the mass ratio of the delivery agent to difelikefalin can preferably be about 20:1 to about 200:1. In some embodiments, in the pharmaceutical composition of the present application, the mass ratio of the delivery agent to difelikefalin can preferably be about about 25:1 to about 200:1, about 30:1 to about 200:1, about 35:1 to about 200:1, about 40:1 to about 200:1, about 50:1 to about 200:1, about 60:1 to about 200:1, about 70:1 to about 200:1, about 80:1 to about 200:1, about 90:1 to about 200:1, about 100:1 to about 200:1, about 20:1 to about 190:1, about 30:1 to about 190:1, about 40:1 to about 190:1, about 50:1 to about 190:1, about 60:1 to about 190:1, about 70:1 to about 190:1, about 80:1 to about 190:1, about 20:1 to about 180:1, about 30:1 to about 180:1, about 40:1 to about 180:1, about 50:1 to about 180:1, about 60:1 to about 180:1, about 70:1 to about 180:1, about 20:1 to about 170:1, about 30:1 to about 170:1, about 40:1 to about 170:1, about 50:1 to about 170:1, about 60:1 to about 170:1, about 20:1 to about 160:1, about 30:1 to about 160:1, about 40:1 to about 160:1, about 50:1 to about 160:1, about 20:1 to about 150:1, about 30:1 to about 150:1, about 40:1 to about 150:1, about 20:1 to about 140:1, about 30:1 to about 140:1, about 20:1 to about 130:1, about 20:1 to about 120:1, about 20:1 to about 100:1, about 20:1 to about 80:1, about 20:1 to about 60:1, about 20:1 to about 50:1, about 20:1 to about 40:1, or about 20:1 to about 30:1.

In some embodiments, based on the total mass of the pharmaceutical composition, the mass content of the delivery agent in the core can be about 8 wt% to about 50 wt%, about 10 wt% to about 45 wt%, about 10 wt% to about 40 wt%, about 11 wt% to about 40 wt%, about 12 wt% to about 35 wt%, about 12 wt% to about 32 wt%, about 10 wt% to about 32 wt%. In some embodiments, in the core, the mass ratio of the delivery agent to difelikefalin can be about 20:1 to about 150:1, about 30:1 to about 120:1, about 35:1 to about 110:1, about 36:1 to about 100:1.

In some embodiments, based on the total mass of the pharmaceutical composition, the mass content of the delivery agent in the outer layer can be about 15 wt% to about 55 wt%, about 18 wt% to about 50 wt%, about 20 wt% to about 45 wt%, about 22 wt% to about 45 wt%.

In some embodiments, in the pharmaceutical composition of the present application, the mass ratio of the delivery agent to difelikefalin can preferably be about 20:1 to about 50:1. In some embodiments, in the pharmaceutical composition of the present application, the mass ratio of the delivery agent to difelikefalin can preferably be about 200:1 to about 250:1. In some embodiments, in the pharmaceutical composition of the present application, the mass ratio of the delivery agent to difelikefalin can preferably be about 220:1 to about 250:1.

In some embodiments, the auxiliary agent can include, but is not limited to, phosphates (including hydrophosphates), tartrates, acetates, citrates, glutamates, carbonates (e.g., bicarbonates, such as bicarbonates containing aluminum and an alkaline earth metal), or any combination thereof. In some embodiments, the auxiliary agent can include, but is not limited to, any one or a combination of sodium phosphate, disodium hydrogen phosphate, sodium tartrate, sodium acetate, sodium citrate, sodium glutamate, sodium carbonate, anhydrous calcium hydrogen phosphate, hydrotalcite, and other pharmaceutically acceptable salts of the same acid radical. In some embodiments, the auxiliary agent can further comprise any one or a combination of anhydrous disodium hydrogen phosphate, anhydrous calcium hydrogen phosphate, anhydrous sodium carbonate, and other pharmaceutically acceptable salts of the same acid radical. Other pharmaceutically acceptable salts of the same acid radicals described above are selected from sodium salts, potassium salts or calcium salts. Compared with the oral preparation without an auxiliary agent, the oral preparation with an auxiliary agent can reduce the variability of the *in vivo* drug effectiveness (especially the maximum plasma concentration) of difelikefalin among the administered individuals.

In some embodiments, the auxiliary agent can be present in the unit dosage form of the pharmaceutical composition of the present application in an amount of about 10 mg to about 1500 mg. In some embodiments, the amount of the auxiliary agent can be about 10 mg to about 1000 mg, 20 mg to about 1000 mg, about 30 mg to about 800 mg, about 40 mg to about 600 mg, about 50 mg to about 500 mg, about 60 mg to about 400 mg, about 70 mg to about 300 mg, about 80 mg to about 200 mg, about 90 mg to about 100 mg. In some embodiments, the amount of the auxiliary agent can be about 50 mg to about 200 mg, about 50 mg to about 150 mg, about 50 mg to about 100 mg, about 55 mg to about 200 mg, about 55 mg to about 150 mg, about 55 mg to about 100 mg, about 60 mg to about 200 mg, about 60 mg to about 150 mg, about 60 mg to about 100 mg, about 70 mg to about 200 mg, about 70 mg to about 150 mg, about 70 mg to about 100 mg, about 70 mg to about 90 mg, about 70 mg to about 85 mg, about 70 mg to about 80 mg, about 75 mg to about 200 mg, about 75 mg to about 150 mg, about 75 mg to about 100 mg, or about 75 mg to about 90 mg.

In some embodiments, the unit dosage form of the pharmaceutical composition of the present application can comprise at least about 10 mg, at least 20 mg, at least about 30 mg, at least about 40 mg, at least about 50 mg, at least about 60 mg, at least about 70 mg, at least about 80 mg, at least about 90 mg, at least about 100 mg, at least about 110 mg, at least about 120 mg, at least about 130 mg, at least about 140 mg, at least about 150 mg, at least about 160 mg, at least about 170 mg, at least about 180 mg, at least about 190 mg, at least about 200 mg, at least about 220 mg, at least about 240 mg, at least about 260 mg, at least about 280 mg, at least about 300 mg, at least about 350 mg, at least about 400 mg, at least about 500 mg, at least about 750 mg, or at least about 1000 mg of the auxiliary agent. In some embodiments, the unit dosage form of the pharmaceutical composition of the present application can comprise no more than 200 mg, no more than 250 mg, no more than 300 mg, no more than 400 mg, no more than 500 mg, no more than 1000 mg, or no more than 1500 mg of the auxiliary agent.

In some embodiments, based on the total mass of the pharmaceutical composition, the mass content of the auxiliary agent can be about 5 wt% to about 70 wt%. In some embodiments, based on the total mass of the pharmaceutical composition, the mass content of the auxiliary agent can be about 5 wt% to about 65 wt%, about 5 wt% to about 60 wt%, about 5 wt% to about 55 wt%, about 5 wt% to about 50 wt%, about 5 wt% to about 45 wt%, about 5 wt% to about 40 wt%, about 5 wt% to about 35 wt%, about 6 wt% to about 70 wt%, about 7 wt% to about 70 wt%, about 8 wt% to about 70 wt%, about 9 wt% to about 70 wt%, about 10 wt% to about 70 wt%, about 10 wt% to about 69 wt%, about 10 wt% to about 68 wt%, about 10 wt% to about 67 wt%, about 10 wt% to about 66 wt%, about 10 wt% to about 65 wt%, about 11 wt% to about 65 wt%, about 12 wt% to about 65 wt%, about 13 wt% to about 65 wt%, about 14 wt% to about 65 wt%, about 15 wt% to about 65 wt%, about 15 wt% to about 64 wt%, about 15 wt% to about 63 wt%, about 15 wt% to about 62 wt%, about 15 wt% to about 61 wt%, about 15 wt% to about 60 wt%, about 16 wt% to about 60 wt%, about 17 wt% to about 60 wt%, about 18 wt% to about 60 wt%, about 19 wt% to about 60 wt%, about 20 wt% to about 60 wt%, about 20 wt% to about 55 wt%, about 20 wt% to about 50 wt%, about 20 wt% to about 45 wt%, about 20 wt% to about 40 wt%, about 25 wt% to about 40 wt%, about 25 wt% to about 35 wt%, or about 30 wt% to about 35 wt%.

In some embodiments, based on the total mass of the pharmaceutical composition, the mass content of the auxiliary agent can preferably be about 10 wt% to about 40 wt%, about 6 wt% to about 25 wt%. In some embodiments, based on the total mass of the pharmaceutical composition, the mass content of the auxiliary agent can preferably be about 11 wt% to about 40 wt%, about 12 wt% to about 40 wt%, about 13 wt% to about 40 wt%, about 14 wt% to about 40 wt%, about 15 wt% to about 40 wt%, about 15 wt% to about 39 wt%, about 15 wt% to about 38 wt%, about 15 wt% to about 37 wt%, about 15 wt% to about 36 wt%, about 15 wt% to about 35 wt%, about 16 wt% to about 35 wt%, about 17 wt% to about 35 wt%, about 18 wt% to about 35 wt%, about 19 wt% to about 35 wt%, about 20 wt% to about 35 wt%, about 20 wt% to about 34 wt%, about 20 wt% to about 33 wt%, about 20 wt% to about 32 wt%, about 20 wt% to about 31 wt%, about 20 wt% to about 30 wt%, about 21 wt% to about 30 wt%, about 22 wt% to about 30 wt%, about 23 wt% to about 30 wt%, about 24 wt% to about 30 wt%, about 25 wt% to about 30 wt%, about 25 wt% to about 29 wt%, about 25 wt% to about 28 wt%, about 25 wt% to about 27 wt%, about 10 wt% to about 35 wt%, about 10 wt% to about 30 wt%, about 10 wt% to about 25 wt%, about 10 wt% to about 20 wt%, about 10 wt% to about 15 wt%, about 15 wt% to about 30 wt%, about 15 wt% to about 25 wt%, about 15 wt% to about 20 wt%, about 20 wt% to about 40 wt%, about 20 wt% to about 25 wt%, about 25 wt% to about 40 wt%, about 25 wt% to about 35 wt%, about 30 wt% to about 40 wt%, about 30 wt% to about 35 wt%, or about 35 wt% to about 40 wt%. In some embodiments, based on the total mass of the pharmaceutical composition, the mass content of the auxiliary agent can preferably be about 10 wt% to about 30 wt%, about 12 wt% to about 30 wt%, about 13 wt% to about 30 wt%, about 14 wt% to about 30 wt%, about 15 wt% to about 30 wt%, about 15 wt% to about 29 wt%, about 15 wt% to about 28 wt%, about 15 wt% to about 27 wt%, about 15 wt% to about 26 wt%, about 15 wt% to about 25 wt%, about 16 wt% to about 25 wt%, about 17 wt% to about 25 wt%, about 18 wt% to about 25 wt%, about 18 wt% to about 24 wt%, about 18 wt% to about 23 wt%, about 18 wt% to about 22 wt%, about 18 wt% to about 21 wt%, about 18 wt% to about 20 wt%, about 19 wt% to about 25 wt%, about 20 wt% to about 25 wt%, about 20 wt% to about 24 wt%, about 20 wt% to about 23 wt%, about 20 wt% to about 22 wt%, about 20 wt% to about 21 wt%, about 21 wt% to about 30 wt%, about 22 wt% to about 30 wt%, about 23 wt% to about 30 wt%, about 24 wt% to about 30 wt%, about 25 wt% to about 30 wt%, about 25 wt% to about 29 wt%, about 25 wt% to about 28 wt%, about 25 wt% to about 27 wt%, about 10 wt% to about 25 wt%, about 10 wt% to about 20 wt%, about 10 wt% to about 19 wt%, about 10 wt% to about 18 wt%, about 10 wt% to about 16 wt%, about 10 wt% to about 15 wt%, about 10 wt% to about 20 wt%, about 10 wt% to about 15 wt%, about 15 wt% to about 30 wt%, about 15 wt% to about 25 wt%, about 15 wt% to about 20 wt%, about 20 wt% to about 30 wt%, about 20 wt% to about 25 wt%, or about 25 wt% to about 30 wt%. In some embodiments, based on the total mass of the pharmaceutical composition, the mass content of the auxiliary agent can preferably be about 25 wt% to about 30 wt%.

In some embodiments, based on the total mass of the pharmaceutical composition, the mass content of the auxiliary agent in the core can be about 0 wt% to about 20 wt%, about 2 wt% to about 20 wt%, about 3 wt% to about 18 wt%, about 4 wt% to about 15 wt%, about 4 wt% to about 14 wt%, about 0 wt% to about 18 wt%, about 0 wt% to about 15 wt%, about 0 wt% to about 13 wt%, about 4 wt% to about 13 wt%.

In some embodiments, based on the total mass of the pharmaceutical composition, the mass content of the auxiliary agent in the outer layer can be 0 wt% to about 20 wt%, about 2 wt% to about 20 wt%, about 3 wt% to about 19 wt%, about 5 wt% to about 18 wt%, about 0 wt% to about 19 wt%, about 0 wt% to about 18 wt%, about 0 wt% to about 13 wt%.

In some embodiments, in the pharmaceutical composition of the present application, the mass ratio of the delivery agent to the auxiliary agent can be about 0.5:1 to about 10:1. In some embodiments, in the pharmaceutical composition of the present application, the mass ratio of the delivery agent to the auxiliary agent can be about 1:1 to about 10:1, about 1:1 to about 9:1, about 1:1 to about 8:1, about 1:1 to about 7:1, about 1:1 to about 6:1, about 1:1 to about 5:1, about 1:1 to about 4:1, about 1:1 to about 3:1, about 1:1 to about 2:1, about 2:1 to about 5:1, about 2:1 to about 4:1, or about 2:1 to about 3:1.

In some embodiments, in the pharmaceutical composition of the present application, the mass ratio of the delivery agent to the auxiliary agent can preferably be about 1:1 to about 3:1. In some embodiments, in the pharmaceutical composition of the present application, the mass ratio of the delivery agent to the auxiliary agent can be preferably about 1:1 to about 3:1, about 1.1:1 to about 2.9:1, about 1.2:1 to about 2.8:1, about 1.3:1 to about 2.7:1, about 1.4:1 to about 2.6:1, about 1.5:1 to about 2.5:1, about 1.6:1 to about 2.4:1, about 1.7:1 to about 2.3:1, about 1.8:1 to about 2.2:1, about 1.9:1 to about 2.1:1, about 2:1 to about 3:1, about 2:1 to about 2.9:1, about 2:1 to about 2.8:1, about 2:1 to about 2.7:1, about 2:1 to about 2.6:1, about 2:1 to about 2.5:1, about 2:1 to about 2.4:1, about 2:1 to about 2.3:1, about 2:1 to about 2.2:1, about 2:1 to about 2.1:1, about 2.5:1 to about 3:1, about 2.5:1 to about 2.9:1, about 2.5:1 to about 2.8:1, about 2.5:1 to about 2.7:1, about 2.5:1 to about 2.6:1, about 2.7:1 to about 3:1, about 2.7:1 to about 2.9:1, about 2.7:1 to about 2.8:1. In some embodiments, in the pharmaceutical composition of the present application, the mass ratio of the delivery agent to the auxiliary agent can be preferably about 1:1 to about 2.5:1, about 1:1 to about 2.0:1, about 1:1 to about 1.5:1, about 1.1:1 to about 1.5:1, about 1.2:1 to about 1.5:1, about 1.3:1 to about 1.5:1, about 1.4:1 to about 1.5:1, about 1.5:1 to about 2.5:1, about 1.5:1 to about 2.0:1, about 1.6:1 to about 2.0:1, about 1.7:1 to about 2.0:1, about 1.8:1 to about 2.0:1, about 1.9:1 to about 2.0:1, about 2.0:1 to about 2.5:1, about 2.0:1 to about 2.4:1, about 2.0:1 to about 2.3:1, about 2.0:1 to about 2.2:1, about 2.0:1 to about 2.1:1.

In some embodiments, in the pharmaceutical composition of the present application, the molar ratio of the delivery agent to the auxiliary agent can be about 0.2:1 to about 6:1. In some embodiments, in the pharmaceutical composition of the present application, the molar ratio of the delivery agent to the auxiliary agent can be about 0.5:1 to about 6:1, about 1:1 to about 6:1, about 1:1 to about 5:1, about 1:1 to about 4:1, about 1:1 to about 3:1, about 1:1 to about 2:1, about 2:1 to about 5:1, about 2:1 to about 4:1, about 2:1 to about 3:1, about 3:1 to about 6:1, about 3:1 to about 5:1, about 3:1 to about 4:1, about 4:1 to about 6:1, about 4:1 to about 5:1, or about 5:1 to about 6:1. In some embodiments, in the pharmaceutical composition of the present application, the molar ratio of the delivery agent to the auxiliary agent can preferably be about 0.5:1 to about 3:1. In some embodiments, in the pharmaceutical composition of the present application, the molar ratio of the delivery agent to the auxiliary agent can preferably be about 0.5:1 to about 3:1, about 0.5:1 to about 2.5:1, about 0.5:1 to about 2:1, about 0.5:1 to about 1.5:1, about 0.5:1 to about 1:1, about 0.6:1 to about 1:1, about 0.7:1 to about 1:1, about 0.8:1 to about 1:1, about 0.9:1 to about 1:1, about 1:1 to about 3:1, about 1:1 to about 2.5:1, about 1:1 to about 2:1, about 1:1 to about 1.5:1, about 1.1:1 to about 1.5:1, about 1.2:1 to about 1.5:1, about 1.3:1 to about 1.5:1, about 1.4:1 to about 1.5:1, about 1.5:1 to about 3:1, about 1.5:1 to about 2.5:1, about 1.5:1 to about 2:1, about 1.6:1 to about 2:1, about 1.7:1 to about 2:1, about 1.8:1 to about 2:1, about 1.9:1 to about 2:1, about 2:1 to about 3:1, about 2:1 to about 2.5:1, about 2.1:1 to about 2.5:1, about 2.2:1 to about 2.5:1, about 2.3:1 to about 2.5:1, about 2.4:1 to about 2.5:1, about 2.5:1 to about 3:1, about 2.6:1 to about 3:1, about 2.7:1 to about 3:1, about 2.8:1 to about 3:1, or about 2.9:1 to about 3:1.

The "pharmaceutically acceptable non-active ingredients" described in the present application refers to other pharmaceutically acceptable non-active ingredients other than the delivery agent and the auxiliary agent described in the present application, comprising diluents, disintegrants, binders, lubricants, protease inhibitors, glidants, wetting agents, flavoring agents, spices, sweetening agents, coloring agents, fillers, granulating agents, anti-caking agents and preservatives. The skilled in the art can make routine selections and adjustments based on the requirements of the formability of the preparation.

In some embodiments, the pharmaceutical composition described in the present application can further comprise one or more non-active ingredients selected from diluents, disintegrants, binders, and lubricants. In some embodiments, based on the total mass of the pharmaceutical composition, the mass content of the one or more non-active ingredients described above can be about 0.1 wt% to about 60 wt%. In some embodiments, based on the total mass of the pharmaceutical composition, the mass content of the one or more non-active ingredients described above can be about 0.1 wt% to about 81 wt%. In some embodiments, based on the total mass of the pharmaceutical composition, the mass content of the one or more non-active ingredients described above can be about 10 wt% to about 50 wt%, about 20 wt% to about 60 wt%, about 24 wt% to about 37 wt%. In some embodiments, based on the total mass of the pharmaceutical composition, the mass content of the one or more non-active ingredients described above in the core can be about 5 wt% to about 30 wt%, about 6 wt% to about 25 wt%, about 8 wt% to about 20 wt%, about 8 wt% to about 18 wt%. In some embodiments, based on the total mass of the pharmaceutical composition, the mass content of the one or more non-active ingredients described above in the outer layer can be about 5 wt% to about 35 wt%, about 8 wt% to about 30 wt%, about 10 wt% to about 30 wt%, about 12 wt% to about 28 wt%.

The term "diluent" as used in the present disclosure refers to any pharmaceutically acceptable diluent that can be used to practice the present disclosure. Examples of pharmaceutically acceptable diluents include, but are not limited to, talc, calcium carbonate, calcium hydrogen phosphate, microcrystalline cellulose, powdered cellulose, kaolin, mannitol, silicic acid, sorbitol, starch, pregelatinized starch, lactose, glucose, fructose, galactose, trehalose, sucrose, maltose, maltodextrin, cross-linked povidone, and mixtures thereof. In some embodiments, exemplary diluents can include, but are not limited to, microcrystalline cellulose, lactose, mannitol, calcium hydrogen phosphate, maltodextrin, pregelatinized starch, and the like, and any one or any combination of compound excipients comprising any one or more of microcrystalline cellulose, lactose, mannitol, calcium hydrogen phosphate, maltodextrin, pregelatinized starch, and the like. Exemplary compound excipients can include, but are not limited to, microcrystalline cellulose-calcium hydrogen phosphate compound excipients, microcrystalline cellulose-povidone compound excipients, microcrystalline cellulose-cross-linked povidone compound excipients, microcrystalline cellulose-mannitol compound excipients, microcrystalline cellulose-lactose compound excipients, microcrystalline cellulose-pregelatinized starch compound excipients, pregelatinized starch-calcium hydrogen phosphate compound excipients, pregelatinized starch-povidone compound excipients, pregelatinized starch-cross-linked povidone compound excipients, pregelatinized starch-mannitol compound excipients, pregelatinized starch-lactose compound excipients, and the like. In some embodiments, exemplary diluents can include, but are not limited to, any one or more of microcrystalline cellulose, powdered cellulose, maltodextrin, mannitol, and microcrystalline cellulose-mannitol compound excipients. In some embodiments, based on the total mass of the pharmaceutical composition, the mass content of the diluent can be about 20 wt% to about 60 wt%, about 16 wt% to about 32 wt%. In some embodiments, based on the total mass of the pharmaceutical composition, the mass content of the diluent in the core can be about 0 wt% to about 20 wt%, about 0 wt% to about 15 wt%, about 5 wt% to about 20 wt%, about 6 wt% to about 15 wt%. In some embodiments, based on the total mass of the pharmaceutical composition, the mass content of the diluent in the outer layer can be about 5 wt% to about 30 wt%, about 8 wt% to about 26 wt%. In some embodiments, the diluent in the pharmaceutical composition can comprise microcrystalline cellulose. In some embodiments, the diluent in the pharmaceutical composition can comprise microcrystalline cellulose and mannitol. In some embodiments, the diluent in the pharmaceutical composition can comprise a microcrystalline cellulose-mannitol compound excipient.

The term "disintegrant" as used in the present disclosure refers to any pharmaceutically acceptable disintegrant that can be used in the practice of the present disclosure. Examples of pharmaceutically acceptable disintegrants include, but are not limited to, agar, calcium carbonate, microcrystalline cellulose, croscarmellose sodium, low-substituted hydroxypropylcellulose, carboxymethyl starch sodium, cross-linked povidone, povidone, polacrilin potassium, sodium starch glycolate, potato or cassava starch, other starches, pregelatinized starch, clays, other celluloses, gums, or mixtures thereof. In some embodiments, exemplary disintegrants can include, but are not limited to, any one or any combination of cross-linked povidone, croscarmellose sodium, low-substituted hydroxypropylcellulose, carboxymethyl starch sodium, and the like. In some embodiments, exemplary disintegrants can include, but are not limited to, cross-linked povidone, or mixtures thereof. In some embodiments, the pharmaceutical composition described in the present application may not comprise a disintegrant. In some embodiments, the pharmaceutical composition described in the present application can comprise a disintegrant. In some embodiments, based on the total mass of the pharmaceutical composition, the mass content of the disintegrant can be about 0.5 wt% to about 10 wt%, about 0.5 wt% to about 4.4 wt%. In some embodiments, based on the total mass of the pharmaceutical composition, the mass content of the disintegrant in the core can be about 0 wt% to about 5 wt%, about 0 wt% to about 2 wt%, about 0.8 wt% to about 2 wt%, about 0 wt% to about 1.5 wt%. In some embodiments, based on the total mass of the pharmaceutical composition, the mass content of the disintegrant in the outer layer can be about 0 wt% to about 5 wt%, about 0 wt% to about 4 wt%, about 0.8 wt% to about 4 wt%. In some embodiments, the disintegrant in the pharmaceutical composition can comprise cross-linked povidone.

The term "binder" as used in the present disclosure refers to any pharmaceutically acceptable binder that can be used in the practice of the present disclosure. Examples of pharmaceutically acceptable binders include, but are not limited to, starches (e.g., corn starch, potato starch, pregelatinized starch, and other starches), maltodextrin, gelatin, natural and synthetic gums such as gum arabic, guar gum, cellulose and the derivative thereof (e.g., methylcellulose, hydroxyethylcellulose, hydroxyethylmethylcellulose, hydroxypropylcellulose, and hydroxypropylmethylcellulose, ethylcellulose, cellulose acetate, carboxymethylcellulose calcium, sodium carboxymethylcellulose, carboxymethylcellulose, and microcrystalline cellulose), polyvinyl alcohol, polyvinyl pyrrolidone, and mixtures thereof. In some embodiments, exemplary binders can include, but are not limited to, any one or any combination of povidone, copovidone, hydroxypropylcellulose, hypromellose, sodium carboxymethylcellulose, ethyl cellulose, and the like. In some embodiments, the pharmaceutical composition described in the present application may not comprise a binder. In some embodiments, the pharmaceutical composition described in the present application can comprise a binder. In some embodiments, based on the total mass of the pharmaceutical composition, the mass content of the binder can be about 0.1 wt% to about 4 wt%, about 0.5 wt% to about 4.5 wt%. In some embodiments, based on the total mass of the pharmaceutical composition, the mass content of the binder in the core can be about 0 wt% to about 5 wt%, about 0 wt% to about 3 wt%, about 0 wt% to about 2.5 wt%. In some embodiments, based on the total mass of the pharmaceutical composition, the mass content of the binder in the outer layer can be about 0 wt% to about 5 wt%, about 0 wt% to about 3 wt%, about 0 wt% to about 2.5 wt%. In some embodiments, the binder in the pharmaceutical composition can comprise povidone (e.g., povidone K90). In some embodiments, the binder in the pharmaceutical composition can comprise hydroxypropylcellulose.

The term "lubricant" as used in the present disclosure refers to any pharmaceutically acceptable lubricant that can be used in the practice of the present disclosure. Examples of pharmaceutically acceptable lubricants include, but are not limited to, calcium stearate, magnesium stearate, mineral oil, light mineral oil, glycerol, sorbitol, mannitol, polyethylene glycol, sodium stearyl fumarate, glyceryl behenate, other glycols, stearic acid, sodium lauryl sulfate, talc, hydrogenated vegetable oils (e.g., peanut oil, cottonseed oil, sunflower seed oil, sesame oil, olive oil, corn oil, and soybean oil), zinc stearate, ethyl oleate, ethyl laurate, agar, Syloid^{®} silica gel, and mixtures thereof. In some embodiments, exemplary lubricants can include, but are not limited to, any one or any combination of magnesium stearate, stearic acid, sodium stearyl fumarate, glyceryl behenate, polyethylene glycol, and the like. In some embodiments, exemplary lubricants can include, but are not limited to, magnesium stearate, stearic acid, calcium stearate, or any combination thereof. In some embodiments, the pharmaceutical composition described in the present application may not comprise a lubricant. In some embodiments, the pharmaceutical composition described in the present application can comprise a lubricant. In some embodiments, based on the total mass of the pharmaceutical composition, the mass content of the lubricant can be about 1 wt% to about 6 wt%. In some embodiments, based on the total mass of the pharmaceutical composition, the mass content of the lubricant in the core can be about 0 wt% to about 3 wt%, about 0 wt% to about 1.5 wt%, about 0 wt% to about 1.4 wt%. In some embodiments, based on the total mass of the pharmaceutical composition, the mass content of the lubricant in the outer layer can be about 0 wt% to about 3 wt%, about 0 wt% to about 2.5 wt%, about 0 wt% to about 2 wt%. In some embodiments, the lubricant in the pharmaceutical composition can comprise magnesium stearate.

In some embodiments, based on the total mass of the pharmaceutical composition, the mass content of difelikefalin in the core can be about 0.1 wt% to about 1 wt%, about 0.2 wt% to about 0.9 wt%.

In some embodiments, the pharmaceutical composition described in the present application can further comprise a protease inhibitor. Exemplary protease inhibitors can include, but are not limited to, any one or any combination of casein hydrolysate, disodium edetate, pepstatin, trypsin inhibitor, and the like. In some embodiments, based on the total mass of the pharmaceutical composition, the mass content of the protease inhibitor can be 0.2 wt% to 10 wt%.

In some embodiments, the pharmaceutical composition described in the present application can further comprise a glidant. Exemplary glidants can include, but are not limited to, any one or any combination of colloidal silica, talc, and the like. In some embodiments, based on the total mass of the pharmaceutical composition, the mass content of the glidant can be about 0.5 wt% to about 5 wt%.

In some embodiments, the pharmaceutical composition described in the present application can further comprise a wetting agent. Exemplary wetting agents can include, but are not limited to, any one or any combination of polyethylene glycol, poloxamer, phospholipid, Tween 80, Span 40, propylene glycol monolaurate, sodium dodecyl sulfate, and the like. In some embodiments, based on the total mass of the pharmaceutical composition, the mass content of the wetting agent can be 0.2 wt% to 2 wt%.

In some embodiments, the pharmaceutical composition described in the present application can further comprise a flavoring agent. Exemplary flavoring agents can include, but are not limited to, breath-freshening compounds such as menthol, spearmint, and cinnamon, flavoring matters such as fruit flavoring agents (e.g., cherry, orange, grape, and the like), active substances for cleaning teeth and oral cavity (e.g., quaternary ammonium bases), and sweetening agents. Exemplary sweetening agents can include, but are not limited to, one or more artificial sweetening agents, one or more natural sweetening agents, or a combination thereof. Artificial sweetening agents comprise, for example, acesulfame and various salts thereof, aspartame, neotame, sodium cyclamate, saccharin and various salts thereof, stevia, neohesperidin dihydrochalcone, mogroside, and chlorine derivatives of sucrose such as sucralose. Natural sweetening agents comprise, for example, glucose, dextrose, invert sugar, fructose, sucrose, glycyrrhizin, monoammonium glycyrrhizate, stevioside, natural enhanced sweetening agents such as fructus momordicae, polyols such as sorbitol, mannitol, xylitol, erythritol, and the like.

In some embodiments, the pharmaceutical composition described in the present application can comprise difelikefalin, a pharmaceutically acceptable salt of NAC, an auxiliary agent, and a diluent. In some embodiments, based on the total mass of the pharmaceutical composition, the mass content of difelikefalin can be about 0.1 wt% to about 10 wt%, the mass content of the pharmaceutically acceptable salt of NAC can be about 15 wt% to about 75 wt%, the mass content of the auxiliary agent can be about 5 wt% to about 70 wt%, and the mass content of the diluent can be about 20 wt% to about 60 wt%. In some embodiments, based on the total mass of the pharmaceutical composition, the mass content of difelikefalin can be about 0.1 wt% to about 10 wt%, the mass content of the pharmaceutically acceptable salt of NAC can be about 12 wt% to about 75 wt%, the mass content of the auxiliary agent can be about 5 wt% to about 70 wt%, and the mass content of the diluent can be about 8 wt% to about 80 wt%. In some embodiments, based on the total mass of the pharmaceutical composition, the mass content of difelikefalin can be about 0.1 wt% to about 2 wt%, the mass content of the pharmaceutically acceptable salt of NAC can be about 15 wt% to about 75 wt%, the mass content of the auxiliary agent can be about 5 wt% to about 70 wt%, and the mass content of the diluent can be about 20 wt% to about 60 wt%. In some embodiments, the weight ratio of difelikefalin: the pharmaceutically acceptable salt of NAC: auxiliary agent: diluent can be 1: (5-500): (2-300): (5-200). In some embodiments, the weight ratio of difelikefalin: the pharmaceutically acceptable salt of NAC: auxiliary agent: diluent can be 1: (50-300): (50-200): (50-200). In some embodiments, the weight ratio of difelikefalin: the pharmaceutically acceptable salt of NAC: auxiliary agent: diluent can be 1: (200-300): (100-150): (50-100). In some embodiments, the pharmaceutically acceptable salt of NAC can be NAC sodium salt, NAC potassium salt or NAC calcium salt. In some embodiments, the auxiliary agent can be disodium hydrogen phosphate. In some embodiments, the diluent can comprise microcrystalline cellulose. In some embodiments, the diluent can comprise microcrystalline cellulose and mannitol. In some embodiments, the diluent can comprise microcrystalline cellulose and a microcrystalline cellulose-mannitol compound excipient.

In some embodiments, the pharmaceutical composition described in the present application can comprise difelikefalin, a pharmaceutically acceptable salt of NAC, an auxiliary agent, a diluent, and a lubricant. In some embodiments, based on the total mass of the pharmaceutical composition, the mass content of difelikefalin can be about 0.1 wt% to about 10 wt%, the mass content of the pharmaceutically acceptable salt of NAC can be about 15 wt% to about 75 wt%, the mass content of the auxiliary agent can be about 5 wt% to about 70 wt%, the mass content of the diluent can be about 20 wt% to about 60 wt%, and the mass content of the lubricant can be about 1 wt% to about 6 wt%. In some embodiments, based on the total mass of the pharmaceutical composition, the mass content of difelikefalin can be about 0.1 wt% to about 10 wt%, the mass content of the pharmaceutically acceptable salt of NAC can be about 12 wt% to about 75 wt%, the mass content of the auxiliary agent can be about 5 wt% to about 70 wt%, the mass content of the diluent can be about 8 wt% to about 80 wt%, and the mass content of the lubricant can be about 1 wt% to about 6 wt%. In some embodiments, based on the total mass of the pharmaceutical composition, the mass content of difelikefalin can be about 0.1 wt% to about 2 wt%, the mass content of the pharmaceutically acceptable salt of NAC can be about 15 wt% to about 75 wt%, the mass content of the auxiliary agent can be about 5 wt% to about 70 wt%, the mass content of the diluent can be about 20 wt% to about 60 wt%, and the mass content of the lubricant can be about 1 wt% to about 6 wt%. In some embodiments, the weight ratio of difelikefalin: the pharmaceutically acceptable salt of NAC: auxiliary agent: diluent: lubricant can be 1: (5-500): (2-300): (5-200): (2-20). In some embodiments, the weight ratio of difelikefalin: the pharmaceutically acceptable salt of NAC: auxiliary agent: diluent: lubricant can be 1: (50-300): (50-200): (50-200): (2-20). In some embodiments, the weight ratio of difelikefalin: the pharmaceutically acceptable salt of NAC: auxiliary agent: diluent: lubricant can be 1: (200-300): (100-150): (50-100): (3-10). In some embodiments, the pharmaceutically acceptable salt of NAC can be NAC sodium salt, NAC potassium salt or NAC calcium salt. In some embodiments, the auxiliary agent can be disodium hydrogen phosphate. In some embodiments, the diluent can comprise microcrystalline cellulose. In some embodiments, the diluent can comprise microcrystalline cellulose and mannitol. In some embodiments, the diluent can comprise microcrystalline cellulose and a microcrystalline cellulose-mannitol compound excipient. In some embodiments, the lubricant can comprise magnesium stearate.

In some embodiments, the pharmaceutical composition described in the present application can comprise difelikefalin, a pharmaceutically acceptable salt of NAC, an auxiliary agent, a diluent, a lubricant, and a binder. In some embodiments, based on the total mass of the pharmaceutical composition, the mass content of difelikefalin can be about 0.1 wt% to about 10 wt%, the mass content of the pharmaceutically acceptable salt of NAC can be about 15 wt% to about 75 wt%, the mass content of the auxiliary agent can be about 5 wt% to about 70 wt%, the mass content of the diluent can be about 20 wt% to about 60 wt%, the mass content of the lubricant can be about 1 wt% to about 6 wt%, and the mass content of the binder can be about 0.1 wt% to about 4 wt%. In some embodiments, based on the total mass of the pharmaceutical composition, the mass content of difelikefalin can be about 0.1 wt% to about 10 wt%, the mass content of the pharmaceutically acceptable salt of NAC can be about 12 wt% to about 75 wt%, the mass content of the auxiliary agent can be about 5 wt% to about 70 wt%, the mass content of the diluent can be about 8 wt% to about 80 wt%, the mass content of the lubricant can be about 1 wt% to about 6 wt%, and the mass content of the binder can be about 0.1 wt% to about 4 wt%. In some embodiments, the pharmaceutical composition described in the present application can comprise difelikefalin, a pharmaceutically acceptable salt of NAC, an auxiliary agent, a diluent, a lubricant, and a binder. In some embodiments, based on the total mass of the pharmaceutical composition, the mass content of difelikefalin can be about 0.1 wt% to about 2 wt%, the mass content of the pharmaceutically acceptable salt of NAC can be about 15 wt% to about 75 wt%, the mass content of the auxiliary agent can be about 5 wt% to about 70 wt%, the mass content of the diluent can be about 20 wt% to about 60 wt%, the mass content of the lubricant can be about 1 wt% to about 6 wt%, and the mass content of the binder can be about 0.1 wt% to about 4 wt%. In some embodiments, the weight ratio of difelikefalin: the pharmaceutically acceptable salt of NAC: auxiliary agent: diluent: lubricant: binder can be 1: (5-500): (2-300): (5-200): (2-20): (0.5-20). In some embodiments, the weight ratio of difelikefalin: the pharmaceutically acceptable salt of NAC: auxiliary agent: diluent: lubricant: binder can be 1: (50-300): (50-200): (50-200): (5-20): (1-6). In some embodiments, the weight ratio of difelikefalin: the pharmaceutically acceptable salt of NAC: auxiliary agent: diluent: lubricant: binder can be 1: (200-300): (100-150): (100-150): (5-15): (1-4). In some embodiments, the pharmaceutically acceptable salt of NAC can be NAC sodium salt, NAC potassium salt or NAC calcium salt. In some embodiments, the auxiliary agent can be disodium hydrogen phosphate. In some embodiments, the diluent can comprise microcrystalline cellulose. In some embodiments, the diluent can comprise pregelatinized starch and a microcrystalline cellulose-calcium hydrogen phosphate compound excipient. In some embodiments, the lubricant can comprise magnesium stearate. In some embodiments, the binder can be povidone. In some embodiments, the binder can be hydroxypropylcellulose. In some embodiments, the pharmaceutical composition described above can further comprise a disintegrant. In some embodiments, the disintegrant can be cross-linked povidone. In some embodiments, the weight ratio of difelikefalin to disintegrant can be 1:10 to 1:40. In some embodiments, the weight ratio of difelikefalin to disintegrant can be 1:15 to 1:30.

In some embodiments, the pharmaceutical composition can comprise a kind of granule. The granule can comprise difelikefalin, a delivery agent, and an auxiliary agent. Optionally, the granule can further comprise one or more non-active ingredients selected from a diluent, a disintegrant, a binder, and a lubricant. In some embodiments, the granule can comprise difelikefalin, a delivery agent, an auxiliary agent, and a diluent. In some embodiments, the granule can comprise difelikefalin, a delivery agent, an auxiliary agent, a diluent, and a lubricant. In some embodiments, difelikefalin, a delivery agent, an auxiliary agent, and optionally one or more non-active ingredients can be granulated after being homogeneously mixed. Granulation methods can comprise dry granulation, wet granulation, extrusion granulation, centrifugal granulation, and spray drying granulation.

In some embodiments, the pharmaceutical composition can be made from two or more granules with different compositions. In some embodiments, the pharmaceutical composition can comprise a first granule and a second granule.

In some embodiments, the first granule can comprise difelikefalin. In some embodiments, the first granule can comprise difelikefalin and optionally a pharmaceutically acceptable non-active ingredient. In some embodiments, the first granule can comprise difelikefalin and a delivery agent. In some embodiments, the first granule can comprise difelikefalin, a delivery agent, and an auxiliary agent. In some embodiments, the second granule can comprise a delivery agent. In some embodiments, the second granule can comprise a delivery agent, and an auxiliary agent. In some embodiments, the second granule can comprise a delivery agent, an auxiliary agent, and optionally a pharmaceutically acceptable non-active ingredient.

In some embodiments, the first granule can comprise difelikefalin and one or more non-active ingredients selected from a diluent, a disintegrant, a binder, and a lubricant. In some embodiments, the first granule can comprise difelikefalin and a diluent. In some embodiments, the first granule can comprise difelikefalin, a diluent, and a lubricant. In some embodiments, the first granule can comprise difelikefalin, a diluent, a binder, and a lubricant. In some embodiments, the first granule can comprise difelikefalin, a delivery agent, an auxiliary agent, a diluent, a disintegrant, a binder, and a lubricant. In some embodiments, the second granule can comprise one or more of a delivery agent, an auxiliary agent, a diluent, a disintegrant, a binder, and a lubricant. In some embodiments, the second granule can comprise a delivery agent, an auxiliary agent, and one or more non-active ingredients selected from a diluent, a disintegrant, a binder, and a lubricant. In some embodiments, the second granule can comprise a delivery agent, an auxiliary agent, and a diluent. In some embodiments, the second granule can comprise a delivery agent, an auxiliary agent, a diluent, and a lubricant. In some embodiments, the second granule can comprise a delivery agent, an auxiliary agent, a diluent, a lubricant, and a binder. In some embodiments, the second granule can comprise a delivery agent, an auxiliary agent, a diluent, a lubricant, a disintegrant, and a binder. In some embodiments, the first granule can comprise difelikefalin, a delivery agent, and an auxiliary agent, and the second granule can comprise one or more of a delivery agent, an auxiliary agent, a diluent, a disintegrant, a binder, and a lubricant. In some embodiments, the first granule can comprise difelikefalin, a delivery agent, an auxiliary agent, and one or more selected from a diluent, a disintegrant, a binder, and a lubricant, and the second granule can comprise one or more of a delivery agent, an auxiliary agent, a diluent, a disintegrant, a binder, and a lubricant. In some embodiments, difelikefalin and one or more non-active ingredients can be homogeneously mixed and then formed into a first granule, and a delivery agent, an auxiliary agent, and one or more non-active ingredients can be homogeneously mixed and then formed into a second granule. The first granule can be homogeneously mixed with the second granule, which are then granulated. In some embodiments, a lubricant can be added when mixing the first granule and the second granule. In some embodiments, difelikefalin and one or more non-active ingredients can be homogeneously mixed and then formed into a first granule, which can then be granulated after being homogeneously mixed with a delivery agent, an auxiliary agent, and one or more non-active ingredients. In some embodiments, a delivery agent, an auxiliary agent and one or more non-active ingredients can be homogeneously mixed and then formed into a second granule, which can then be granulated after being homogeneously mixed with difelikefalin and one or more non-active ingredients. Granulation methods can comprise dry granulation, wet granulation, extrusion granulation, centrifugal granulation, and spray drying granulation.

In some embodiments, the first granule can comprise difelikefalin, an auxiliary agent, and optionally a pharmaceutically acceptable non-active ingredient. In some embodiments, the first granule can comprise difelikefalin, an auxiliary agent, and one or more non-active ingredients selected from a diluent, a disintegrant, a binder, and a lubricant. In some embodiments, the first granule can comprise difelikefalin, an auxiliary agent, and a diluent. In some embodiments, the first granule can comprise difelikefalin, an auxiliary agent, a diluent, and a lubricant. In some embodiments, the first granule can comprise difelikefalin, an auxiliary agent, a diluent, a binder, and a lubricant. In some embodiments, the second granule can comprise a delivery agent, an auxiliary agent, and one or more non-active ingredients selected from a diluent, a disintegrant, a binder, and a lubricant. In some embodiments, the second granule can comprise a delivery agent, an auxiliary agent, and a diluent. In some embodiments, the second granule can comprise a delivery agent, an auxiliary agent, a diluent, and a lubricant. In some embodiments, the second granule can comprise a delivery agent, an auxiliary agent, a diluent, a lubricant, and a binder. In some embodiments, difelikefalin, an auxiliary agent, and one or more non-active ingredients can be homogeneously mixed and then formed into a first granule, and a delivery agent, an auxiliary agent, and one or more non-active ingredients can be homogeneously mixed and then formed into a second granule. The first granule can be homogeneously mixed with the second granule, which are then granulated. In some embodiments, a lubricant can be added when mixing the first granule and the second granule. In some embodiments, difelikefalin, an auxiliary agent, and one or more non-active ingredients can be homogeneously mixed and then formed into a first granule, which can then be granulated after being homogeneously mixed with a delivery agent, an auxiliary agent, and one or more non-active ingredients. In some embodiments, a delivery agent, an auxiliary agent, and one or more non-active ingredients can be homogeneously mixed and then formed into a second granule, which can then be granulated after being homogeneously mixed with difelikefalin, an auxiliary agent, and one or more non-active ingredients. Granulation methods can comprise dry granulation, wet granulation, extrusion granulation, centrifugal granulation, and spray drying granulation.

In some embodiments, the first granule can comprise difelikefalin, a delivery agent, and optionally a pharmaceutically acceptable non-active ingredient. In some embodiments, the first granule can comprise difelikefalin, a delivery agent, and one or more non-active ingredients selected from a diluent, a disintegrant, a binder, and a lubricant. In some embodiments, the first granule can comprise difelikefalin, a delivery agent, and a diluent. In some embodiments, the first granule can comprise difelikefalin, a delivery agent, a diluent, and a lubricant. In some embodiments, the first granule can comprise difelikefalin, a delivery agent, a diluent, a binder, and a lubricant. In some embodiments, the second granule can comprise a delivery agent, an auxiliary agent, and one or more non-active ingredients selected from a diluent, a disintegrant, a binder, and a lubricant. In some embodiments, the second granule can comprise a delivery agent, an auxiliary agent, and a diluent. In some embodiments, the second granule can comprise a delivery agent, an auxiliary agent, a diluent, and a lubricant. In some embodiments, the second granule can comprise a delivery agent, an auxiliary agent, a diluent, a lubricant, and a binder. In some embodiments, difelikefalin, a delivery agent, and one or more non-active ingredients can be homogeneously mixed and then formed into a first granule, and a delivery agent, an auxiliary agent, and one or more non-active ingredients can be homogeneously mixed and then formed into a second granule. The first granule can be homogeneously mixed with the second granule, which are then granulated. In some embodiments, a lubricant can be added when mixing the first granule and the second granule. In some embodiments, difelikefalin, a delivery agent, and one or more non-active ingredients can be homogeneously mixed and then formed into a first granule, which can then be granulated after being homogeneously mixed with a delivery agent, an auxiliary agent, and one or more non-active ingredients. In some embodiments, a delivery agent, an auxiliary agent and one or more non-active ingredients can be homogeneously mixed and then formed into a second granule, which can then be granulated after being homogeneously mixed with difelikefalin, a delivery agent, and one or more non-active ingredients. Granulation methods can comprise dry granulation, wet granulation, extrusion granulation, centrifugal granulation, and spray drying granulation.

In some embodiments, the first granule can comprise difelikefalin, a delivery agent, an auxiliary agent, and optionally a pharmaceutically acceptable non-active ingredient. In some embodiments, the first granule can comprise difelikefalin, a delivery agent, an auxiliary agent, and one or more non-active ingredients selected from a diluent, a disintegrant, a binder, and a lubricant. In some embodiments, the first granule can comprise difelikefalin, a delivery agent, an auxiliary agent, and a diluent. In some embodiments, the first granule can comprise difelikefalin, a delivery agent, an auxiliary agent, a diluent, and a lubricant. In some embodiments, the first granule can comprise difelikefalin, a delivery agent, an auxiliary agent, a diluent, a binder, and a lubricant. In some embodiments, the second granule can comprise a delivery agent, an auxiliary agent, and one or more non-active ingredients selected from a diluent, a disintegrant, a binder, and a lubricant. In some embodiments, the second granule can comprise a delivery agent, an auxiliary agent, and a diluent. In some embodiments, the second granule can comprise a delivery agent, an auxiliary agent, a diluent, and a lubricant. In some embodiments, the second granule can comprise a delivery agent, an auxiliary agent, a diluent, a lubricant, and a binder. In some embodiments, the second granule can comprise a delivery agent, an auxiliary agent, a diluent, a lubricant, a disintegrant, and a binder. In some embodiments, difelikefalin, a delivery agent, an auxiliary agent, and one or more non-active ingredients can be homogeneously mixed and then formed into a first granule, and a delivery agent, an auxiliary agent, and one or more non-active ingredients can be homogeneously mixed and then formed into a second granule. The first granule can be homogeneously mixed with the second granule, which are then granulated. In some embodiments, a lubricant can be added when mixing the first granule and the second granule. In some embodiments, difelikefalin, a delivery agent, an auxiliary agent, and one or more non-active ingredients can be homogeneously mixed and then formed into a first granule, which can then be granulated after being homogeneously mixed with a delivery agent, an auxiliary agent, and one or more non-active ingredients. In some embodiments, a delivery agent, an auxiliary agent and one or more non-active ingredients can be homogeneously mixed and then formed into a second granule, which can then be granulated after being homogeneously mixed with difelikefalin, a delivery agent, an auxiliary agent, and one or more non-active ingredients. Granulation methods can comprise dry granulation, wet granulation, extrusion granulation, centrifugal granulation, and spray drying granulation.

In some embodiments, the first granule can comprise difelikefalin, a delivery agent, an auxiliary agent, and one or more non-active ingredients selected from a diluent, a disintegrant, a binder, and a lubricant. In some embodiments, the first granule can comprise difelikefalin, a delivery agent, an auxiliary agent, and a diluent. In some embodiments, the first granule can comprise difelikefalin, a delivery agent, an auxiliary agent, a diluent, and a lubricant. In some embodiments, the first granule can comprise difelikefalin, a delivery agent, an auxiliary agent, a diluent, a binder, and a lubricant. In some embodiments, the second granule can comprise an auxiliary agent, and one or more non-active ingredients selected from a diluent, a disintegrant, a binder, and a lubricant. In some embodiments, the second granule can comprise an auxiliary agent and a diluent. In some embodiments, the second granule can comprise an auxiliary agent, a diluent, and a lubricant. In some embodiments, the second granule can comprise an auxiliary agent, a diluent, a lubricant, and a binder. In some embodiments, difelikefalin, a delivery agent, an auxiliary agent, and one or more non-active ingredients can be homogeneously mixed and then formed into a first granule, and an auxiliary agent, and one or more non-active ingredients can be homogeneously mixed and then formed into a second granule. The first granule can be homogeneously mixed with the second granule, which are then granulated. In some embodiments, a lubricant can be added when mixing the first granule and the second granule. In some embodiments, difelikefalin, a delivery agent, an auxiliary agent, and one or more non-active ingredients can be homogeneously mixed and then formed into a first granule, which can then be granulated after being homogeneously mixed with an auxiliary agent, and one or more non-active ingredients. In some embodiments, an auxiliary agent, and one or more non-active ingredients can be homogeneously mixed and then formed into a second granule, which can then be granulated after being homogeneously mixed with difelikefalin, a delivery agent, an auxiliary agent, and one or more non-active ingredients. Granulation methods can comprise dry granulation, wet granulation, extrusion granulation, centrifugal granulation, and spray drying granulation.

In some embodiments, the first granule can comprise difelikefalin, a delivery agent, an auxiliary agent, and one or more non-active ingredients selected from a diluent, a disintegrant, a binder, and a lubricant. In some embodiments, the first granule can comprise difelikefalin, a delivery agent, an auxiliary agent, and a diluent. In some embodiments, the first granule can comprise difelikefalin, a delivery agent, an auxiliary agent, a diluent, and a lubricant. In some embodiments, the first granule can comprise difelikefalin, a delivery agent, an auxiliary agent, a diluent, a binder, and a lubricant. In some embodiments, the second granule can comprise a delivery agent, and one or more non-active ingredients selected from a diluent, a disintegrant, a binder, and a lubricant. In some embodiments, the second granule can comprise a delivery agent and a diluent. In some embodiments, the second granule can comprise a delivery agent, a diluent, and a lubricant. In some embodiments, the second granule can comprise a delivery agent, a diluent, a lubricant, and a binder. In some embodiments, difelikefalin, a delivery agent, an auxiliary agent, and one or more non-active ingredients can be homogeneously mixed and then formed into a first granule, and a delivery agent and one or more non-active ingredients can be homogeneously mixed and then formed into a second granule. The first granule can be homogeneously mixed with the second granule, which are then granulated. In some embodiments, a lubricant can be added when mixing the first granule and the second granule. In some embodiments, difelikefalin, a delivery agent, an auxiliary agent, and one or more non-active ingredients can be homogeneously mixed and then formed into a first granule, which can then be granulated after being homogeneously mixed with a delivery agent and one or more non-active ingredients. In some embodiments, a delivery agent and one or more non-active ingredients can be homogeneously mixed and then formed into a second granule, which can then be granulated after being homogeneously mixed with difelikefalin, a delivery agent, an auxiliary agent, and one or more non-active ingredients. Granulation methods can comprise dry granulation, wet granulation, extrusion granulation, centrifugal granulation, and spray drying granulation.

In some embodiments, the first granule can comprise difelikefalin, an auxiliary agent, and one or more non-active ingredients selected from a diluent, a disintegrant, a binder, and a lubricant. In some embodiments, the first granule can comprise difelikefalin, an auxiliary agent, and a diluent. In some embodiments, the first granule can comprise difelikefalin, an auxiliary agent, a diluent, and a lubricant. In some embodiments, the first granule can comprise difelikefalin, an auxiliary agent, a diluent, a binder, and a lubricant. In some embodiments, the second granule can comprise a delivery agent and one or more non-active ingredients selected from a diluent, a disintegrant, a binder, and a lubricant. In some embodiments, the second granule can comprise a delivery agent and a diluent. In some embodiments, the second granule can comprise a delivery agent, a diluent, and a lubricant. In some embodiments, the second granule can comprise a delivery agent, a diluent, a lubricant, and a binder. In some embodiments, difelikefalin, an auxiliary agent, and one or more non-active ingredients can be homogeneously mixed and then formed into a first granule, and a delivery agent and one or more non-active ingredients can be homogeneously mixed and then formed into a second granule. The first granule can be homogeneously mixed with the second granule, which are then granulated. In some embodiments, a lubricant can be added when mixing the first granule and the second granule. In some embodiments, difelikefalin, an auxiliary agent, and one or more non-active ingredients can be homogeneously mixed and then formed into a first granule, which can then be granulated after being homogeneously mixed with a delivery agent and one or more non-active ingredients. In some embodiments, a delivery agent, and one or more non-active ingredients can be homogeneously mixed and then formed into a second granule, which can then be granulated after being homogeneously mixed with difelikefalin, an auxiliary agent, and one or more non-active ingredients. Granulation methods can comprise dry granulation, wet granulation, extrusion granulation, centrifugal granulation, and spray drying granulation.

In some embodiments, the first granule can comprise difelikefalin, a delivery agent, and one or more non-active ingredients selected from a diluent, a disintegrant, a binder, and a lubricant. In some embodiments, the first granule can comprise difelikefalin, a delivery agent, and a diluent. In some embodiments, the first granule can comprise difelikefalin, a delivery agent, a diluent, and a lubricant. In some embodiments, the first granule can comprise difelikefalin, a delivery agent, a diluent, a binder, and a lubricant. The second granule can comprise an auxiliary agent and one or more non-active ingredients selected from a diluent, a disintegrant, a binder, and a lubricant. In some embodiments, the second granule can comprise an auxiliary agent and a diluent. In some embodiments, the second granule can comprise an auxiliary agent, a diluent, and a lubricant. In some embodiments, the second granule can comprise an auxiliary agent, a diluent, a lubricant, and a binder. In some embodiments, difelikefalin, a delivery agent, and one or more non-active ingredients can be homogeneously mixed and then formed into a first granule, and an auxiliary agent and one or more non-active ingredients can be homogeneously mixed and then formed into a second granule. The first granule can be homogeneously mixed with the second granule, which are then granulated. In some embodiments, a lubricant can be added when mixing the first granule and the second granule. In some embodiments, difelikefalin, a delivery agent, and one or more non-active ingredients can be homogeneously mixed and then formed into a first granule, which can then be granulated after being homogeneously mixed with an auxiliary agent and one or more non-active ingredients. In some embodiments, an auxiliary agent and one or more non-active ingredients can be homogeneously mixed and then formed into a second granule, which can then be granulated after being homogeneously mixed with difelikefalin, a delivery agent, and one or more non-active ingredients. Granulation methods can comprise dry granulation, wet granulation, extrusion granulation, centrifugal granulation, and spray drying granulation.

In some embodiments, the first granule can comprise difelikefalin, a delivery agent, and one or more non-active ingredients selected from a diluent, a disintegrant, a binder, and a lubricant. In some embodiments, the first granule can comprise difelikefalin, a delivery agent, and a diluent. In some embodiments, the first granule can comprise difelikefalin, a delivery agent, a diluent, and a lubricant. In some embodiments, the first granule can comprise difelikefalin, a delivery agent, a diluent, a binder, and a lubricant. In some embodiments, the second granule can comprise a delivery agent and one or more non-active ingredients selected from a diluent, a disintegrant, a binder, and a lubricant. In some embodiments, the second granule can comprise a delivery agent and a diluent. In some embodiments, the second granule can comprise a delivery agent, a diluent, and a lubricant. In some embodiments, the second granule can comprise a delivery agent, a diluent, a lubricant, and a binder. In some embodiments, difelikefalin, a delivery agent, and one or more non-active ingredients can be homogeneously mixed and then formed into a first granule, and a delivery agent and one or more non-active ingredients can be homogeneously mixed and then formed into a second granule. The first granule can be homogeneously mixed with the second granule, which are then granulated. In some embodiments, a lubricant can be added when mixing the first granule and the second granule. In some embodiments, difelikefalin, a delivery agent, and one or more non-active ingredients can be homogeneously mixed and then formed into a first granule, which can then be granulated after being homogeneously mixed with a delivery agent and one or more non-active ingredients. In some embodiments, a delivery agent and one or more non-active ingredients can be homogeneously mixed and then formed into a second granule, which can then be granulated after being homogeneously mixed with difelikefalin, a delivery agent, and one or more non-active ingredients. Granulation methods can comprise dry granulation, wet granulation, extrusion granulation, centrifugal granulation, and spray drying granulation.

In some embodiments, the first granule can comprise difelikefalin and a delivery agent. In some embodiments, the first granule can comprise difelikefalin, a delivery agent, and an auxiliary agent. In some embodiments, the first granule can comprise difelikefalin, a delivery agent, an auxiliary agent, and optionally a pharmaceutically acceptable non-active ingredient. In some embodiments, the first granule can comprise difelikefalin, a delivery agent, an auxiliary agent, and one or more non-active ingredients selected from a diluent, a disintegrant, a binder, and a lubricant. In some embodiments, the first granule can comprise difelikefalin, a delivery agent, an auxiliary agent, and a diluent. In some embodiments, the first granule can comprise difelikefalin, a delivery agent, an auxiliary agent, a diluent, and a lubricant. In some embodiments, the first granule can comprise difelikefalin, a delivery agent, an auxiliary agent, a diluent, a binder, and a lubricant. In some embodiments, the first granule can comprise difelikefalin, a delivery agent, an auxiliary agent, a diluent, a disintegrant, a binder, and a lubricant. In some embodiments, the second granule can comprise one or more of a delivery agent, an auxiliary agent, a diluent, a disintegrant, a binder, and a lubricant. In some embodiments, the second granule can comprise a delivery agent and one or more non-active ingredients selected from an auxiliary agent, a diluent, a disintegrant, a binder, and a lubricant. In some embodiments, the second granule can comprise a delivery agent, an auxiliary agent, and one or more non-active ingredients selected from a diluent, a disintegrant, a binder, and a lubricant. In some embodiments, the second granule can comprise a delivery agent, an auxiliary agent, and a diluent. In some embodiments, the second granule can comprise a delivery agent, an auxiliary agent, a diluent, and a lubricant. In some embodiments, the second granule can comprise a delivery agent, an auxiliary agent, a diluent, a lubricant, and a binder. In some embodiments, the second granule can comprise a delivery agent, an auxiliary agent, a diluent, a lubricant, a disintegrant, and a binder. In some embodiments, difelikefalin, a delivery agent, an auxiliary agent, and one or more non-active ingredients can be homogeneously mixed and then formed into a first granule, and one or more of a delivery agent, an auxiliary agent, a diluent, a disintegrant, a binder, and a lubricant can be homogeneously mixed and then formed into a second granule. In some embodiments, difelikefalin, a delivery agent, an auxiliary agent, and one or more non-active ingredients can be homogeneously mixed and then formed into a first granule, and a delivery agent, an auxiliary agent, and one or more non-active ingredients can be homogeneously mixed and then formed into a second granule. In some embodiments, the pharmaceutical composition is a tablet, comprising: a core obtained by compressing the first granule, and an outer layer enclosing the core which is obtained by compressing the second granule.

In some embodiments, the present application provides a difelikefalin oral pharmaceutical composition, comprising an outer layer and a core enclosed by the outer layer, wherein,
the core comprises difelikefalin and a delivery agent; and
the outer layer comprises a delivery agent.

In some embodiments, the pharmaceutical composition can comprise an outer layer and a core, wherein the outer layer encloses the core.

In some embodiments, the outer layer does not comprise difelikefalin. In some embodiments, difelikefalin or a pharmaceutically acceptable salt thereof is the only active ingredient in the pharmaceutical composition.

In some embodiments, the outer layer can comprise optionally a pharmaceutically acceptable non-active ingredient, and the core can comprise difelikefalin and optionally a pharmaceutically acceptable non-active ingredient. In some embodiments, the outer layer can comprise: a delivery agent and optionally one or more selected from an auxiliary agent, a diluent, a disintegrant, a binder, and a lubricant, and the core can comprise difelikefalin and a delivery agent. In some embodiments, the outer layer can comprise a delivery agent and an auxiliary agent, and the core can comprise difelikefalin, a delivery agent, and an auxiliary agent.

In some embodiments, the outer layer can comprise optionally one or more non-active ingredients selected from a diluent, a disintegrant, a binder, and a lubricant, and the core can comprise difelikefalin and optionally one or more non-active ingredients selected from a diluent, a disintegrant, a binder, and a lubricant.

In some embodiments, the outer layer can comprise optionally a pharmaceutically acceptable non-active ingredient, and the core can comprise difelikefalin, a delivery agent, and optionally a pharmaceutically acceptable non-active ingredient.

In some embodiments, the outer layer can comprise optionally one or more non-active ingredients selected from a diluent, a disintegrant, a binder, and a lubricant, and the core can comprise difelikefalin, a delivery agent, and optionally one or more non-active ingredients selected from a diluent, a disintegrant, a binder, and a lubricant.

In some embodiments, the outer layer can comprise optionally a pharmaceutically acceptable non-active ingredient, and the core can comprise difelikefalin, an auxiliary agent, and optionally a pharmaceutically acceptable non-active ingredient.

In some embodiments, the outer layer can comprise optionally one or more non-active ingredients selected from a diluent, a disintegrant, a binder, and a lubricant, and the core can comprise difelikefalin, an auxiliary agent, and optionally one or more non-active ingredients selected from a diluent, a disintegrant, a binder, and a lubricant.

In some embodiments, the outer layer can comprise optionally a pharmaceutically acceptable non-active ingredient, and the core can comprise difelikefalin, a delivery agent, an auxiliary agent, and optionally a pharmaceutically acceptable non-active ingredient.

In some embodiments, the outer layer can comprise optionally one or more non-active ingredients selected from a diluent, a disintegrant, a binder, and a lubricant, and the core can comprise difelikefalin, a delivery agent, an auxiliary agent, and optionally one or more non-active ingredients selected from a diluent, a disintegrant, a binder, and a lubricant.

In some embodiments, the outer layer can comprise a delivery agent and optionally a pharmaceutically acceptable non-active ingredient, and the core can comprise difelikefalin and optionally a pharmaceutically acceptable non-active ingredient.

In some embodiments, the outer layer can comprise a delivery agent and optionally one or more non-active ingredients selected from a diluent, a disintegrant, a binder, and a lubricant, and the core can comprise difelikefalin and optionally one or more non-active ingredients selected from a diluent, a disintegrant, a binder, and a lubricant.

In some embodiments, the outer layer can comprise a delivery agent and optionally a pharmaceutically acceptable non-active ingredient, and the core can comprise difelikefalin, a delivery agent, and optionally a pharmaceutically acceptable non-active ingredient.

In some embodiments, the outer layer can comprise a delivery agent and optionally one or more non-active ingredients selected from a diluent, a disintegrant, a binder, and a lubricant, and the core can comprise difelikefalin, a delivery agent, and optionally one or more non-active ingredients selected from a diluent, a disintegrant, a binder, and a lubricant.

In some embodiments, the outer layer can comprise a delivery agent and optionally a pharmaceutically acceptable non-active ingredient, and the core can comprise difelikefalin, an auxiliary agent, and optionally a pharmaceutically acceptable non-active ingredient.

In some embodiments, the outer layer can comprise a delivery agent and optionally one or more non-active ingredients selected from a diluent, a disintegrant, a binder, and a lubricant, and the core can comprise difelikefalin, an auxiliary agent, and optionally one or more non-active ingredients selected from a diluent, a disintegrant, a binder, and a lubricant.

In some embodiments, the outer layer can comprise a delivery agent and optionally a pharmaceutically acceptable non-active ingredient, and the core can comprise difelikefalin, a delivery agent, an auxiliary agent, and optionally a pharmaceutically acceptable non-active ingredient.

In some embodiments, the outer layer can comprise a delivery agent and optionally one or more non-active ingredients selected from a diluent, a disintegrant, a binder, and a lubricant, and the core can comprise difelikefalin, a delivery agent, an auxiliary agent, and optionally one or more non-active ingredients selected from a diluent, a disintegrant, a binder, and a lubricant.

In some embodiments, the outer layer can comprise an auxiliary agent and optionally a pharmaceutically acceptable non-active ingredient, and the core can comprise difelikefalin and optionally a pharmaceutically acceptable non-active ingredient.

In some embodiments, the outer layer can comprise an auxiliary agent and optionally one or more non-active ingredients selected from a diluent, a disintegrant, a binder, and a lubricant, and the core can comprise difelikefalin and optionally one or more non-active ingredients selected from a diluent, a disintegrant, a binder, and a lubricant.

In some embodiments, the outer layer can comprise an auxiliary agent and optionally a pharmaceutically acceptable non-active ingredient, and the core can comprise difelikefalin, a delivery agent, and optionally a pharmaceutically acceptable non-active ingredient.

In some embodiments, the outer layer can comprise an auxiliary agent and optionally one or more non-active ingredients selected from a diluent, a disintegrant, a binder, and a lubricant, and the core can comprise difelikefalin, a delivery agent, and optionally one or more non-active ingredients selected from a diluent, a disintegrant, a binder, and a lubricant.

In some embodiments, the outer layer can comprise an auxiliary agent and optionally a pharmaceutically acceptable non-active ingredient, and the core can comprise difelikefalin, an auxiliary agent, and optionally a pharmaceutically acceptable non-active ingredient.

In some embodiments, the outer layer can comprise an auxiliary agent and optionally one or more non-active ingredients selected from a diluent, a disintegrant, a binder, and a lubricant, and the core can comprise difelikefalin, an auxiliary agent, and optionally one or more non-active ingredients selected from a diluent, a disintegrant, a binder, and a lubricant.

In some embodiments, the outer layer can comprise an auxiliary agent and optionally a pharmaceutically acceptable non-active ingredient, and the core can comprise difelikefalin, a delivery agent, an auxiliary agent, and optionally a pharmaceutically acceptable non-active ingredient.

In some embodiments, the outer layer can comprise an auxiliary agent and optionally one or more non-active ingredients selected from a diluent, a disintegrant, a binder, and a lubricant, and the core can comprise difelikefalin, a delivery agent, an auxiliary agent, and optionally one or more non-active ingredients selected from a diluent, a disintegrant, a binder, and a lubricant.

In some embodiments, the outer layer can comprise a delivery agent, an auxiliary agent, and optionally a pharmaceutically acceptable non-active ingredient, and the core can comprise difelikefalin and optionally a pharmaceutically acceptable non-active ingredient.

In some embodiments, the outer layer can comprise a delivery agent, an auxiliary agent, and optionally one or more non-active ingredients selected from a diluent, a disintegrant, a binder, and a lubricant, and the core can comprise difelikefalin and optionally one or more non-active ingredients selected from a diluent, a disintegrant, a binder, and a lubricant.

In some embodiments, the outer layer can comprise a delivery agent, an auxiliary agent, and optionally a pharmaceutically acceptable non-active ingredient, and the core can comprise difelikefalin, a delivery agent, and optionally a pharmaceutically acceptable non-active ingredient.

In some embodiments, the outer layer can comprise a delivery agent, an auxiliary agent, and optionally one or more non-active ingredients selected from a diluent, a disintegrant, a binder, and a lubricant, and the core can comprise difelikefalin, a delivery agent, and optionally one or more non-active ingredients selected from a diluent, a disintegrant, a binder, and a lubricant.

In some embodiments, the outer layer can comprise a delivery agent, an auxiliary agent, and optionally a pharmaceutically acceptable non-active ingredient, and the core can comprise difelikefalin, an auxiliary agent, and optionally a pharmaceutically acceptable non-active ingredient.

In some embodiments, the outer layer can comprise a delivery agent, an auxiliary agent, and optionally one or more non-active ingredients selected from a diluent, a disintegrant, a binder, and a lubricant, and the core can comprise difelikefalin, an auxiliary agent, and optionally one or more non-active ingredients selected from a diluent, a disintegrant, a binder, and a lubricant.

In some embodiments, the outer layer can comprise a delivery agent, an auxiliary agent, and optionally a pharmaceutically acceptable non-active ingredient, and the core can comprise difelikefalin, a delivery agent, an auxiliary agent, and optionally a pharmaceutically acceptable non-active ingredient.

In some embodiments, the outer layer can comprise one or more of a delivery agent, an auxiliary agent, a diluent, a disintegrant, a binder, and a lubricant. In some embodiments, the outer layer can comprise a delivery agent, an auxiliary agent, and optionally one or more non-active ingredients selected from a diluent, a disintegrant, a binder, and a lubricant, and the core can comprise difelikefalin, a delivery agent, an auxiliary agent, and optionally one or more non-active ingredients selected from a diluent, a disintegrant, a binder, and a lubricant. In some embodiments, the outer layer can comprise a delivery agent, an auxiliary agent, a diluent, a binder, and a lubricant. In some embodiments, the outer layer can comprise a delivery agent, an auxiliary agent, a diluent, a disintegrant, and a lubricant. In some embodiments, the outer layer can comprise a delivery agent, an auxiliary agent, a diluent, a disintegrant, a binder, and a lubricant. In some embodiments, the outer layer can comprise an auxiliary agent, a diluent, a disintegrant, a binder, and a lubricant. In some embodiments, the outer layer can comprise a diluent, a disintegrant, a binder, and a lubricant. In some embodiments, the core can comprise difelikefalin, a delivery agent, an auxiliary agent, a diluent, a binder, and a lubricant. In some embodiments, the core can comprise difelikefalin, a delivery agent, an auxiliary agent, a diluent, a disintegrant, and a lubricant. In some embodiments, the core can comprise difelikefalin, a delivery agent, an auxiliary agent, a diluent, a disintegrant, a binder, and a lubricant. In some embodiments, the outer layer can comprise a delivery agent, an auxiliary agent, a diluent, a binder, and a lubricant, and the core can comprise difelikefalin, a delivery agent, an auxiliary agent, a diluent, a disintegrant, a binder, and a lubricant. In some embodiments, the outer layer can comprise a delivery agent, an auxiliary agent, a diluent, a disintegrant, and a lubricant, and the core can comprise difelikefalin, a delivery agent, an auxiliary agent, a diluent, a disintegrant, and a lubricant. In some embodiments, the outer layer can comprise a delivery agent, an auxiliary agent, a diluent, a disintegrant, a binder, and a lubricant, and the core can comprise difelikefalin, a delivery agent, an auxiliary agent, a diluent, a disintegrant, a binder, and a lubricant.

In some embodiments, the outer layer is consisted of a pharmaceutically acceptable non-active ingredient, and the core is consisted of difelikefalin and optionally a pharmaceutically acceptable non-active ingredient.

In some embodiments, the outer layer is consisted of a pharmaceutically acceptable non-active ingredient, and the core is consisted of difelikefalin, a delivery agent, and optionally a pharmaceutically acceptable non-active ingredient.

In some embodiments, the outer layer is consisted of a pharmaceutically acceptable non-active ingredient, and the core is consisted of difelikefalin, an auxiliary agent, and optionally a pharmaceutically acceptable non-active ingredient.

In some embodiments, the outer layer is consisted of a pharmaceutically acceptable non-active ingredient, and the core is consisted of difelikefalin, a delivery agent, an auxiliary agent, and optionally a pharmaceutically acceptable non-active ingredient.

In some embodiments, the outer layer is consisted of a delivery agent and a pharmaceutically acceptable non-active ingredient, and the core is consisted of difelikefalin and optionally a pharmaceutically acceptable non-active ingredient.

In some embodiments, the outer layer is consisted of a delivery agent and a pharmaceutically acceptable non-active ingredient, and the core is consisted of difelikefalin, a delivery agent, and optionally a pharmaceutically acceptable non-active ingredient.

In some embodiments, the outer layer is consisted of a delivery agent and a pharmaceutically acceptable non-active ingredient, and the core is consisted of difelikefalin, an auxiliary agent, and optionally a pharmaceutically acceptable non-active ingredient.

In some embodiments, the outer layer is consisted of a delivery agent and a pharmaceutically acceptable non-active ingredient, and the core is consisted of difelikefalin, a delivery agent, an auxiliary agent, and optionally a pharmaceutically acceptable non-active ingredient.

In some embodiments, the outer layer is consisted of an auxiliary agent and a pharmaceutically acceptable non-active ingredient, and the core is consisted of difelikefalin and optionally a pharmaceutically acceptable non-active ingredient.

In some embodiments, the outer layer is consisted of an auxiliary agent and a pharmaceutically acceptable non-active ingredient, and the core is consisted of difelikefalin, a delivery agent, and optionally a pharmaceutically acceptable non-active ingredient.

In some embodiments, the outer layer is consisted of an auxiliary agent and a pharmaceutically acceptable non-active ingredient, and the core is consisted of difelikefalin, an auxiliary agent, and optionally a pharmaceutically acceptable non-active ingredient.

In some embodiments, the outer layer is consisted of an auxiliary agent and a pharmaceutically acceptable non-active ingredient, and the core is consisted of difelikefalin, a delivery agent, an auxiliary agent, and optionally a pharmaceutically acceptable non-active ingredient.

In some embodiments, the outer layer is consisted of a delivery agent, an auxiliary agent and a pharmaceutically acceptable non-active ingredient, and the core is consisted of difelikefalin and optionally a pharmaceutically acceptable non-active ingredient.

In some embodiments, the outer layer is consisted of a delivery agent, an auxiliary agent and a pharmaceutically acceptable non-active ingredient, and the core is consisted of difelikefalin, a delivery agent, and optionally a pharmaceutically acceptable non-active ingredient.

In some embodiments, the outer layer is consisted of a delivery agent, an auxiliary agent and a pharmaceutically acceptable non-active ingredient, and the core is consisted of difelikefalin, an auxiliary agent, and optionally a pharmaceutically acceptable non-active ingredient.

In some embodiments, the outer layer is consisted of a delivery agent, an auxiliary agent and a pharmaceutically acceptable non-active ingredient, and the core is consisted of difelikefalin, a delivery agent, an auxiliary agent, and optionally a pharmaceutically acceptable non-active ingredient.

In some embodiments, the outer layer comprises difelikefalin.

In some embodiments, the outer layer can comprise difelikefalin and optionally a pharmaceutically acceptable non-active ingredient, and the core can comprise a delivery agent, an auxiliary agent, and optionally a pharmaceutically acceptable non-active ingredient.

In some embodiments, the outer layer can comprise a difelikefalin and optionally one or more non-active ingredients selected from a diluent, a disintegrant, a binder, and a lubricant, and the core can comprise a delivery agent, an auxiliary agent, and optionally one or more non-active ingredients selected from a diluent, a disintegrant, a binder, and a lubricant.

In some embodiments, the outer layer can comprise difelikefalin, a delivery agent and optionally a pharmaceutically acceptable non-active ingredient, and the core can comprise a delivery agent, an auxiliary agent, and optionally a pharmaceutically acceptable non-active ingredient.

In some embodiments, the outer layer can comprise a difelikefalin, a delivery agent and optionally one or more non-active ingredients selected from a diluent, a disintegrant, a binder, and a lubricant, and the core can comprise a delivery agent, an auxiliary agent, and optionally one or more non-active ingredients selected from a diluent, a disintegrant, a binder, and a lubricant.

In some embodiments, the outer layer can comprise difelikefalin, an auxiliary agent and optionally a pharmaceutically acceptable non-active ingredient, and the core can comprise a delivery agent, an auxiliary agent, and optionally a pharmaceutically acceptable non-active ingredient.

In some embodiments, the outer layer can comprise a difelikefalin, an auxiliary agent, and optionally one or more non-active ingredients selected from a diluent, a disintegrant, a binder, and a lubricant, and the core can comprise a delivery agent, an auxiliary agent, and optionally one or more non-active ingredients selected from a diluent, a disintegrant, a binder, and a lubricant.

In some embodiments, the outer layer can comprise difelikefalin, a delivery agent, an auxiliary agent, and optionally a pharmaceutically acceptable non-active ingredient, and the core can comprise a delivery agent, an auxiliary agent, and optionally a pharmaceutically acceptable non-active ingredient.

In some embodiments, the outer layer can comprise a difelikefalin, a delivery agent, an auxiliary agent, and optionally one or more non-active ingredients selected from a diluent, a disintegrant, a binder, and a lubricant, and the core can comprise a delivery agent, an auxiliary agent, and optionally one or more non-active ingredients selected from a diluent, a disintegrant, a binder, and a lubricant.

In some embodiments, the outer layer can comprise difelikefalin and optionally a pharmaceutically acceptable non-active ingredient, and the core can comprise difelikefalin, a delivery agent, an auxiliary agent, and optionally a pharmaceutically acceptable non-active ingredient.

In some embodiments, the outer layer can comprise difelikefalin and optionally one or more non-active ingredients selected from a diluent, a disintegrant, a binder, and a lubricant, and the core can comprise difelikefalin, a delivery agent, an auxiliary agent, and optionally one or more non-active ingredients selected from a diluent, a disintegrant, a binder, and a lubricant.

In some embodiments, the outer layer can comprise difelikefalin, a delivery agent, and optionally a pharmaceutically acceptable non-active ingredient, and the core can comprise difelikefalin, a delivery agent, an auxiliary agent, and optionally a pharmaceutically acceptable non-active ingredient.

In some embodiments, the outer layer can comprise difelikefalin, a delivery agent and optionally one or more non-active ingredients selected from a diluent, a disintegrant, a binder, and a lubricant, and the core can comprise difelikefalin, a delivery agent, an auxiliary agent, and optionally one or more non-active ingredients selected from a diluent, a disintegrant, a binder, and a lubricant.

In some embodiments, the outer layer can comprise difelikefalin, an auxiliary agent, and optionally a pharmaceutically acceptable non-active ingredient, and the core can comprise difelikefalin, a delivery agent, an auxiliary agent, and optionally a pharmaceutically acceptable non-active ingredient.

In some embodiments, the outer layer can comprise difelikefalin, an auxiliary agent, and optionally one or more non-active ingredients selected from a diluent, a disintegrant, a binder, and a lubricant, and the core can comprise difelikefalin, a delivery agent, an auxiliary agent, and optionally one or more non-active ingredients selected from a diluent, a disintegrant, a binder, and a lubricant.

In some embodiments, the outer layer can comprise difelikefalin, a delivery agent, an auxiliary agent, and optionally a pharmaceutically acceptable non-active ingredient, and the core can comprise difelikefalin, a delivery agent, an auxiliary agent, and optionally a pharmaceutically acceptable non-active ingredient.

In some embodiments, the outer layer can comprise difelikefalin, a delivery agent, an auxiliary agent, and optionally one or more non-active ingredients selected from a diluent, a disintegrant, a binder, and a lubricant, and the core can comprise difelikefalin, a delivery agent, an auxiliary agent, and optionally one or more non-active ingredients selected from a diluent, a disintegrant, a binder, and a lubricant.

In some embodiments, the outer layer is consisted of a delivery agent. In some embodiments, the outer layer is consisted of a delivery agent and an auxiliary agent. In some embodiments, the outer layer is consisted of a delivery agent, an auxiliary agent, and a diluent. In some embodiments, the outer layer is consisted of a delivery agent, an auxiliary agent, and a disintegrant. In some embodiments, the outer layer is consisted of a delivery agent, an auxiliary agent, and a binder. In some embodiments, the outer layer is consisted of a delivery agent, an auxiliary agent, a binder, and a disintegrant. In some embodiments, the outer layer is consisted of a delivery agent, an auxiliary agent, a diluent, and a lubricant. In some embodiments, the outer layer is consisted of a delivery agent, an auxiliary agent, a diluent, a lubricant, and a binder. In some embodiments, the outer layer is consisted of a delivery agent, an auxiliary agent, a diluent, a binder, and a disintegrant. In some embodiments, the outer layer is consisted of a delivery agent, an auxiliary agent, a diluent, a lubricant, a binder, and a disintegrant.

In some embodiments, the core is consisted of difelikefalin and a delivery agent. In some embodiments, the core is consisted of difelikefalin, a delivery agent, and an auxiliary agent. In some embodiments, the core is consisted of difelikefalin, a delivery agent, an auxiliary agent, and a diluent. In some embodiments, the core is consisted of difelikefalin, a delivery agent, an auxiliary agent, and a disintegrant. In some embodiments, the core is consisted of difelikefalin, a delivery agent, an auxiliary agent, and a binder. In some embodiments, the core is consisted of difelikefalin, a delivery agent, an auxiliary agent, a binder, and a disintegrant. In some embodiments, the core is consisted of difelikefalin, a delivery agent, an auxiliary agent, a diluent, and a lubricant. In some embodiments, the core is consisted of difelikefalin, a delivery agent, an auxiliary agent, a diluent, a lubricant, and a binder. In some embodiments, the core is consisted of difelikefalin, a delivery agent, an auxiliary agent, a diluent, a binder, and a disintegrant. In some embodiments, the core is consisted of difelikefalin, a delivery agent, an auxiliary agent, a diluent, a lubricant, a binder, and a disintegrant.

In some embodiments, the outer layer is consisted of a delivery agent, and the core is consisted of difelikefalin and a delivery agent. In some embodiments, the outer layer is consisted of a delivery agent, and the core is consisted of difelikefalin, a delivery agent, and an auxiliary agent. In some embodiments, the outer layer is consisted of a delivery agent and an auxiliary agent, and the core is consisted of difelikefalin and a delivery agent. In some embodiments, the outer layer is consisted of a delivery agent and an auxiliary agent, and the core is consisted of difelikefalin, a delivery agent, and an auxiliary agent. In some embodiments, the outer layer is consisted of a delivery agent, an auxiliary agent, and a diluent, and the core is consisted of difelikefalin, a delivery agent, an auxiliary agent, and a diluent. In some embodiments, the outer layer is consisted of a delivery agent, an auxiliary agent, and a diluent, and the core is consisted of difelikefalin, a delivery agent, an auxiliary agent, and a disintegrant. In some embodiments, the outer layer is consisted of a delivery agent, an auxiliary agent, and a diluent, and the core is consisted of difelikefalin, a delivery agent, an auxiliary agent, and a binder. In some embodiments, the outer layer is consisted of a delivery agent, an auxiliary agent, and a diluent, and the core is consisted of difelikefalin, a delivery agent, an auxiliary agent, a binder, and a disintegrant. In some embodiments, the outer layer is consisted of a delivery agent, an auxiliary agent, and a diluent, and the core is consisted of difelikefalin, a delivery agent, an auxiliary agent, a diluent, and a lubricant. In some embodiments, the outer layer is consisted of a delivery agent, an auxiliary agent, and a diluent, and the core is consisted of difelikefalin, a delivery agent, an auxiliary agent, a diluent, a lubricant, and a binder. In some embodiments, the outer layer is consisted of a delivery agent, an auxiliary agent, and a diluent, and the core is consisted of difelikefalin, a delivery agent, an auxiliary agent, a diluent, a binder, and a disintegrant. In some embodiments, the outer layer is consisted of a delivery agent, an auxiliary agent, and a diluent, and the core is consisted of difelikefalin, a delivery agent, an auxiliary agent, a diluent, a lubricant, a binder, and a disintegrant.

In some embodiments, the outer layer is consisted of a delivery agent, an auxiliary agent, and a disintegrant, and the core is consisted of difelikefalin, a delivery agent, an auxiliary agent, and a diluent. In some embodiments, the outer layer is consisted of a delivery agent, an auxiliary agent, and a disintegrant, and the core is consisted of difelikefalin, a delivery agent, an auxiliary agent, and a disintegrant. In some embodiments, the outer layer is consisted of a delivery agent, an auxiliary agent, and a disintegrant, and the core is consisted of difelikefalin, a delivery agent, an auxiliary agent, and a binder. In some embodiments, the outer layer is consisted of a delivery agent, an auxiliary agent, and a disintegrant, and the core is consisted of difelikefalin, a delivery agent, an auxiliary agent, a binder, and a disintegrant. In some embodiments, the outer layer is consisted of a delivery agent, an auxiliary agent, and a disintegrant, and the core is consisted of difelikefalin, a delivery agent, an auxiliary agent, a diluent, and a lubricant. In some embodiments, the outer layer is consisted of a delivery agent, an auxiliary agent, and a disintegrant, and the core is consisted of difelikefalin, a delivery agent, an auxiliary agent, a diluent, a lubricant, and a binder. In some embodiments, the outer layer is consisted of a delivery agent, an auxiliary agent, and a disintegrant, and the core is consisted of difelikefalin, a delivery agent, an auxiliary agent, a diluent, a binder, and a disintegrant. In some embodiments, the outer layer is consisted of a delivery agent, an auxiliary agent, and a disintegrant, and the core is consisted of difelikefalin, a delivery agent, an auxiliary agent, a diluent, a lubricant, a binder, and a disintegrant.

In some embodiments, the outer layer is consisted of a delivery agent, an auxiliary agent, and a binder, and the core is consisted of difelikefalin, a delivery agent, an auxiliary agent, and a diluent. In some embodiments, the outer layer is consisted of a delivery agent, an auxiliary agent, and a binder, and the core is consisted of difelikefalin, a delivery agent, an auxiliary agent, and a disintegrant. In some embodiments, the outer layer is consisted of a delivery agent, an auxiliary agent, and a binder, and the core is consisted of difelikefalin, a delivery agent, an auxiliary agent, and a binder. In some embodiments, the outer layer is consisted of a delivery agent, an auxiliary agent, and a binder, and the core is consisted of difelikefalin, a delivery agent, an auxiliary agent, a binder, and a disintegrant. In some embodiments, the outer layer is consisted of a delivery agent, an auxiliary agent, and a binder, and the core is consisted of difelikefalin, a delivery agent, an auxiliary agent, a diluent, and a lubricant. In some embodiments, the outer layer is consisted of a delivery agent, an auxiliary agent, and a binder, and the core is consisted of difelikefalin, a delivery agent, an auxiliary agent, a diluent, a lubricant, and a binder. In some embodiments, the outer layer is consisted of a delivery agent, an auxiliary agent, and a binder, and the core is consisted of difelikefalin, a delivery agent, an auxiliary agent, a diluent, a binder, and a disintegrant. In some embodiments, the outer layer is consisted of a delivery agent, an auxiliary agent, and a binder, and the core is consisted of difelikefalin, a delivery agent, an auxiliary agent, a diluent, a lubricant, a binder, and a disintegrant.

In some embodiments, the outer layer is consisted of a delivery agent, an auxiliary agent, a binder, and a disintegrant, and the core is consisted of difelikefalin, a delivery agent, an auxiliary agent, and a diluent. In some embodiments, the outer layer is consisted of a delivery agent, an auxiliary agent, a binder, and a disintegrant, and the core is consisted of difelikefalin, a delivery agent, an auxiliary agent, and a disintegrant. In some embodiments, the outer layer is consisted of a delivery agent, an auxiliary agent, a binder, and a disintegrant, and the core is consisted of difelikefalin, a delivery agent, an auxiliary agent, and a binder. In some embodiments, the outer layer is consisted of a delivery agent, an auxiliary agent, a binder, and a disintegrant, and the core is consisted of difelikefalin, a delivery agent, an auxiliary agent, a binder, and a disintegrant. In some embodiments, the outer layer is consisted of a delivery agent, an auxiliary agent, a binder, and a disintegrant, and the core is consisted of difelikefalin, a delivery agent, an auxiliary agent, a diluent, and a lubricant. In some embodiments, the outer layer is consisted of a delivery agent, an auxiliary agent, a binder, and a disintegrant, and the core is consisted of difelikefalin, a delivery agent, an auxiliary agent, a diluent, a lubricant, and a binder. In some embodiments, the outer layer is consisted of a delivery agent, an auxiliary agent, a binder, and a disintegrant, and the core is consisted of difelikefalin, a delivery agent, an auxiliary agent, a diluent, a binder, and a disintegrant. In some embodiments, the outer layer is consisted of a delivery agent, an auxiliary agent, a binder, and a disintegrant, and the core is consisted of difelikefalin, a delivery agent, an auxiliary agent, a diluent, a lubricant, a binder, and a disintegrant.

In some embodiments, the outer layer is consisted of a delivery agent, an auxiliary agent, a diluent, and a lubricant, and the core is consisted of difelikefalin, a delivery agent, an auxiliary agent, and a diluent. In some embodiments, the outer layer is consisted of a delivery agent, an auxiliary agent, a diluent, and a lubricant, and the core is consisted of difelikefalin, a delivery agent, an auxiliary agent, and a disintegrant. In some embodiments, the outer layer is consisted of a delivery agent, an auxiliary agent, a diluent, and a lubricant, and the core is consisted of difelikefalin, a delivery agent, an auxiliary agent, and a binder. In some embodiments, the outer layer is consisted of a delivery agent, an auxiliary agent, a diluent, and a lubricant, and the core is consisted of difelikefalin, a delivery agent, an auxiliary agent, a binder, and a disintegrant. In some embodiments, the outer layer is consisted of a delivery agent, an auxiliary agent, a diluent, and a lubricant, and the core is consisted of difelikefalin, a delivery agent, an auxiliary agent, a diluent, and a lubricant. In some embodiments, the outer layer is consisted of a delivery agent, an auxiliary agent, a diluent, and a lubricant, and the core is consisted of difelikefalin, a delivery agent, an auxiliary agent, a diluent, a lubricant, and a binder. In some embodiments, the outer layer is consisted of a delivery agent, an auxiliary agent, a diluent, and a lubricant, and the core is consisted of difelikefalin, a delivery agent, an auxiliary agent, a diluent, a binder, and a disintegrant. In some embodiments, the outer layer is consisted of a delivery agent, an auxiliary agent, a diluent, and a lubricant, and the core is consisted of difelikefalin, a delivery agent, an auxiliary agent, a diluent, a lubricant, a binder, and a disintegrant.

In some embodiments, the outer layer is consisted of a delivery agent, an auxiliary agent, a diluent, a lubricant, and a binder, and the core is consisted of difelikefalin, a delivery agent, an auxiliary agent, and a diluent. In some embodiments, the outer layer is consisted of a delivery agent, an auxiliary agent, a diluent, a lubricant, and a binder, and the core is consisted of difelikefalin, a delivery agent, an auxiliary agent, and a disintegrant. In some embodiments, the outer layer is consisted of a delivery agent, an auxiliary agent, a diluent, a lubricant, and a binder, and the core is consisted of difelikefalin, a delivery agent, an auxiliary agent, and a binder. In some embodiments, the outer layer is consisted of a delivery agent, an auxiliary agent, a diluent, a lubricant, and a binder, and the core is consisted of difelikefalin, a delivery agent, an auxiliary agent, a binder, and a disintegrant. In some embodiments, the outer layer is consisted of a delivery agent, an auxiliary agent, a diluent, a lubricant, and a binder, and the core is consisted of difelikefalin, a delivery agent, an auxiliary agent, a diluent, and a lubricant. In some embodiments, the outer layer is consisted of a delivery agent, an auxiliary agent, a diluent, a lubricant, and a binder, and the core is consisted of difelikefalin, a delivery agent, an auxiliary agent, a diluent, a lubricant, and a binder. In some embodiments, the outer layer is consisted of a delivery agent, an auxiliary agent, a diluent, a lubricant, and a binder, and the core is consisted of difelikefalin, a delivery agent, an auxiliary agent, a diluent, a binder, and a disintegrant. In some embodiments, the outer layer is consisted of a delivery agent, an auxiliary agent, a diluent, a lubricant, and a binder, and the core is consisted of difelikefalin, a delivery agent, an auxiliary agent, a diluent, a lubricant, a binder, and a disintegrant.

In some embodiments, the outer layer is consisted of a delivery agent, an auxiliary agent, a diluent, a binder, and a disintegrant, and the core is consisted of difelikefalin, a delivery agent, an auxiliary agent, and a diluent. In some embodiments, the outer layer is consisted of a delivery agent, an auxiliary agent, a diluent, a binder, and a disintegrant, and the core is consisted of difelikefalin, a delivery agent, an auxiliary agent, and a disintegrant. In some embodiments, the outer layer is consisted of a delivery agent, an auxiliary agent, a diluent, a binder, and a disintegrant, and the core is consisted of difelikefalin, a delivery agent, an auxiliary agent, and a binder. In some embodiments, the outer layer is consisted of a delivery agent, an auxiliary agent, a diluent, a binder, and a disintegrant, and the core is consisted of difelikefalin, a delivery agent, an auxiliary agent, a binder, and a disintegrant. In some embodiments, the outer layer is consisted of a delivery agent, an auxiliary agent, a diluent, a binder, and a disintegrant, and the core is consisted of difelikefalin, a delivery agent, an auxiliary agent, a diluent, and a lubricant. In some embodiments, the outer layer is consisted of a delivery agent, an auxiliary agent, a diluent, a binder, and a disintegrant, and the core is consisted of difelikefalin, a delivery agent, an auxiliary agent, a diluent, a lubricant, and a binder. In some embodiments, the outer layer is consisted of a delivery agent, an auxiliary agent, a diluent, a binder, and a disintegrant, and the core is consisted of difelikefalin, a delivery agent, an auxiliary agent, a diluent, a binder, and a disintegrant. In some embodiments, the outer layer is consisted of a delivery agent, an auxiliary agent, a diluent, a binder, and a disintegrant, and the core is consisted of difelikefalin, a delivery agent, an auxiliary agent, a diluent, a lubricant, a binder, and a disintegrant.

In some embodiments, the outer layer is consisted of a delivery agent, an auxiliary agent, a diluent, a lubricant, a binder, and a disintegrant, and the core is consisted of difelikefalin, a delivery agent, an auxiliary agent, and a diluent. In some embodiments, the outer layer is consisted of a delivery agent, an auxiliary agent, a diluent, a lubricant, a binder, and a disintegrant, and the core is consisted of difelikefalin, a delivery agent, an auxiliary agent, and a disintegrant. In some embodiments, the outer layer is consisted of a delivery agent, an auxiliary agent, a diluent, a lubricant, a binder, and a disintegrant, and the core is consisted of difelikefalin, a delivery agent, an auxiliary agent, and a binder. In some embodiments, the outer layer is consisted of a delivery agent, an auxiliary agent, a diluent, a lubricant, a binder, and a disintegrant, and the core is consisted of difelikefalin, a delivery agent, an auxiliary agent, a binder, and a disintegrant. In some embodiments, the outer layer is consisted of a delivery agent, an auxiliary agent, a diluent, a lubricant, a binder, and a disintegrant, and the core is consisted of difelikefalin, a delivery agent, an auxiliary agent, a diluent, and a lubricant. In some embodiments, the outer layer is consisted of a delivery agent, an auxiliary agent, a diluent, a lubricant, a binder, and a disintegrant, and the core is consisted of difelikefalin, a delivery agent, an auxiliary agent, a diluent, a lubricant, and a binder. In some embodiments, the outer layer is consisted of a delivery agent, an auxiliary agent, a diluent, a lubricant, a binder, and a disintegrant, and the core is consisted of difelikefalin, a delivery agent, an auxiliary agent, a diluent, a binder, and a disintegrant. In some embodiments, the outer layer is consisted of a delivery agent, an auxiliary agent, a diluent, a lubricant, a binder, and a disintegrant, and the core is consisted of difelikefalin, a delivery agent, an auxiliary agent, a diluent, a lubricant, a binder, and a disintegrant.

In some embodiments, based on the total mass of the pharmaceutical composition, the mass content of the core is 10 wt%-60 wt%. In some embodiments, based on the total mass of the pharmaceutical composition, the mass content of the core is 10 wt%-60 wt%, 12 wt%-58 wt%, 15 wt%-55 wt%, 18 wt%-53 wt%, 20 wt%-50 wt%, 22 wt%-48 wt%, 25 wt%-45 wt%, 28 wt%-42 wt%, 30 wt%-40 wt%, 33 wt%-45 wt%, 35 wt%-50 wt%, 37 wt%-52 wt%, 40 wt%-55 wt%, 45 wt%-60 wt%.

In some embodiments, based on the mass of the outer layer, the mass content of the delivery agent in the outer layer can be 30 wt%-75 wt%, the mass content of the auxiliary agent can be 0 wt%-40 wt%, the mass content of the diluent can be 1 wt%-30 wt%, the mass content of the binder can be 0 wt%-7.5 wt%, the mass content of the lubricant can be 0.5 wt%-5 wt%, and the mass content of the disintegrant can be 0 wt%-10 wt%.

In some embodiments, based on the mass of the outer layer, the mass content of the delivery agent in the outer layer can be 30 wt%-76 wt%, the mass content of the auxiliary agent can be 0 wt%-40 wt%, the mass content of the diluent can be 1 wt%-97 wt%, the mass content of the binder can be 0 wt%-7.5 wt%, the mass content of the lubricant can be 0.5 wt%-5 wt%, and the mass content of the disintegrant can be 0 wt%-10 wt%.

In some embodiments, based on the mass of the outer layer, the mass content of the delivery agent in the outer layer can be 30 wt%-75 wt%, 32 wt%-73 wt%, 35 wt%-70 wt%, 37 wt%-68 wt%, 40 wt%-65 wt%, 43 wt%-62 wt%, 45 wt%-60 wt%, 48 wt%-55 wt%, 50 wt%-75 wt%, 52 wt%-73 wt%, 55 wt%-70 wt%.

In some embodiments, based on the mass of the outer layer, the mass content of the auxiliary agent in the outer layer can be 0 wt%-40 wt%, 0 wt%-25 wt%, 0.1 wt%-40 wt%, 1 wt%-35 wt%, 3 wt%-32 wt%, 5 wt%-30 wt%, 7 wt%-28 wt%, 10 wt%-25 wt%, 12 wt%-23 wt%, 15 wt%-30 wt%, 18 wt%-27 wt%, 20 wt%-25 wt%.

In some embodiments, based on the mass of the outer layer, the mass content of the diluent in the outer layer can be 1 wt%-97 wt%, 1 wt%-95 wt%, 1 wt%-90 wt%, 1 wt%-85 wt%, 1 wt%-80 wt%, 1 wt%-75 wt%, 1 wt%-70 wt%, 1 wt%-65 wt%, 1 wt%-60 wt%, 1 wt%-55 wt%, 1 wt%-50 wt%, 1 wt%-45 wt%, 1 wt%-40 wt%, 1 wt%-35 wt%, 1 wt%-30 wt%, 3 wt%-28 wt%, 5 wt%-25 wt%, 7 wt%-22 wt%, 10 wt%-20 wt%, 12 wt%-18 wt%, 15 wt%-30 wt%, 20 wt%-30 wt%, 16 wt%-36 wt%.

In some embodiments, based on the mass of the outer layer, the mass content of the binder in the outer layer can be 0 wt%-7.5 wt%, 0 wt%-4.2 wt%, 0.5 wt%-7.5 wt%, 0.7 wt%-7.0 wt%, 0.9 wt%-6.5 wt%, 1.0 wt%-6.0 wt%, 1.5 wt%-5.5 wt%, 2.0 wt%-5.0 wt%, 2.5 wt%-4.5 wt%, 3.0 wt%-6 wt%, 3.5 wt%-7.0 wt%.

In some embodiments, based on the mass of the outer layer, the mass content of the lubricant in the outer layer can be 0.5 wt%-5 wt%, 0 wt%-3 wt%, 0.8 wt%-4.7 wt%, 1 wt%-4.5 wt%, 1.2 wt%-4.3 wt%, 1.5 wt%-4.0 wt%, 1.8 wt%-3.7 wt%, 2 wt%-3.5 wt%, 2.2 wt%-3.8 wt%, 2.5 wt%-4 wt%, 2.7 wt%-4.3 wt%, 3 wt%-4.5 wt%, 3.5 wt%-5 wt%.

In some embodiments, based on the mass of the outer layer, the mass content of the disintegrant in the outer layer can be 0 wt%-10 wt%, 0 wt%-7 wt%, 0.1 wt%-10 wt%, 0.1 wt%-9.5 wt%, 0.5 wt%-9 wt%, 1 wt%-8.5 wt%, 1.5 wt%-8 wt%, 2 wt%-7.5 wt%, 2.5 wt%-7 wt%, 3 wt%-6.5 wt%, 3.5 wt%-6 wt%, 4 wt%-5.5 wt%, 4.5 wt%-6 wt%, 5 wt%-6.5 wt%.

In some embodiments, based on the mass of the core, the mass content of difelikefalin in the core is 0.1 wt%-20 wt%, the mass content of the delivery agent can be 30 wt%-75 wt%, the mass content of the auxiliary agent can be 0 wt%-40 wt%, the mass content of the disintegrant can be 0 wt%-10 wt%, the mass content of the diluent can be 1 wt%-30 wt%, the mass content of the binder can be 0 wt%-7.5 wt%, and the mass content of the lubricant can be 0.5 wt%-5 wt%.

In some embodiments, based on the mass of the core, the mass content of difelikefalin in the core is 0.1 wt%-20 wt%, the mass content of the delivery agent can be 30 wt%-75 wt%, the mass content of the auxiliary agent can be 0 wt%-95 wt%, the mass content of the disintegrant can be 0 wt%-10 wt%, the mass content of the diluent can be 1 wt%-85 wt%, the mass content of the binder can be 0 wt%-7.5 wt%, and the mass content of the lubricant can be 0.5 wt%-5 wt%.

In some embodiments, based on the mass of the core, the mass content of difelikefalin in the core can be 0.1 wt%-20 wt%, 0.2 wt%-19 wt%, 0.5 wt%-18 wt%, 0.7 wt%-17 wt%, 0.9 wt%-16 wt%, 1 wt%-15 wt%, 1.2 wt%-14 wt%, 1.5 wt%-13 wt%, 1.7 wt%-12 wt%, 2 wt%-11 wt%, 2.2 wt%-10 wt%, 2.5 wt%-9 wt%, 3 wt%-8 wt%, 4 wt%-7 wt%, 5 wt%-6 wt%, 0.6 wt%-2.2 wt%, 0.6 wt%-10 wt%.

In some embodiments, based on the mass of the core, the mass content of the delivery agent in the core can be 30 wt%-75 wt%, 32 wt%-72 wt%, 35 wt%-70 wt%, 37 wt%-67 wt%, 40 wt%-65 wt%, 42 wt%-62 wt%, 45 wt%-60 wt%, 50 wt%-65 wt%, 55 wt%-70 wt%, 60 wt%-75 wt%, 37.5 wt%-67 wt%, 45 wt%-67 wt%, 45 wt%-63.5 wt%.

In some embodiments, based on the mass of the core, the mass content of the auxiliary agent in the core can be 0 wt%-95 wt%, 0 wt%-90 wt%, 0 wt%-80 wt%, 0 wt%-70 wt%, 0 wt%-60 wt%, 0 wt%-50 wt%, 0 wt%-40 wt%, 0.1 wt%-40 wt%, 0 wt%-25 wt%, 1 wt%-38 wt%, 3 wt%-35 wt%, 5 wt%-32 wt%, 7 wt%-30 wt%, 8 wt%-25 wt%, 10 wt%-28 wt%, 12 wt%-25 wt%, 15 wt%-22 wt%, 18 wt%-25 wt%, 20 wt%-28 wt%, 23 wt%-30 wt%, 25 wt%-40 wt%.

In some embodiments, based on the mass of the core, the mass content of the disintegrant in the core can be 0 wt%-10 wt%, 0.5 wt%-10 wt%, 1 wt%-9.5 wt%, 1.5 wt%-9 wt%, 2 wt%-8.5 wt%, 2.5 wt%-8 wt%, 3 wt%-7.5 wt%, 3.5 wt%-7 wt%, 4 wt%-6.5 wt%, 4.5 wt%-6 wt%, 5 wt%-6.5 wt%, 5.5 wt%-7.5 wt%, 6 wt%-9 wt%, 1.5 wt%-6 wt%, 2 wt%-5.5 wt%, 2.5 wt%-5 wt%, 1.5 wt%-4.5 wt%, 0 wt%-5 wt%, 0 wt%-6 wt%, 0 wt%-8 wt%, 0 wt%-4.2 wt%.

In some embodiments, based on the mass of the core, the mass content of the diluent in the core can be 1 wt%-85 wt%, 2 wt%-80 wt%, 5 wt%-75 wt%, 10 wt%-70 wt%, 15 wt%-65 wt%, 20 wt%-60 wt%, 25 wt%-55 wt%, 30 wt%-50 wt%, 35 wt%-45 wt%, 1 wt%-70 wt%, 1 wt%-60 wt%, 1 wt%-50 wt%, 1 wt%-40 wt%, 1 wt%-30 wt%, 2 wt%-28 wt%, 5 wt%-25 wt%, 7 wt%-23 wt%, 10 wt%-20 wt%, 12 wt%-18 wt%, 15 wt%-20 wt%, 17 wt%-31 wt%, 17 wt%-22 wt%, 20 wt%-25 wt%, 23 wt%-28 wt%, 25 wt%-30 wt%.

In some embodiments, based on the mass of the core, the mass content of the binder in the core can be 0 wt%-7.5 wt%, 0 wt%-4.5 wt%, 0.5 wt%-7.5 wt%, 1 wt%-7 wt%, 1.5 wt%-6.5 wt%, 2 wt%-6 wt%, 2.5 wt%-5.5 wt%, 3 wt%-5 wt%, 2.5 wt%-4.5 wt%, 2 wt%-4 wt%, 1.5 wt%-3.5 wt%, 1 wt%-3 wt%, 0.5 wt%-2.5 wt%, 3.5 wt%-5.5 wt%, 4 wt%-6 wt%, 4.5 wt%-6.5 wt%, 5 wt%-7 wt%, 6.5 wt%-7.5 wt%.

In some embodiments, based on the mass of the core, the mass content of the lubricant in the core can be 0.5 wt%-5 wt%, 0.8 wt%-4.7 wt%, 1 wt%-4.5 wt%, 1.2 wt%-4.3 wt%, 1.5 wt%-4.0 wt%, 1.8 wt%-3.7 wt%, 2 wt%-3.5 wt%, 2.2 wt%-3.8 wt%, 2.5 wt%-4 wt%, 2.7 wt%-4.3 wt%, 3 wt%-4.5 wt%, 3.5 wt%-5 wt%.

In some embodiments, the delivery agent can be sodium N-[8-(2-hydroxybenzoyl)amino]octanoate (SNAC). In some embodiments, the auxiliary agent can be disodium hydrogen phosphate. In some embodiments, the diluent can comprise microcrystalline cellulose or mannitol. In some embodiments, the diluent can be microcrystalline cellulose and mannitol. In some embodiments, the binder can be hydroxypropyl cellulose or povidone. In some embodiments, the binder can be hydroxypropyl cellulose. In some embodiments, the lubricant can be magnesium stearate. In some embodiments, the disintegrant can be cross-linked povidone.

In some embodiments, the core and the outer layer are compressed together, and the outer layer completely encloses the core. In some embodiments, the core can be compressed first, and then a compression after the outer layer enclosing the core is performed. In some embodiments, the outer layer can be formed by compressing at least two tablets or granules up and down, with the core being located at the center of the outer layer, and the external surface of the core closely attached to the inner wall of the outer layer, forming two parts, an inner part and an outer part, with completely separated material components. In some embodiments, the core can be a solid body of any shape, including but not limited to at least one of a sphere, a cube, a cuboid, a cylinder, a cone, a truncated cone, a truncated pyramid, a prism, a pyramid, a polyhedron, an ellipse, and a capsule shape. In some embodiments, the outer layer can be a hollow body of any shape, including but not limited to at least one of a hollow sphere, a hollow cube, a hollow cuboid, a hollow cylinder, a hollow cone, a hollow truncated cone, a hollow truncated pyramid, a hollow prism, a hollow pyramid, a hollow polyhedron, a hollow ellipsoid, and a hollow capsule shape.

In some embodiments, the first granule can be prepared after homogeneously mixing the components comprised in the core. In some embodiments, the second granule can be prepared after homogeneously mixing the components comprised in the outer layer. In some embodiments, at least two second granules can be compressed against the first granule. In some embodiments, the first granule can be compressed by placing it between two second granules. In some embodiments, one second granule can be pre-compressed to obtain a pre-compressed tablet, one first granule is placed at the center of the pre-compressed tablet as a core, and then another second granule is placed on the core for compression to obtain a tablet. In some embodiments, one second granule can be pre-compressed to obtain a pre-compressed tablet, one first granule is placed at the center of the pre-compressed tablet as a core, and then another second granule is placed on the core on the opposite side of the pre-compressed tablet for compression to obtain a tablet.

In some embodiments, for a pharmaceutical composition comprising an outer layer and a core, the release initiation timepoint of the outer layer can be earlier than the release initiation timepoint of the core. In some embodiments, the release initiation timepoint of the outer layer or core is the time point, at which the outer layer or core begins to dissolve and release, in the dissolution profile of the pharmaceutical composition. In some embodiments, when the core begins to dissolve, the outer layer is not completely dissolved. In some embodiments, the release initiation timepoint of the core is 8 min-35 min later than the release initiation timepoint of the outer layer. In some embodiments, the release initiation timepoint of the core is 9 min-33 min later than the release initiation timepoint of the outer layer. In some embodiments, the release initiation timepoint of the core is 10 min-30 min later than the release initiation timepoint of the outer layer. In some embodiments, the release initiation timepoint of the core is 12 min-28 min later than the release initiation timepoint of the outer layer. In some embodiments, the release initiation timepoint of the core is 15 min-25 min later than the release initiation timepoint of the outer layer. In some embodiments, the release initiation timepoint of the core is 18 min-23 min later than the release initiation timepoint of the outer layer. In some embodiments, the release initiation timepoint of the core is 10 min-20 min later than the release initiation timepoint of the outer layer.

As used herein, "release initiation timepoint" refers to the time at which the drug ingredient begins to dissolve and release.

As used herein, "about" refers to any value within a variation of ±20%, such that "about 10" would include 8 to 12. Unless otherwise specified herein, recitation of ranges of values herein is merely intended to serve as a shorthand method of referring individually to each individual value falling within the range, and each individual value is incorporated into the specification as if it were individually recited herein. All methods described herein can be performed in any suitable order, unless otherwise specified herein or otherwise clearly contradicted by context.

"Therapeutically effective amount" or "effective amount" herein refers to an amount that can produce a desired response in a patient or individual in need thereof, including preventing or slowing the occurrence of a disease, alleviating the severity of a disease, improving the patient's condition, inducing an immune response, delaying or at least slowing the progression of a disease, or curing the disease, which can be determined by the skilled in the art in combination with the contents of the present disclosure based on the patient's or individual's age, gender, weight, physical condition, family history, risk of disease, and the like.

"Optional" or "optionally" means that the event or circumstance subsequently described may, but need not, occur, and the description includes a situation where the event or circumstance does or does not occur. "Optionally comprising" means that the object it modifies may or may not be comprised. For example, "the granules can optionally comprise a pharmaceutically acceptable non-active ingredient" means that the granules can comprise or not comprise the pharmaceutically acceptable non-active ingredient.

Unless otherwise clearly specified in the context, singular terms herein cover plural referents, and *vice versa*. Similarly, unless otherwise clearly specified in the context, the word "or" herein is intended to include "and".

"Pharmaceutically acceptable" refers to those compounds, materials, compositions and/or dosage forms that are, within the scope of sound medical judgment, suitable for use in contact with the tissues of humans and animals without undue toxicity, irritation, allergic response or other problems or complications, commensurate with a reasonable benefit-to-risk ratio.

The use of any and all examples, or exemplary language, provided herein is intended merely to illustrate certain materials and methods and is not intended to limit the scope. No language in the specification should be construed as indicating that any non-claimed element is necessary to the practice of the disclosed materials or methods.

As shown in the present application and the claims, unless an exception is specified clearly in the context, the words "a", "an", "a kind of", and/or "the" and the like do not specifically refer to the singular and may also include the plural. Generally, the terms "comprise", "comprises", and "comprising" as well as "contain", "contains", and "containing" or their equivalents (include, includes, and including), merely indicate that the steps and elements specifically identified are included, and these steps and elements do not constitute an exclusive list, and the method or composition may also include other steps or elements.

### EXAMPLES

Embodiments of the present invention will now be described with reference to the following examples. It should be understood that these examples are disclosed only by way of illustrating the present invention, and shall not be construed as limiting the scope of the present invention in any way. Unless otherwise specified, the reagents, materials, instruments, and the like involved in the following examples are commercially available or can be obtained according to conventional knowledge in the art.

### Example 1 - Tablet Composition A

0.1 g of difelikefalin acetate (calculated as difelikefalin, with a content of about 80%), 0.2 g of povidone K90, 6 g of microcrystalline cellulose, and 0.5 g of magnesium stearate were weighed, respectively. About 1/3 of microcrystalline cellulose and difelikefalin acetate were manually stirred and mixed for 30 s, and passed through a No. 4 standard sieve once; then povidone K90, magnesium stearate, and the remaining about 2/3 of microcrystalline cellulose were sequentially added thereto, and mixed in three dimensions for 10 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

30 g of SNAC, 15 g of anhydrous disodium hydrogen phosphate, 6 g of microcrystalline cellulose, and 0.8 g of magnesium stearate were weighed, respectively. Anhydrous disodium hydrogen phosphate, magnesium stearate, SNAC, and microcrystalline cellulose were sequentially added to a mixer and mixed in three dimensions for 20 min; and a single-punch tablet press was used to simulate dry granulation. The compression mold was a φ20 mm flat tablet, the fill weight was adjusted within the range of 1 g-2 g, and the tablet hardness was within the range of 2 kg-5 kg. After the compressing was completed, the mixture was milled, and qualified granules with a granule size between No. 2 and No. 5 standard sieves were collected and recorded as Granule II.

6.8 g of Granule I described above, 38.8 g of Granule II described above, and 0.1 g of magnesium stearate were mixed in three dimensions for 5 min, and compressing was performed using a single-punch tablet press to obtain tablets with a tablet weight of about 550 mg. The content of difelikefalin in each tablet was about 1 mg.

### Example 2 - Tablet Composition B

0.1 g of difelikefalin acetate (calculated as difelikefalin, with a content of about 80%), 0.2 g of povidone K90, 6 g of microcrystalline cellulose, and 0.5 g of magnesium stearate were weighed, respectively. About 1/3 of microcrystalline cellulose and difelikefalin acetate were manually stirred and mixed for 30 s, and passed through a No. 4 standard sieve once; then povidone K90, magnesium stearate, and the remaining about 2/3 of microcrystalline cellulose were sequentially added thereto, and mixed in three dimensions for 10 min; and a single-punch tablet press was used to simulate dry granulation. The compression mold was a 6 mm × 12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

30 g of SNAC, 6 g of microcrystalline cellulose, and 0.8 g of magnesium stearate were weighed, respectively. Magnesium stearate, SNAC, and microcrystalline cellulose were sequentially added to a mixer and mixed in three dimensions for 20 min; and a single-punch tablet press was used to simulate dry granulation. The compression mold was a φ20 mm flat tablet, the fill weight was adjusted within the range of 1 g-2 g, and the tablet hardness was within the range of 2 kg-5 kg. After the compressing was completed, the mixture was milled, and qualified granules with a granule size between No. 2 and No. 5 standard sieves were collected and recorded as Granule II.

6.8 g of Granule I described above, 25.2 g of Granule II described above, and 0.1 g of magnesium stearate were mixed in three dimensions for 5 min, and compressing was performed using a single-punch tablet press to obtain tablets with a tablet weight of about 400 mg. The content of difelikefalin in each tablet was about 1 mg.

The difference between Tablet Composition A and Tablet Composition B lies in that Tablet Composition B does not comprise anhydrous disodium hydrogen phosphate.

### Example 3 - Tablet Composition C

0.13 g of difelikefalin acetate (calculated as difelikefalin, with a content of about 80%), 30 g of SNAC, 15 g of anhydrous disodium hydrogen phosphate, 12 g of microcrystalline cellulose, and 1 g of magnesium stearate were weighed, respectively. About 1/3 of microcrystalline cellulose and difelikefalin acetate were manually stirred and mixed for 30 s, and passed through a No. 4 standard sieve once; then SNAC, anhydrous disodium hydrogen phosphate, magnesium stearate, and the remaining about 2/3 of microcrystalline cellulose were sequentially added thereto, and mixed in three dimensions for 10 min; compressing was performed using a single-punch tablet press to obtain tablets with a tablet weight of about 550 mg. The content of difelikefalin in each tablet was about 1 mg.

### Example 4 - Tablet Composition D

0.1 g of difelikefalin acetate (calculated as difelikefalin, with a content of about 80%), 0.2 g of povidone K90, 6 g of microcrystalline cellulose, and 0.5 g of magnesium stearate were weighed, respectively. About 1/3 of microcrystalline cellulose and difelikefalin acetate were manually stirred and mixed for 30 s, and passed through a No. 4 standard sieve once; then povidone K90, magnesium stearate, and the remaining about 2/3 of microcrystalline cellulose were sequentially added thereto, and mixed in three dimensions for 10 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

30 g of SNAC, 15 g of anhydrous disodium hydrogen phosphate, 6 g of microcrystalline cellulose, and 0.8 g of magnesium stearate were weighed, respectively. Anhydrous disodium hydrogen phosphate, magnesium stearate, SNAC, and microcrystalline cellulose were sequentially added to a mixer and mixed in three dimensions for 20 min; and a single-punch tablet press was used to simulate dry granulation. The compression mold was a φ20 mm flat tablet, the fill weight was adjusted within the range of 1 g-2 g, and the tablet hardness was within the range of 2 kg-5 kg. After the compressing was completed, the mixture was milled, and qualified granules with a granule size between No. 2 and No. 5 standard sieves were collected and recorded as Granule II.

6.8 g of Granule I described above, 38.53 g of Granule II described above, and 0.1 g of magnesium stearate were mixed in three dimensions for 5 min, and compressing was performed using a single-punch tablet press to obtain tablets with a tablet weight of about 140 mg. The content of difelikefalin in each tablet was about 0.25 mg.

### Example 5 - Tablet Composition E

0.1 g of difelikefalin acetate (calculated as difelikefalin, with a content of about 80%), 0.2 g of povidone K90, 6 g of microcrystalline cellulose, and 0.5 g of magnesium stearate were weighed, respectively. About 1/3 of microcrystalline cellulose and difelikefalin acetate were manually stirred and mixed for 30 s, and passed through a No. 4 standard sieve once; then povidone K90, magnesium stearate, and the remaining about 2/3 of microcrystalline cellulose were sequentially added thereto, and mixed in three dimensions for 10 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

30 g of SNAC, 6 g of microcrystalline cellulose, and 0.8 g of magnesium stearate were weighed, respectively. Magnesium stearate, SNAC, and microcrystalline cellulose were sequentially added to a mixer and mixed in three dimensions for 20 min; and a single-punch tablet press was used to simulate dry granulation. The compression mold was a φ20 mm flat tablet, the fill weight was adjusted within the range of 1 g-2 g, and the tablet hardness was within the range of 2 kg-5 kg. After the compressing was completed, the mixture was milled, and qualified granules with a granule size between No. 2 and No. 5 standard sieves were collected and recorded as Granule II.

6.8 g of Granule I described above, 25.6 g of Granule II described above, and 0.1 g of magnesium stearate were mixed in three dimensions for 5 min, and compressing was performed using a single-punch tablet press to obtain tablets with a tablet weight of about 100 mg. The content of difelikefalin in each tablet was about 0.25 mg.

The difference between Tablet Composition D and Tablet Composition E lies in that Tablet Composition E does not comprise anhydrous disodium hydrogen phosphate.

### Example 6 - Tablet Composition F

6 g of SNAC, 0.125 g of difelikefalin acetate (calculated as difelikefalin, with a content of about 80%), 0.1 g of hydroxypropyl cellulose, 3 g of microcrystalline cellulose, 0.5 g of cross-linked povidone, 3 g of anhydrous disodium hydrogen phosphate, 0.5 g of magnesium stearate were weighed, respectively. About 1/3 of microcrystalline cellulose and difelikefalin acetate were manually stirred and mixed for 30 s, and passed through a No. 4 standard sieve once; then hydroxypropyl cellulose, magnesium stearate, cross-linked povidone, SNAC, anhydrous disodium hydrogen phosphate, and the remaining microcrystalline cellulose were sequentially added thereto, and mixed in three dimensions for 15 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

14 g of SNAC, 3.5 g of anhydrous disodium hydrogen phosphate, 8 g of microcrystalline cellulose, 0.2 g of hydroxypropyl cellulose, and 0.6 g of magnesium stearate were weighed, respectively. SNAC, magnesium stearate, anhydrous disodium hydrogen phosphate, hydroxypropyl cellulose, and microcrystalline cellulose were sequentially added to a mixer, and mixed in three dimensions for 30 min; and a single-punch tablet press was used to simulate dry granulation. The compression mold was a φ20 mm flat tablet, the fill weight was adjusted within the range of 1 g-2 g, and the tablet hardness was within the range of 1 kg-3 kg. After the compressing was completed, the mixture was milled, and qualified granules with a granule size between No. 2 and No. 5 standard sieves were collected and recorded as Granule II.

The Granule I described above was compressed using a single-punch tablet press to obtain a core with a weight of about 140 mg.

The core described above was compressed with the Granule II described above. The specific operation was that about 180 mg of Granule II was weighed and pre-compressed in a single-punch tablet press; one core was then placed in the center of the pre-compressed tablet; about 180 mg of Granule II was weighed again, added to the mold, and compressed. The finished tablet consisted of an outer layer formed by Granule II and a core formed by Granule I, and the outer layer completely enclosed the core inside.

### Example 7 - Tablet Composition G

6 g of SNAC, 0.1 g of hydroxypropyl cellulose, 3 g of microcrystalline cellulose, 0.5 g of cross-linked povidone, 3 g of anhydrous disodium hydrogen phosphate, 0.5 g of magnesium stearate were weighed, respectively. SNAC, anhydrous disodium hydrogen phosphate, hydroxypropyl cellulose, magnesium stearate, cross-linked povidone, and microcrystalline cellulose were sequentially added to a mixer, and mixed in three dimensions for 15 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

14 g of SNAC, 0.095 g of difelikefalin acetate (calculated as difelikefalin, with a content of about 80%), 3.5 g of anhydrous disodium hydrogen phosphate, 8 g of microcrystalline cellulose, 0.2 g of hydroxypropyl cellulose, and 0.6 g of magnesium stearate were weighed, respectively. About 1/3 of microcrystalline cellulose and difelikefalin acetate were manually stirred and mixed for 30 s, and passed through a No. 4 standard sieve once. Then SNAC, magnesium stearate, anhydrous disodium hydrogen phosphate, hydroxypropyl cellulose, and the remaining microcrystalline cellulose were sequentially added thereto, and mixed in three dimensions for 30 min; and a single-punch tablet press was used to simulate dry granulation. The compression mold was a φ20 mm flat tablet, the fill weight was adjusted within the range of 1 g-2 g, and the tablet hardness was within the range of 1 kg-3 kg. After the compressing was completed, the mixture was milled, and qualified granules with a granule size between No. 2 and No. 5 standard sieves were collected and recorded as Granule II.

The Granule I described above was compressed using a single-punch tablet press to obtain a core with a weight of about 140 mg.

The core described above was compressed with the Granule II described above. The specific operation was that about 180 mg of Granule II was weighed and pre-compressed in a single-punch tablet press; one core was then placed in the center of the pre-compressed tablet; about 180 mg of Granule II was weighed again, added to the mold, and compressed. The finished tablet consisted of an outer layer formed by Granule II and a core formed by Granule I, and the outer layer completely enclosed the core inside.

### Example 8 - Tablet Composition H

8 g of SNAC, 0.125 g of difelikefalin acetate (calculated as difelikefalin, with a content of about 80%), 0.1 g of hydroxypropyl cellulose, 2.8 g of microcrystalline cellulose, 0.5 g of cross-linked povidone, 0.5 g of magnesium stearate were weighed, respectively. About 1/3 of microcrystalline cellulose and difelikefalin acetate were manually stirred and mixed for 30 s, and passed through a No. 4 standard sieve once; then hydroxypropyl cellulose, magnesium stearate, cross-linked povidone, SNAC, and the remaining microcrystalline cellulose were sequentially added thereto, and mixed in three dimensions for 15 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

12 g of SNAC, 7 g of anhydrous disodium hydrogen phosphate, 8 g of microcrystalline cellulose, 0.3 g of hydroxypropyl cellulose, and 0.7 g of magnesium stearate were weighed, respectively. SNAC, magnesium stearate, anhydrous disodium hydrogen phosphate, hydroxypropyl cellulose, and microcrystalline cellulose were sequentially added to a mixer, and mixed in three dimensions for 30 min; and a single-punch tablet press was used to simulate dry granulation. The compression mold was a φ20 mm flat tablet, the fill weight was adjusted within the range of 1 g-2 g, and the tablet hardness was within the range of 1 kg-3 kg. After the compressing was completed, the mixture was milled, and qualified granules with a granule size between No. 2 and No. 5 standard sieves were collected and recorded as Granule II.

The Granule I described above was compressed using a single-punch tablet press to obtain a core with a weight of about 120 mg.

The core described above was compressed with the Granule II described above. The specific operation was that about 140 mg of Granule II was weighed and pre-compressed in a single-punch tablet press; one core was then placed in the center of the pre-compressed tablet; about 140 mg of Granule II was weighed again, added to the mold, and compressed. The finished tablet consisted of an outer layer formed by Granule II and a core formed by Granule I, and the outer layer completely enclosed the core inside.

### Example 9 - Tablet Composition I

0.125 g of difelikefalin acetate (calculated as difelikefalin, with a content of about 80%), 0.5 g of cross-linked povidone, and 11.4 g of anhydrous calcium hydrogen phosphate were weighed, respectively. Difelikefalin acetate was passed through a No. 4 standard sieve once; then cross-linked povidone and anhydrous calcium hydrogen phosphate were sequentially added thereto, and mixed in three dimensions for 15 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

20 g of SNAC, 4 g of anhydrous calcium hydrogen phosphate, 3.5 g of microcrystalline cellulose, and 0.5 g of magnesium stearate were weighed, respectively. SNAC, magnesium stearate, anhydrous calcium hydrogen phosphate, and microcrystalline cellulose were sequentially added to a mixer and mixed in three dimensions for 30 min; and a single-punch tablet press was used to simulate dry granulation. The compression mold was a φ20 mm flat tablet, the fill weight was adjusted within the range of 1 g-2 g, and the tablet hardness was within the range of 1 kg-3 kg. After the compressing was completed, the mixture was milled, and qualified granules with a granule size between No. 2 and No. 5 standard sieves were collected and recorded as Granule II.

The Granule I described above was compressed using a single-punch tablet press to obtain a core with a weight of about 120 mg.

The core described above was compressed with the Granule II described above. The specific operation was that about 140 mg of Granule II was weighed and pre-compressed in a single-punch tablet press; one core was then placed in the center of the pre-compressed tablet; about 140 mg of Granule II was weighed again, added to the mold, and compressed. The finished tablet consisted of an outer layer formed by Granule II and a core formed by Granule I, and the outer layer completely enclosed the core inside.

### Example 10 - Tablet Composition J

0.125 g of difelikefalin acetate (calculated as difelikefalin, with a content of about 80%), 0.5 g of hydroxypropyl cellulose, 11 g of microcrystalline cellulose, 1 g of cross-linked povidone, and 0.3 g of magnesium stearate were weighed, respectively. About 1/3 of microcrystalline cellulose and difelikefalin acetate were manually stirred and mixed for 30 s, and passed through a No. 4 standard sieve once; then hydroxypropyl cellulose, magnesium stearate, cross-linked povidone, and the remaining microcrystalline cellulose were sequentially added thereto, and mixed in three dimensions for 15 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

16 g of SNAC, 6 g of anhydrous disodium hydrogen phosphate, 4.9 g of microcrystalline cellulose, 0.5 g of hydroxypropyl cellulose, and 0.6 g of magnesium stearate were weighed, respectively. SNAC, magnesium stearate, anhydrous disodium hydrogen phosphate, hydroxypropyl cellulose, and microcrystalline cellulose were sequentially added to the mixer, and mixed in three dimensions for 30 min; a single-punch tablet press was used to simulate dry granulation. The compression mold was a φ20 mm flat tablet, the fill weight was adjusted within the range of 1 g-2 g, and the tablet hardness was within the range of 1 kg-3 kg. After the compressing was completed, the mixture was milled, and qualified granules with a granule size between No. 2 and No. 5 standard sieves were collected and recorded as Granule II.

The Granule I described above was compressed using a single-punch tablet press to obtain a core with a weight of about 120 mg.

The core described above was compressed with the Granule II described above. The specific operation was that about 140 mg of Granule II was weighed and pre-compressed in a single-punch tablet press; one core was then placed in the center of the pre-compressed tablet; about 140 mg of Granule II was weighed again, added to the mold, and compressed. The finished tablet consisted of an outer layer formed by Granule II and a core formed by Granule I, and the outer layer completely enclosed the core inside.

### Example 11 - Tablet Composition K

6 g of SNAC, 0.121 g of difelikefalin acetate (calculated as difelikefalin, with a content of about 80%), 0.09 g of hydroxypropyl cellulose, 2.4 g of microcrystalline cellulose, 0.6 g of cross-linked povidone, 3 g of anhydrous disodium hydrogen phosphate, and 0.24 g of magnesium stearate were weighed, respectively. About 1/3 of microcrystalline cellulose and difelikefalin acetate were manually stirred and mixed for 30 s, and passed through a No. 4 standard sieve once; then hydroxypropyl cellulose, magnesium stearate, cross-linked povidone, SNAC, anhydrous disodium hydrogen phosphate, and the remaining microcrystalline cellulose were sequentially added thereto, and mixed in three dimensions for 15 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

14 g of SNAC, 7 g of anhydrous sodium carbonate, 5.6 g of microcrystalline cellulose, and 0.56 g of magnesium stearate were weighed, respectively. SNAC, magnesium stearate, anhydrous sodium carbonate, and microcrystalline cellulose were sequentially added to a mixer and mixed in three dimensions for 30 min; and a single-punch tablet press was used to simulate dry granulation. The compression mold was a φ20 mm flat tablet, the fill weight was adjusted within the range of 1 g-2 g, and the tablet hardness was within the range of 1 kg-3 kg. After the compressing was completed, the mixture was milled, and qualified granules with a granule size between No. 2 and No. 5 standard sieves were collected and recorded as Granule II.

The Granule I described above was compressed using a single-punch tablet press to obtain a core with a weight of about 125 mg.

The core described above was compressed with the Granule II described above. The specific operation was that about 138 mg of Granule II was weighed and pre-compressed in a single-punch tablet press; one core was then placed in the center of the pre-compressed tablet; and about 135 mg of Granule II was weighed again, added to the mold, and compressed. The finished tablet consisted of an outer layer formed by Granule II and a core formed by Granule I, and the outer layer completely enclosed the core inside.

### Example 12 - Tablet Composition L

0.125 g of difelikefalin acetate (calculated as difelikefalin, with a content of about 80%), 0.3 g of hydroxypropyl cellulose, and 13.3 g of anhydrous calcium hydrogen phosphate were weighed, respectively. About 1/3 of anhydrous calcium hydrogen phosphate and all difelikefalin acetate were manually stirred and mixed for 30 s, and passed through a No. 4 standard sieve once; then hydroxypropyl cellulose and the remaining anhydrous calcium hydrogen phosphate were sequentially added thereto, and mixed in three dimensions for 15 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

20 g of SNAC, 6 g of microcrystalline cellulose, and 0.6 g of magnesium stearate were weighed, respectively. SNAC, magnesium stearate, and microcrystalline cellulose were sequentially added to a mixer, and mixed in three dimensions for 30 min; and a single-punch tablet press was used to simulate dry granulation. The compression mold was a φ20 mm flat tablet, the fill weight was adjusted within the range of 1 g-2 g, and the tablet hardness was within the range of 1 kg-3 kg. After the compressing was completed, the mixture was milled, and qualified granules with a granule size between No. 2 and No. 5 standard sieves were collected and recorded as Granule II.

Granule I and Granule II described above were used to compress a three-layer tablet. The specific operation was that about 133 mg of Granule II was weighed and pre-compressed in a single-punch tablet press, 134 mg of Granule I was then weighed and pre-compressed in a single-punch tablet press, and finally 133 mg of Granule II was weighed and compressed in a single-punch tablet press. The resulting three tablets were then combined together to form a three-layer tablet.

### Example 13 - Tablet Composition M

10 g of SNAC, 0.125 g of difelikefalin acetate (calculated as difelikefalin, with a content of about 80%), 0.5 g of cross-linked povidone, 3 g of anhydrous disodium hydrogen phosphate, 6 g of mannitol, and 0.5 g of magnesium stearate were weighed, respectively. About 1/3 of mannitol and difelikefalin acetate were manually stirred and mixed for 30 s, and passed through a No. 4 standard sieve once; then SNAC, magnesium stearate, cross-linked povidone, anhydrous disodium hydrogen phosphate, and the remaining mannitol were sequentially added thereto, and mixed in three dimensions for 15 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

12 g of SNAC, 2 g of anhydrous disodium hydrogen phosphate, 2 g of microcrystalline cellulose, 2.5 g of mannitol, 1 g of cross-linked povidone, and 0.5 g of magnesium stearate were weighed, respectively. SNAC, magnesium stearate, cross-linked povidone, anhydrous disodium hydrogen phosphate, mannitol, and microcrystalline cellulose were sequentially added to a mixer, and mixed in three dimensions for 30 min; and a single-punch tablet press was used to simulate dry granulation. The compression mold was a φ20 mm flat tablet, the fill weight was adjusted within the range of 1 g-2 g, and the tablet hardness was within the range of 1 kg-3 kg. After the compressing was completed, the mixture was milled, and qualified granules with a granule size between No. 2 and No. 5 standard sieves were collected and recorded as Granule II.

The Granule I described above was compressed using a single-punch tablet press to obtain a core with a weight of about 200 mg.

The core described above was compressed with the Granule II described above. The specific operation was that about 100 mg of Granule II was weighed and pre-compressed in a single-punch tablet press; one core was then placed in the center of the pre-compressed tablet; and about 100 mg of Granule II was weighed again, added to the mold, and compressed. The finished tablet consisted of an outer layer formed by Granule II and a core formed by Granule I, and the outer layer completely enclosed the core inside.

### Example 14 - Tablet Composition N

10 g of SNAC, 0.125 g of difelikefalin acetate (calculated as difelikefalin, with a content of about 80%), 0.5 g of cross-linked povidone, 3 g of anhydrous disodium hydrogen phosphate, 6 g of mannitol, and 0.5 g of magnesium stearate were weighed, respectively. About 1/3 of mannitol and difelikefalin acetate were manually stirred and mixed for 30 s, and passed through a No. 4 standard sieve once; then SNAC, magnesium stearate, cross-linked povidone, anhydrous disodium hydrogen phosphate, and the remaining mannitol were sequentially added thereto, and mixed in three dimensions for 15 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

12 g of SNAC, 2 g of anhydrous disodium hydrogen phosphate, 2 g of microcrystalline cellulose, 2.5 g of mannitol, 1 g of cross-linked povidone, and 0.5 g of magnesium stearate were weighed, respectively. SNAC, magnesium stearate, cross-linked povidone, anhydrous disodium hydrogen phosphate, mannitol and microcrystalline cellulose were added to the mixer, and mixed in three dimensions for 30 min; and a single-punch tablet press was used to simulate dry granulation. The compression mold was a φ20 mm flat tablet, the fill weight was adjusted within the range of 1 g-2 g, and the tablet hardness was within the range of 1 kg-3 kg. After the compressing was completed, the mixture was milled, and qualified granules with a granule size between No. 2 and No. 5 standard sieves were collected and recorded as Granule II.

20 g of Granule I described above and 20 g of Granule II described above were mixed in three dimensions for 5 min, and compressing was performed using a single-punch tablet press to obtain tablets with a tablet weight of about 400 mg. The content of difelikefalin in each tablet was about 1 mg.

The difference between Tablet Composition M and Tablet Composition N lies in that Tablet Composition N is an ordinary tablet compressed after homogeneous mixing of Granule 1 and Granule 2.

### Example 15 - Tablet Composition O

10 g of SNAC, 0.125 g of difelikefalin acetate (calculated as difelikefalin, with a content of about 80%), 0.5 g of povidone K90, 4.6 g of microcrystalline cellulose, 5 g of anhydrous disodium hydrogen phosphate, and 0.3 g of magnesium stearate were weighed, respectively. About 1/3 of microcrystalline cellulose and difelikefalin acetate were manually stirred and mixed for 30 s, and passed through a No. 4 standard sieve once; then SNAC, magnesium stearate, anhydrous disodium hydrogen phosphate, povidone K90, and the remaining microcrystalline cellulose were sequentially added thereto, and mixed in three dimensions for 15 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

9 g of SNAC, 5 g of anhydrous disodium hydrogen phosphate, 4 g of mannitol, 1.4 g of cross-linked povidone, and 0.6 g of magnesium stearate were weighed, respectively. SNAC, magnesium stearate, anhydrous disodium hydrogen phosphate, cross-linked povidone, and mannitol were sequentially added to a mixer, and mixed in three dimensions for 30 min; and a single-punch tablet press was used to simulate dry granulation. The compression mold was a φ20 mm flat tablet, the fill weight was adjusted within the range of 1 g-2 g, and the tablet hardness was within the range of 1 kg-3 kg. After the compressing was completed, the mixture was milled, and qualified granules with a granule size between No. 2 and No. 5 standard sieves were collected and recorded as Granule II.

The Granule I described above was compressed using a single-punch tablet press to obtain a core with a weight of about 200 mg.

The core described above was compressed with the Granule II described above. The specific operation was that about 100 mg of Granule II was weighed and pre-compressed in a single-punch tablet press; one core was then placed in the center of the pre-compressed tablet; and about 100 mg of Granule II was weighed again, added to the mold, and compressed. The finished tablet consisted of an outer layer formed by Granule II and a core formed by Granule I, and the outer layer completely enclosed the core inside.

### Example 16 - Tablet Composition P

10 g of SNAC, 0.24 g of difelikefalin acetate (calculated as difelikefalin, with a content of about 80%), 0.8 g of hydroxypropyl cellulose, 1.8 g of mannitol, 1.8 g of microcrystalline cellulose, 5 g of anhydrous disodium hydrogen phosphate, and 0.5 g of magnesium stearate were weighed, respectively. About 1/3 of microcrystalline cellulose and difelikefalin acetate were manually stirred and mixed for 30 s, and passed through a No. 4 standard sieve once; then hydroxypropyl cellulose, mannitol, magnesium stearate, SNAC, anhydrous disodium hydrogen phosphate, and the remaining microcrystalline cellulose were sequentially added thereto, and mixed in three dimensions for 15 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

10 g of SNAC, 5 g of anhydrous disodium hydrogen phosphate, 1.8 g of microcrystalline cellulose, 1.8 g of mannitol, 0.8 g of hydroxypropyl cellulose, and 0.6 g of magnesium stearate were weighed, respectively. SNAC, magnesium stearate, anhydrous disodium hydrogen phosphate, hydroxypropyl cellulose, mannitol, and microcrystalline cellulose were sequentially added to a mixer and mixed in three dimensions for 30 min; and a single-punch tablet press was used to simulate dry granulation. The compression mold was a φ20 mm flat tablet, the fill weight was adjusted within the range of 1 g-2 g, and the tablet hardness was within the range of 1 kg-3 kg. After the compressing was completed, the mixture was milled, and qualified granules with a granule size between No. 2 and No. 5 standard sieves were collected and recorded as Granule II.

The Granule I described above was compressed using a single-punch tablet press to obtain a core with a weight of about 200 mg.

The core described above was compressed with the Granule II described above. The specific operation was that about 100 mg of Granule II was weighed and pre-compressed in a single-punch tablet press; one core was then placed in the center of the pre-compressed tablet; about 100 mg of Granule II was weighed again, added to the mold, and compressed. The finished tablet consisted of an outer layer formed by Granule II and a core formed by Granule I, and the outer layer completely enclosed the core inside. The content of difelikefalin in each tablet was about 2 mg.

### Example 17 - Tablet Composition Q

7.3 g of SNAC, 0.25 g of difelikefalin acetate (calculated as difelikefalin, with a content of about 80%), 1 g of mannitol, 1 g of microcrystalline cellulose, 1 g of anhydrous disodium hydrogen phosphate, 0.5 g of povidone K90, 0.2 g of cross-linked povidone, and 0.3 g of magnesium stearate were weighed, respectively. About 1/3 of microcrystalline cellulose and difelikefalin acetate were manually stirred and mixed for 30 s, and passed through a No. 4 standard sieve once; then mannitol, silica, magnesium stearate, SNAC, anhydrous disodium hydrogen phosphate, povidone K90, cross-linked povidone, and the remaining microcrystalline cellulose were sequentially added thereto, and mixed in three dimensions for 15 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

6 g of SNAC, 3 g of anhydrous disodium hydrogen phosphate, 1 g of microcrystalline cellulose, 1 g of silica, 0.5 g of povidone K90, 0.2 g of cross-linked povidone, and 0.3 g of magnesium stearate were weighed, respectively. SNAC, magnesium stearate, anhydrous disodium hydrogen phosphate, povidone K90, cross-linked povidone, silica, and microcrystalline cellulose were sequentially added to a mixer and mixed in three dimensions for 30 min; and a single-punch tablet press was used to simulate dry granulation. The compression mold was a φ20 mm flat tablet, the fill weight was adjusted within the range of 1 g-2 g, and the tablet hardness was within the range of 1 kg-3 kg. After the compressing was completed, the mixture was milled, and qualified granules with a granule size between No. 2 and No. 5 standard sieves were collected and recorded as Granule II.

The Granule I described above was compressed using a single-punch tablet press to obtain a core with a weight of about 120 mg.

The core described above was compressed with the Granule II described above. The specific operation was that about 60 mg of Granule II was weighed and pre-compressed in a single-punch tablet press; one core was then placed in the center of the pre-compressed tablet; and about 60 mg of Granule II was weighed again, added to the mold, and compressed. The finished tablet consisted of an outer layer formed by Granule II and a core formed by Granule I, and the outer layer completely enclosed the core inside. The content of difelikefalin in each tablet was about 2 mg.

### Example 18 - Tablet Composition R

6 g of SNAC, 0.12 g of difelikefalin acetate (calculated as difelikefalin, with a content of about 80%), 0.1 g of hydroxypropyl cellulose, 3 g of microcrystalline cellulose, 0.5 g of cross-linked povidone, 3 g of anhydrous disodium hydrogen phosphate, and 0.5 g of magnesium stearate were weighed, respectively. About 1/3 of microcrystalline cellulose and difelikefalin acetate were manually stirred and mixed for 30 s, and passed through a No. 4 standard sieve once; then hydroxypropyl cellulose, magnesium stearate, cross-linked povidone, SNAC, anhydrous disodium hydrogen phosphate, and the remaining microcrystalline cellulose were sequentially added thereto, and mixed in three dimensions for 15 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

3.5 g of anhydrous disodium hydrogen phosphate, 11 g of microcrystalline cellulose, 11 g of mannitol, 0.2 g of hydroxypropyl cellulose, and 0.6 g of magnesium stearate were weighed, respectively. Magnesium stearate, anhydrous disodium hydrogen phosphate, hydroxypropyl cellulose, mannitol, and microcrystalline cellulose were sequentially added to a mixer, and mixed in three dimensions for 30 min; and a single-punch tablet press was used to simulate dry granulation. The compression mold was a φ20 mm flat tablet, the fill weight was adjusted within the range of 1 g-2 g, and the tablet hardness was within the range of 1 kg-3 kg. After the compressing was completed, the mixture was milled, and qualified granules with a granule size between No. 2 and No. 5 standard sieves were collected and recorded as Granule II.

The Granule I described above was compressed using a single-punch tablet press to obtain a core with a weight of about 140 mg.

The core described above was compressed with the Granule II described above. The specific operation was that about 180 mg of Granule II was weighed and pre-compressed in a single-punch tablet press; one core was then placed in the center of the pre-compressed tablet; and about 180 mg of Granule II was weighed again, added to the mold, and compressed. The finished tablet consisted of an outer layer formed by Granule II and a core formed by Granule I, and the outer layer completely enclosed the core inside.

### Example 19 - Tablet Composition S

6 g of SNAC, 0.12 g of difelikefalin acetate (calculated as difelikefalin, with a content of about 80%), 0.1 g of hydroxypropyl cellulose, 3 g of microcrystalline cellulose, 0.5 g of cross-linked povidone, 3 g of anhydrous disodium hydrogen phosphate, and 0.5 g of magnesium stearate were weighed, respectively. About 1/3 of microcrystalline cellulose and difelikefalin acetate were manually stirred and mixed for 30 s, and passed through a No. 4 standard sieve once; then hydroxypropyl cellulose, magnesium stearate, cross-linked povidone, SNAC, anhydrous disodium hydrogen phosphate, and the remaining microcrystalline cellulose were sequentially added thereto, and mixed in three dimensions for 15 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

12 g of microcrystalline cellulose, 12 g of mannitol, 0.2 g of hydroxypropyl cellulose, and 0.6 g of magnesium stearate were weighed, respectively. Magnesium stearate, hydroxypropyl cellulose, mannitol, and microcrystalline cellulose were sequentially added to a mixer, and mixed in three dimensions for 30 min; and a single-punch tablet press was used to simulate dry granulation. The compression mold was a φ20 mm flat tablet, the fill weight was adjusted within the range of 1 g-2 g, and the tablet hardness was within the range of 1 kg-3 kg. After the compressing was completed, the mixture was milled, and qualified granules with a granule size between No. 2 and No. 5 standard sieves were collected and recorded as Granule II.

The Granule I described above was compressed using a single-punch tablet press to obtain a core with a weight of about 140 mg.

The core described above was compressed with the Granule II described above. The specific operation was that about 180 mg of Granule II was weighed and pre-compressed in a single-punch tablet press; one core was then placed in the center of the pre-compressed tablet; and about 180 mg of Granule II was weighed again, added to the mold, and compressed. The finished tablet consisted of an outer layer formed by Granule II and a core formed by Granule I, and the outer layer completely enclosed the core inside.

### Example 20 - Tablet Composition T

6 g of SNAC, 0.125 g of difelikefalin acetate (calculated as difelikefalin, with a content of about 80%), 4 g of maltodextrin, and 3 g of anhydrous disodium hydrogen phosphate were weighed, respectively. About 1/3 of maltodextrin and difelikefalin acetate were manually stirred and mixed for 30 s, and passed through a No. 4 standard sieve once; then SNAC, anhydrous disodium hydrogen phosphate, and the remaining maltodextrin were sequentially added thereto, and mixed in three dimensions for 15 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

14 g of SNAC, 3.5 g of anhydrous disodium hydrogen phosphate, and 8 g of maltodextrin were weighed, respectively. SNAC, anhydrous disodium hydrogen phosphate, and maltodextrin were sequentially added to a mixer, and mixed in three dimensions for 30 min; and a single-punch tablet press was used to simulate dry granulation. The compression mold was a φ20 mm flat tablet, the fill weight was adjusted within the range of 1 g-2 g, and the tablet hardness was within the range of 1 kg-3 kg. After the compressing was completed, the mixture was milled, and qualified granules with a granule size between No. 2 and No. 5 standard sieves were collected and recorded as Granule II.

The Granule I described above was compressed using a single-punch tablet press to obtain a core with a weight of about 140 mg.

The core described above was compressed with the Granule II described above. The specific operation was that about 180 mg of Granule II was weighed and pre-compressed in a single-punch tablet press; one core was then placed in the center of the pre-compressed tablet; and about 180 mg of Granule II was weighed again, added to the mold, and compressed. The finished tablet consisted of an outer layer formed by Granule II and a core formed by Granule I, and the outer layer completely enclosed the core inside.

### Example 21 - Tablet Composition U

6 g of SNAC, 0.125 g of difelikefalin acetate (calculated as difelikefalin, with a content of about 80%), 0.1 g of hydroxypropyl cellulose, 3 g of lactose, 0.5 g of cross-linked povidone, 3 g of anhydrous disodium hydrogen phosphate, and 0.5 g of sodium stearyl fumarate were weighed, respectively. About 1/3 of lactose and difelikefalin acetate were manually stirred and mixed for 30 s, and passed through a No. 4 standard sieve once; then hydroxypropyl cellulose, sodium stearyl fumarate, cross-linked povidone, SNAC, anhydrous disodium hydrogen phosphate, and the remaining lactose were sequentially added thereto, and mixed in three dimensions for 15 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

14 g of SNAC, 8 g of lactose, 0.2 g of hydroxypropyl cellulose, and 0.6 g of sodium stearyl fumarate were weighed, respectively. SNAC, sodium stearyl fumarate, hydroxypropyl cellulose, and lactose were sequentially added to a mixer, and mixed in three dimensions for 30 min; and a single-punch tablet press was used to simulate dry granulation. The compression mold was a φ20 mm flat tablet, the fill weight was adjusted within the range of 1 g-2 g, and the tablet hardness was within the range of 1 kg-3 kg. After the compressing was completed, the mixture was milled, and qualified granules with a granule size between No. 2 and No. 5 standard sieves were collected and recorded as Granule II.

The Granule I described above was compressed using a single-punch tablet press to obtain a core with a weight of about 140 mg.

The core described above was compressed with the Granule II described above. The specific operation was that about 180 mg of Granule II was weighed and pre-compressed in a single-punch tablet press; one core was then placed in the center of the pre-compressed tablet; and about 180 mg of Granule II was weighed again, added to the mold, and compressed. The finished tablet consisted of an outer layer formed by Granule II and a core formed by Granule I, and the outer layer completely enclosed the core inside.

### Example 22 - Tablet Composition V

6 g of SNAC, 1.6 g of difelikefalin acetate (calculated as difelikefalin, with a content of about 80%), 0.1 g of hydroxypropyl cellulose, 2 g of microcrystalline cellulose, 2 g of mannitol, 0.8 g of croscarmellose sodium, 3 g of anhydrous disodium hydrogen phosphate, 0.5 g of magnesium stearate were weighed, respectively. About 1/3 of microcrystalline cellulose and difelikefalin acetate were manually stirred and mixed for 30 s, and passed through a No. 4 standard sieve once; then hydroxypropyl cellulose, magnesium stearate, croscarmellose sodium, SNAC, anhydrous disodium hydrogen phosphate, mannitol, and the remaining microcrystalline cellulose were sequentially added thereto, and mixed in three dimensions for 15 min; and a single-punch tablet press was used to simulate dry granulation. The compression mold was a 6 mm×12 mm capsule-shaped shallow concave tablet, the fill weight was adjusted within the range of 100 mg-300 mg, and the tablet hardness was within the range of 5 kg-10 kg. After the compressing was completed, the mixture was milled, and qualified granules with a granule size between No. 2 and No. 5 standard sieves were collected and recorded as Granule I.

14 g of SNAC, 4 g of microcrystalline cellulose, 4 g of mannitol, 0.2 g of hydroxypropyl cellulose, 0.6 g of magnesium stearate, 5 g of anhydrous disodium hydrogen phosphate, and 1.2 g of croscarmellose sodium were weighed, respectively. SNAC, magnesium stearate, hydroxypropyl cellulose, microcrystalline cellulose, anhydrous disodium hydrogen phosphate, croscarmellose sodium, and mannitol were sequentially added to a mixer, and mixed in three dimensions for 30 min; and a single-punch tablet press was used to simulate dry granulation. The compression mold was a φ20 mm flat tablet, the fill weight was adjusted within the range of 1 g-2 g, and the tablet hardness was within the range of 1 kg-3 kg. After the compressing was completed, the mixture was milled, and qualified granules with a granule size between No. 2 and No. 5 standard sieves were collected and recorded as Granule II.

The Granule I described above was compressed using a single-punch tablet press to obtain a core with a weight of about 140 mg.

The core described above was compressed with the Granule II described above. The specific operation was that about 180 mg of Granule II was weighed and pre-compressed in a single-punch tablet press; one core was then placed in the center of the pre-compressed tablet; and about 180 mg of Granule II was weighed again, added to the mold, and compressed. The finished tablet consisted of an outer layer formed by Granule II and a core formed by Granule I, and the outer layer completely enclosed the core inside.

### Example 23 - Animal Pharmacokinetic Experiment

Healthy male beagle dogs were selected as test animals, and were randomly grouped before the experiment. Feeding and experimental conditions: temperature 20°C - 26°C (daily temperature difference ≤ 4°C), relative humidity 40%-70%, with artificial lighting of a 12/12 hour day and night alternation of light and dark, the grouped animals were allowed to acclimatize to the conditions for at least 5 days prior to the experiment.

The beagle dogs in group A were administered Tablet Composition D, the beagle dogs in group B were administered Tablet Composition E, the beagle dogs in group C were administered Tablet Composition F, and the beagle dogs in group D were administered Tablet Composition G. The actual dosage administered to each animal was one tablet each time. Administration was performed by oral gavage. Specifically, the tablet was placed in the dog's throat, the mouth was closed, and 10 mL of purified water was immediately administered after the dog swallowed on its own. The dogs were fixed in a standing position before administration and were released 1 hr after administration.

Fasting: fasting was conducted overnight prior to the test day (T1), the fasting was initiated no later than 8 hr before administration, and first feeding was conducted 4 hr after administration. Water deprivation: water deprivation was initiated no later than 1 hr before administration, and free access to water was restored 2 hr after administration.

Observation during the feeding and experimental periods: including but not limited to the animal's appearance and physical signs, fur, general behavior and activity, mental state, glandular secretions, color of the skin and mucous membranes, respiratory status, fecal characteristics, genitals, death, and the like. If any abnormalities are observed, it shall be determined whether to terminate the experiment.

Sampling was performed 15 min, 30 min, 1 h, 2 h, 3 h, 4 h, 6 h, 8 h, and 12 h after administration. The sampling site was the forelimb vein, and the blood collection volume was no less than 0.5 mL of plasma per time (about 1 mL of blood was collected at each time point, and no less than 0.5 mL of plasma was obtained after anticoagulation and centrifugation for plasma concentration detection). The anticoagulant EDTA was added to the collected samples. After collection of the samples at each time point was completed, the plasma was separated after centrifugation at about 3000 r/min for 10 minutes, and the concentration of difelikefalin in the plasma was detected.

The individual data and relative standard deviation (RSD) of the maximum plasma concentration (Cₘₐₓ), time to peak plasma concentration (Tₘₐₓ), area under the curve (AUCₗₐₛₜ), and absolute bioavailability (F%) of the beagle dogs in different test groups are shown in Table 1 to Table 4 below.

**Table 1 - Tablet Composition D - Specification 0.25 mg**

| Beagle dog No. | #1 | #2 | #3 | #4 | #5 | #6 | Mean | RSD |
|---|---|---|---|---|---|---|---|---|
| Cₘₐₓ (ng/mL) | 15.0 | 10.2 | 3.1 | 8.7 | 9.0 | 1.3 | 7.88 | 63.2% |
| Tₘₐₓ (h) | 0.5 | 1 | 1 | 1 | 1 | 1 | 0.92 | / |
| AUCₗₐₛₜ (h* ng/mL) | 55.6 | 27.2 | 13.5 | 35.6 | 28.4 | 4.1 | 27.41 | 65.3% |
| F% | 9.22% | | | | | | | |

**Table 2 - Tablet Composition E - Specification 0.25 mg**

| Beagle dog No. | #1 | #2 | #3 | #4 | #5 | #6 | Mean | RSD |
|---|---|---|---|---|---|---|---|---|
| Cₘₐₓ (ng/mL) | 10.0 | 9.8 | 11.7 | 4.6 | 1.4 | 0.7 | 7.50 | 74.8% |
| Tₘₐₓ (h) | 1 | 2 | 1 | 1 | 2 | 0.25 | 1.40 | / |
| AUCₗₐₛₜ (h* ng/mL) | 33.25 | 30.51 | 41.6 | 15.24 | 7.39 | 7.4 | 25.60 | 64.3% |
| F% | 7.93% | | | | | | | |

**Table 3 - Tablet Composition F - Specification 1.0 mg**

| Beagle dog No. | #1 | #2 | #3 | #4 | #5 | Mean | RSD |
|---|---|---|---|---|---|---|---|
| Cₘₐₓ (ng/mL) | 33.60 | 41.30 | 61.70 | 46.30 | 68.80 | 50.34 | 28.9% |
| Tₘₐₓ (h) | 2.00 | 1.00 | 1.00 | 1.00 | 1.00 | 1.20 | / |
| AUCₗₐₛₜ (h* ng/mL) | 144.44 | 130.80 | 263.69 | 126.12 | 196.28 | 172.27 | 33.8% |
| F% | 15.87% | | | | | | |

**Table 4 - Tablet Composition G (API in the outer layer) - Specification 1.0 mg**

| Beagle dog No. | #1 | #2 | #3 | #4 | #5 | Mean | RSD |
|---|---|---|---|---|---|---|---|
| Cₘₐₓ (ng/mL) | 36.40 | 4.80 | 13.20 | 4.40 | 36.90 | 19.14 | 85.5% |
| Tₘₐₓ (h) | 1.00 | 1.00 | 2.00 | 1.00 | 1.00 | 1.20 | / |
| AUCₗₐₛₜ (h* ng/mL) | 79.18 | 22.05 | 50.84 | 21.69 | 67.28 | 48.21 | 54.1% |
| F% | 4.70% | | | | | | |

Comparing Table 1 with Table 2, it can be seen that the RSD variability degree of Cₘₐₓ in Table 1 was lower than the RSD variability degree of Cₘₐₓ in Table 2, and the absolute bioavailability in Table 1 was higher than the absolute bioavailability F% in Table 2. Therefore, compared with the beagle dogs administered the drug without the auxiliary agent (i.e., disodium hydrogen phosphate) in Table 2, the variability degree of the peak plasma concentration Cₘₐₓ of difelikefalin among the beagle dogs administered the drug comprising the auxiliary agent in Table 1 reduced and the absolute bioavailability F% increased. Thus, compared with the composition comprising difelikefalin and a delivery agent without an auxiliary agent, when difelikefalin, a delivery agent, and an auxiliary agent were taken as a composition, the variability degree of the peak plasma concentration Cₘₐₓ of difelikefalin among individuals could be effectively reduced and the absolute bioavailability F% could be increased. That is, the auxiliary agent in the present disclosure can effectively reduce the variability degree of the peak plasma concentration Cₘₐₓ of difelikefalin among individuals and increase the absolute bioavailability F%.

Comparing Table 1 with Table 3, it can be seen that, in Table 1, the RSD variability degree of Cₘₐₓ was 63.2%, the RSD variability degree of AUCₗₐₛₜ was 65.3%, the absolute bioavailability was 9.22%; and in Table 3, the RSD variability degree of Cₘₐₓ was reduced to 28.9%, the RSD variability degree of AUCₗₐₛₜ was reduced to 33.8%, and the absolute bioavailability was 15.87%. Therefore, compared with the beagle dogs administered the ordinary tablet in Table 1, the drug administered in Table 3 was the tablet with a specific structure (including the outer layer and the core) of the present disclosure, after the administration thereof, the variability degrees of the peak plasma concentration Cₘₐₓ of difelikefalin and the bioavailability represented by AUCₗₐₛₜ among the beagle dogs significantly reduced, and the absolute bioavailability F% significantly increased. Thus, compared with the pharmaceutical composition in the form of an ordinary tablet, when the pharmaceutical composition with a specific structure of the present disclosure was taken, the variability degrees of the peak plasma concentration Cₘₐₓ of difelikefalin and the bioavailability represented by AUCₗₐₛₜ among individuals could be effectively reduced, and the absolute bioavailability F% could be effectively increased. That is, the pharmaceutical composition with a specific structure of the present disclosure can effectively reduce the variability degrees of the peak plasma concentration Cₘₐₓ of difelikefalin and the bioavailability represented by AUCₗₐₛₜ among individuals, and increase the absolute bioavailability F%.

Comparing Table 3 with Table 4, it can be seen that the RSD variability degree of Cₘₐₓ in Table 4 was 85.5%, the RSD variability degree of AUCₗₐₛₜ was 54.1%, the absolute bioavailability F% was 4.70%; and the RSD variability degree of Cₘₐₓ in Table 3 was reduced to 28.9%, the RSD variability degree of AUCₗₐₛₜ was reduced to 33.8%, and the absolute bioavailability F% was 15.87%. Therefore, compared with the beagle dogs administered the drug in which difelikefalin was set in the outer layer in Table 4, the variability degrees of the peak plasma concentration Cₘₐₓ of difelikefalin and the bioavailability represented by AUCₗₐₛₜ among the beagle dogs administered the drug in which difelikefalin was set in the core in Table 3 significantly reduced, and the absolute bioavailability F% significantly increased. Thus, for the pharmaceutical composition with a structure of an outer layer and a core, compared with the pharmaceutical composition in which difelikefalin was set in the outer layer, when the pharmaceutical composition in which difelikefalin was set in the core was taken, the variability degrees of the peak plasma concentration Cₘₐₓ of difelikefalin and the bioavailability represented by AUCₗₐₛₜ among individuals could be effectively reduced, and the absolute bioavailability F% could be effectively increased. That is, the pharmaceutical composition of the present disclosure in which difelikefalin is set in the core can effectively reduce the variability degrees of the peak plasma concentration Cₘₐₓ of difelikefalin and the bioavailability represented by AUCₗₐₛₜ among individuals, and increase the absolute bioavailability F%.

The compositions H, I, J, K, L, M, N, O, P, Q, R, S, and T prepared above were determined according to the pharmacokinetic experimental method in Example 20 as described above. The experimental results showed that, similar to the differences in bioavailability and variability degree between Tablet Composition D (an ordinary tablet) and Tablet Composition F (a tablet with the specific structure of the present disclosure), the absolute bioavailability F% of Tablet Composition M, Tablet Composition O, Tablet Composition P, and Tablet Composition Q (tablets with the specific structure of the present disclosure) was 15.5%-17.2%, which was significantly increased by at least 60% compared with Tablet Composition N (an ordinary tablet), and the variability degree of AUCₗₐₛₜ of Tablet Composition M, Tablet Composition O, Tablet Composition P, and Tablet Composition Q was reduced by 27.4%-57.2% compared with the variability degree of Tablet Composition N. In addition, compared with Tablet Composition F, the RSD variability degree of AUCₗₐₛₜ of Tablet Composition H (with a core comprising a delivery agent but no auxiliary agent, and an outer layer comprising a delivery agent and an auxiliary agent) was basically unchanged, and the absolute bioavailability F% was reduced by 16.2%. The RSD variability degree of AUCₗₐₛₜ of Tablet Composition K (the auxiliary agent of the outer layer was anhydrous sodium carbonate) was 45%, and the absolute bioavailability F% was 4.5%. The RSD variability degree of AUCₗₐₛₜ of Tablet Composition L (three-layer tablet structure) was 54.1%, and the absolute bioavailability F% was only 3.73%. The RSD variability degree of AUCₗₐₛₜ of Tablet Composition I (with a core comprising no delivery agent but comprising an auxiliary agent, and an outer layer comprising a delivery agent and an auxiliary agent) was 78.6%, and the absolute bioavailability F% was 5.03%. The RSD variability degree of AUCₗₐₛₜ of Tablet Composition J (with a core comprising no delivery agent and auxiliary agent, and an outer layer comprising a delivery agent and an auxiliary agent) was 84%, and the absolute bioavailability F% was 3.92%. The RSD variability degree of AUCₗₐₛₜ of Tablet Composition R (with a core comprising an auxiliary agent and a delivery agent, and an outer layer comprising no delivery agent but comprising an auxiliary agent) was 41%, and the absolute bioavailability F% was 6.34%. The RSD variability degree of AUCₗₐₛₜ of Tablet Composition S (with a core comprising a delivery agent and an auxiliary agent, and an outer layer comprising no delivery agent and auxiliary agent) was 49%, and the absolute bioavailability F% was 4.18%. The RSD variability degrees of AUCₗₐₛₜ and the absolute bioavailabilities F% of Tablet Composition T (with a core only having difelikefalin, a delivery agent, an auxiliary agent, and a diluent, and an outer layer only having a delivery agent, an auxiliary agent, and a diluent), Tablet Composition U (with a core having a delivery agent and an auxiliary agent, and an outer layer comprising a delivery agent but no auxiliary agent), and Tablet Composition V were essentially equivalent to those of Tablet Composition F, Tablet Composition M, Tablet Composition O, Tablet Composition P, and Tablet Composition Q.

### Example 24 - Animal Pharmacodynamic Study

Male C57BL/6J mice were acclimatized to the conditions for at least 7 days prior to the experiment and randomly divided into 4 groups: a negative control group (administered normal saline, no modeling), a pruritus model group (administered normal saline, modeling), a commercially available difelikefalin injection group (0.25 mg/kg, calculated as difelikefalin, the drug's trade name is KORSUVA^{™}, administered by intravenous injection, modeling), and an oral preparation group (prepared according to the proportion of Tablet Composition F, 24 mg/kg, calculated as difelikefalin, administered by gavage, modeling), with 12 mice in each group. The animals were first administered depending on their groups, and the model was established about 15 min after the administration (0.3 mg/kg 5'GNTI was injected subcutaneously at the back of the neck to induce pruritus). After the model was established, a 30-min video recording was collected for each animal, and the number of scratching behaviors caused by pruritus within 30 min was manually counted (raising a hind paw to quickly scratch ears, neck, or lateral back, followed by lowering or gnawing the hind paw, was counted as one scratching). Compared with the untreated pruritus model group, the number of scratching was reduced in animals administered difelikefalin orally or by injection in advance. A fewer average number of scratching in each group within 30 min indicates a stronger anti-pruritic efficacy of the drug.

Results: the average number of scratching in the negative control group was 20±17 (n=12), and the average number of scratching in the pruritus model group was 345±168 (n=12). The number of scratching was significantly increased compared with the negative control group (P<0.001), indicating that the model was successfully established.

When commercially available difelikefalin injection was administered to pruritus mice 15 min in advance, the average number of scratching decreased to 144±166, which was significantly lower than that of the pruritus model group (P<0.01), indicating that difelikefalin could significantly inhibit pruritus in mice through intravenous injection.

When the oral preparation of difelikefalin was administered to pruritus mice 15 min in advance, the average number of scratching decreased to 110±119 (P<0.01), indicating that the oral preparation of difelikefalin could achieve efficacy similar to that of difelikefalin injection.

**Table 5 - Results of the in vivo pruritus animal model experiment**

| No. | Group | Average number of scratching (n=12) |
|---|---|---|
| 1 | Negative control group | 20±17 |
| 2 | Pruritus model group | 345±168^{###} |
| 3 | Commercially available difelikefalin injection group | 144±166^{**} |
| 4 | Difelikefalin oral preparation group | 110±119^{**} |

| | | |
|---|---|---|
| Note: The data in the table are expressed as mean ± standard deviation (Mean ± SD); compared with the negative control group, ###P<0.001; compared with the model group, **P<0.01, ***P<0.001. | | |

The basic concepts have been described above. It is evident to the skilled in the art that the above detailed disclosure is provided by way of example only and does not constitute a limitation to the present disclosure. Although it is not expressly stated herein, various modifications, improvements, and amendments to the present disclosure may be made by the skilled in the art. Such modifications, improvements and amendments are suggested in the present disclosure. Therefore, such modifications, improvements, and amendments still fall within the spirit and scope of the exemplary embodiments of the present disclosure.

Meanwhile, the present disclosure uses specific words to describe the embodiments of the present disclosure. For example, "in some embodiments", "in various embodiments", "one embodiment", "an embodiment", and/or "some embodiments" mean a certain feature, structure or characteristic related to at least one embodiment of the present disclosure. Therefore, it should be emphasized and noted that two or more references to "in some embodiments", "in various embodiments", "one embodiment", "an embodiment", and/or "some embodiments" in different positions of the present disclosure do not necessarily refer to the same embodiment. Furthermore, certain features, structures, or characteristics in one or more embodiments of the present disclosure may be appropriately combined.

Although some embodiments of the invention currently considered useful have been discussed through various examples in the above disclosure, it should be understood that such details are for illustrative purposes only, and the appended claims are not limited to the disclosed embodiments. Instead, the claims are intended to cover all modifications and equivalent combinations that are consistent with the spirit and scope of the embodiments of the present disclosure.

Similarly, it should be noted that in order to simplify the description of the present disclosure and thereby facilitating the understanding of one or more embodiments of the invention, various features may sometimes be combined into a single embodiment in the previous descriptions of embodiments of the present disclosure. However, this method of disclosure does not imply that the object of the present disclosure requires more features than those mentioned in the claims.

For the purposes of description and disclosure, all patents, patent applications, and other publications are hereby expressly incorporated herein by reference. These publications are provided solely because their publication dates are prior to the filing date of the present application. All statements regarding the dates of these documents or expressions as to contents of these documents are based on the information available to the applicant and do not constitute any admission regarding the accuracy of the dates or contents of these documents. Furthermore, any reference to these publications herein does not constitute an admission that the publications form part of the common general knowledge in the art in any country.

Finally, it should be understood that the embodiments described in the present disclosure are only used to illustrate the principles of the embodiments of the present disclosure. Other variations may also fall within the scope of the present disclosure. Therefore, by way of example but not limitation, alternative compositions of the disclosed embodiments may be considered consistent with the teachings of the present disclosure. Accordingly, the embodiments of the present disclosure are not limited to those clearly introduced and described in the present disclosure.

## Claims

1. A difelikefalin oral pharmaceutical composition, comprising an outer layer and a core, wherein the outer layer encloses the core, and wherein
the core comprises difelikefalin and a delivery agent; and
the outer layer comprises a delivery agent.

2. The pharmaceutical composition of claim 1, wherein the core further comprises an auxiliary agent, and/or the outer layer further comprises an auxiliary agent.

3. A difelikefalin oral pharmaceutical composition, wherein the pharmaceutical composition comprises an outer layer and a core, and the outer layer encloses the core, and wherein
the outer layer does not comprise difelikefalin,
the outer layer comprises a delivery agent, an auxiliary agent, and optionally a pharmaceutically acceptable non-active ingredient; and
the core comprises difelikefalin and optionally a pharmaceutically acceptable non-active ingredient.

4. The pharmaceutical composition of claim 3, wherein the core further comprises a delivery agent and/or an auxiliary agent.

5. The pharmaceutical composition of any one of claims 1 to 4, wherein the core further comprises a diluent.

6. The pharmaceutical composition of any one of claims 1 to 5, wherein the core further comprises a lubricant.

7. The pharmaceutical composition of any one of claims 1 to 6, wherein the core further comprises a disintegrant and/or a binder.

8. The pharmaceutical composition of any one of claims 1 to 7, wherein the core comprises difelikefalin, a delivery agent, an auxiliary agent, and one or more of a diluent, a lubricant, a disintegrant, and a binder; and/or
the outer layer comprises a delivery agent and one or more of an auxiliary agent, a diluent, a lubricant, a disintegrant, and a binder.

9. The pharmaceutical composition of any one of claims 1 to 8, wherein the delivery agent is one or more selected from:
N-[8-(2-hydroxybenzoyl)amino]octanoic acid (NAC),
N-[6-(2-hydroxybenzoyl)amino]hexanoic acid (NAH),
N-[10-(2-hydroxybenzoyl)amino]decanoic acid (NAD),
N-[8-(5-chlorosalicyloyl)amino]octanoic acid (5-CNAC),
N-[8-(2-hydroxy-4-methoxybenzoyl)amino]octanoic acid (4-MOAC),
N-[4-(2-hydroxy-4-chlorobenzoyl)amino]butyric acid (4-CNAB), and
a pharmaceutically acceptable salt thereof.

10. The pharmaceutical composition of any one of claims 1 to 9, wherein the delivery agent is one or more selected from:
sodium N-[8-(2-hydroxybenzoyl)amino]octanoate (SNAC),
potassium N-[8-(2-hydroxybenzoyl)amino]octanoate, and/or
calcium N-[8-(2-hydroxybenzoyl)amino]octanoate.

11. The pharmaceutical composition of any one of claims 1 to 10, wherein the delivery agent is SNAC.

12. The pharmaceutical composition of any one of claims 1 to 11, wherein the auxiliary agent comprises one or more of the following compounds: sodium phosphate, disodium hydrogen phosphate, sodium tartrate, sodium acetate, sodium citrate, sodium glutamate, hydrotalcite, and other pharmaceutically acceptable salts comprising the same acid radicals.

13. Use of a pharmaceutical composition of any one of claims 1 to 12 in the preparation of a medicament for treating and/or preventing chronic kidney disease-associated pruritus, chronic liver disease-associated pruritus, and atopic dermatitis.

14. A method of treating and/or preventing chronic kidney disease-associated pruritus, chronic liver disease-associated pruritus, and atopic dermatitis, comprising orally administering to a subject in need thereof the pharmaceutical composition of any one of claims 1 to 12.

15. A pharmaceutical composition of any one of claims 1 to 12 for use in treating and/or preventing chronic kidney disease-associated pruritus, chronic liver disease-associated pruritus, and atopic dermatitis.
